(19) Europäisches Patentamt
European Patent Office
Office européen des brevets

(11) Publication number : **0 486 280 A2**

# (12) EUROPEAN PATENT APPLICATION

(21) Application number : **91310476.6**

(22) Date of filing : **13.11.91**

(51) Int. Cl.[5] : **C07D 221/20,** C07D 211/96, C07D 409/12, C07D 401/12, A61K 31/445, C07D 405/12, C07D 417/14, C07D 417/12, C07D 413/12

(30) Priority : **13.11.90 US 612344**
**13.09.91 US 759254**

(43) Date of publication of application :
**20.05.92 Bulletin 92/21**

(84) Designated Contracting States :
**AT BE CH DE DK ES FR GB GR IT LI LU NL SE**

(71) Applicant : **MERCK & CO. INC.**
**126, East Lincoln Avenue P.O. Box 2000**
**Rahway New Jersey 07065-0900 (US)**

(72) Inventor : **Bock, Mark G.**
**1603 Leon Drive**
**Hatfield, PA 19440 (US)**
Inventor : **Evans, Ben E.**
**501 Perkiomen Avenue**
**Lansdale, PA 19446 (US)**
Inventor : **Freidinger, Roger M.**
**744 Newport Lane**
**Lansdale, PA 19446 (US)**
Inventor : **Gilbert, Kevin**
**665 Elm Street**
**Bally, PA 19503 (US)**
Inventor : **Hobbs, Doug W.**
**843 Garfield Avenue**
**Lansdale, PA 19446 (US)**
Inventor : **Lundell, George F.**
**817 Hoover Road**
**Blue Bell, PA 19442 (US)**
Inventor : **Pettibone, Douglas J.**
**69 Hickory Lane**
**Chalfont, PA 18914 (US)**
Inventor : **Rittle, Kenneth E.**
**1955 Old Woods Road**
**Green Lane, PA 18054 (US)**

(74) Representative : **Thompson, John Dr. et al**
**Merck & Co., Inc. European Patent Department Terlings Park Eastwick Road**
**Harlow, Essex CM20 2QR (GB)**

(54) **Piperidinylcamphorsulfonyl oxytocin antagonists.**

(57) Spirocyclic compounds of the formula :

EP 0 486 280 A2

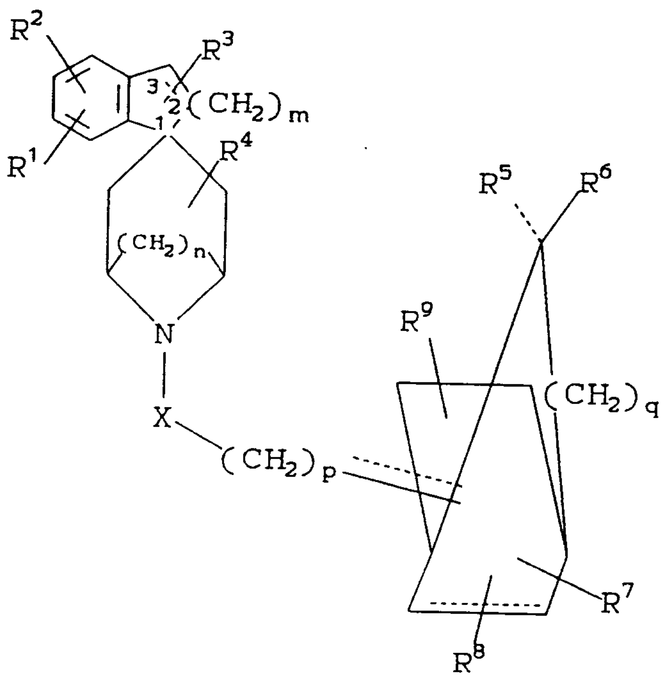

I

are oxytocin and vasopressin antagonists useful in the treatment of pre-term labor, dysmenorrhea and for the stoppage of labor preparatory to cesarean delivery, timing of parturition, uterine hyperactivity, endometriosis, hypertension, congestive heart failure, hyponatremia and cognitive disorders.

## FIELD OF THE INVENTION

The present invention provides novel compounds, novel compositions, methods of their use and methods of their manufacture, such compounds generally pharmacologically useful as agents in obstetric and gynecologic therapy. The aforementioned pharmacologic activities are useful in the treatment of mammals. More specifically, the compounds of the present invention can be used in the treatment of preterm labor, stopping labor preparatory to Cesarean delivery, and in the treatment of dysmenorrhea. At the present time, there is a need in the area of obstetric and gynecologic therapy for such agents.

## BACKGROUND OF THE INVENTION

In the field of obstetrics, one of the most important problems is the management of preterm labor. A significant number of the pregnancies progressing past 20 weeks of gestation experience premature labor and delivery, which is a leading cause of neonatal morbidity and mortality. Despite major advances in neonatal care, retention of the fetus in utero is preferred in most instances.

Tocolytic (uterine-relaxing) agents that are currently in use include $\beta_2$-adrenergic agonists, magnesium sulfate and ethanol. Ritodrine, the leading $\beta_2$-adrenergic agonist, causes a number of cardiovascular and metabolic side effects in the mother, including tachycardia, increased renin secretion, hyperglycemia (and reactive hypoglycemia in the infant). Other $\beta_2$-adrenergic agonists, including terbutaline and albuterol have side effects similar to those of ritodrine. Magnesium sulfate at plasma concentrations above the therapeutic range of 4 to 8 mg/dL can cause inhibition of cardiac conduction and neuromuscular transmission, respiratory depression and cardiac arrest, thus making this agent unsuitable when renal function is impaired. Ethanol is as effective as ritodrine in preventing premature labor, but it does not produce a corresponding reduction in the incidence of fetal respiratory distress that administration of ritodrine does.

It has been proposed that a selective oxytocin antagonist would be the ideal tocolytic agent. In the last few years, evidence has accumulated to strongly suggest that oxytocin is the physiological initiator of labor in several mammalian species including humans. Oxytocin is believed to exert this effect in part by directly contracting the uterine myometrium and in part by enhancing the synthesis and release of contractile prostaglandins from the uterine endometrium/decidua. These prostaglandins may, in addition, be important in the cervical ripening process. By these mechanisms, the process of labor (term and preterm) is initiated by a heightened sensitivity of the uterus to oxytocin, resulting in part as a result of a well-documented increase in the number of oxytocin receptors in this tissue. This "up-regulation" of oxytocin receptors and enhanced uterine sensitivity appears to be due to trophic effects of rising plasma levels of estrogen towards term. By blocking both the direct (contractile) and indirect (enhanced prostaglandin synthesis) effects of oxytocin on the uterus, a selective oxytocin antagonist would likely be more efficacious for treating preterm labor than current regimens. In addition, since oxytocin at term has major effects only on the uterus, such a compound would be expected to have few, if any, side effects.

The compounds of the present invention can also be useful in the treatment of dysmenorrhea. This condition is characterized by cyclic pain associated with menses during ovulatory cycles. The pain is thought to result from uterine contractions and ischemia, probably mediated by the effect of prostaglandins produced in the secretory endometrium. By blocking both the direct and indirect effects of oxytocin on the uterus, a selective oxytocin antagonist can be more efficacious for treating dysmenorrhea then current regimens.

An additional use for the present invention is for the stoppage of labor preparatory to Cesarean delivery. Certain spiroindanylpiperidines and spiroindenylpiperidines are known (U.S. Patents 3,654,287 and 3,666,764), however, they are reported to be useful as anesthetic agents which is quite distinct from the utility of the present invention.

It is, therefore, a purpose of this invention to identify substances which more effectively antagonize the function of oxytocin in disease states in animals, preferably mammals, especially in humans. It is another purpose of this invention to prepare novel compounds which more selectively inhibit oxytocin. It is still another purpose of this invention to develop a method of antagonizing the functions of oxytocin in disease states in mammals. It is also a purpose of this invention to develop a method of preventing or treating oxytocin related disorders of particularly preterm labor and dysmenorrhea.

## SUMMARY OF THE INVENTION

It has now been found that compounds of formula I are antagonists of oxytocin and bind to the oxytocin receptor. These compounds are useful in the treatment and prevention of oxytocin-related disorders of animals, preferably mammals and especially humans. These disorders are primarily preterm labor and dysmenorrhea.

The compounds would also find usefulness for stoppage of labor preparation to Cesarean delivery.

The compounds of the present invention are those of the general structural formula I:

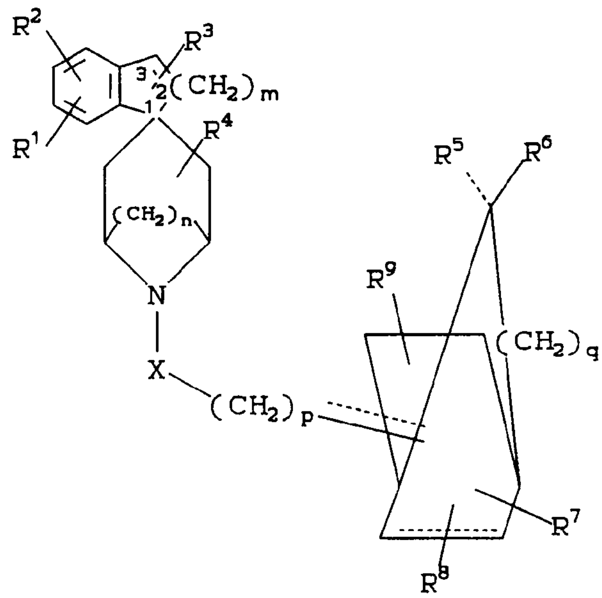

and the pharmaceutically acceptable salts thereof, wherein:

X is

C=O,

$CH_2$,

$PO_2H$,

O

$PO_2H$-O-Y, where Y is $C_{1-10}$ alkyl,

S,

SO and

$SO_2$;

O

C-NH;

$SO_2NH$;

m is an integer of from one to three;

n is an integer of from zero to two;

p is an integer of from zero to two;

q is an integer of from zero to one;

r is an integer of from zero to five;

$R^1$ and $R^2$ are independently

hydrogen,

halogen,

hydroxy,

$C_{1-10}$ alkyl,

$C_{1-10}$ alkoxy and

trifluoro methyl;

$R^3$ is hydrogen,

alkyl,

halogen,

hydroxy, and

oxo

with the proviso that, when $R^3$ is oxo, the 2,3 bond is saturated;

$R^4$ is hydrogen,

phenyl,

$C_{1-10}$ hydroxyalkyl, and

$C_{1-15}$ alkyl;

$R^5$ and $R^6$ are independently
hydrogen and
$C_{1-10}$ alkyl;

further wherein

$R^5$ and $R^6$ together form
oxo,
fused phenyl and
unsubstituted or substituted $C_{3-6}$ cycloalkyl,
wherein said substituents are
hydroxy,
$C_{1-10}$ alkyl,
$C_{1-10}$ hydroxyalkyl,
$C_{1-10}$ alkoxy,
$C_{1-10}$ alkoxyalkoxyalkoxyalkyl;

$R^7$, $R^8$ and $R^9$ are independently
hydrogen,
unsubstituted or substituted $C_{1-10}$ alkyl wherein said substituents are
cyano when C is two to ten,
hydroxyl,
carboxyl,
$C_{1-10}$ alkoxy,
$C_{1-10}$ alkoxycarbonyl, and
unsubstituted or substituted 5-membered heterocyclic rings containing 1 hetero atom, wherein said hetero atom is N and said substituents are
oxo,
amino,
$C_{1-10}$ alkylamino,
$C_{1-10}$ carboxyalkylcarbonylamino,
$C_{1-10}$ dicarboxyalkylamino, and
$C_{1-10}$ dialkoxycarbonylalkylamino;
$C_{1-10}$ dialkyl,
$C_{7-10}$ alkoxy,
$C_{1-10}$ alkoxycarbonyl,
$C_{1-10}$ alkoxycarbonylalkylaminocarbonyl,
fused phenyl,
cyano,
$C_{2-10}$ cyanoalkyl,
phosphate,
$C_{1-10}$ alkyl substituted phosphonate,
sulfate,
$C_{1-10}$ alkyl substituted sulfonate,
halogen substituted $C_{1-10}$ alkyl substituted sulfonate,

$$-(CH_2)_r-\underset{R^{11}}{N}-R^{10}$$

wherein $R^{10}$ is
hydrogen,
$C_{1-10}$ alkylsulfonyl,
$C_{1-10}$ alkarylsulfonyl,
$C_{1-10}$ aralkylsulfonyl,

$C_{1-10}$ alkoxyarylsulfonyl,
aminosulfonyl,
$C_{1-10}$ alkylaminosulfonyl,
$C_{1-10}$ dialkylaminosulfonyl,
unsubstituted or substituted $C_{3-15}$ cycloalkyl, bicycloalkyl or tricycloalkyl wherein said substituent is
oxo and
sulfonyl substituted by unsubstituted or substituted $C_{3-15}$ cycloalkylalkyl, bicycloalkyl or tricycloalkyl wherein said substituent is
oxo,
oxime and
hydroxy;
and wherein $R^{11}$ is
hydrogen,
$C_{1-10}$ alkyl,
$C_{1-10}$ carboxyalkyl,
$C_{1-10}$ alkoxycarbonylalkyl;

$$-(CH_2)_r-\underset{R^{11}}{N}-CO-R^{12},$$

wherein $R^{11}$ is as defined above and $R^{12}$ is
hydrogen,
amino,
$C_{1-10}$ alkylamino,
unsubstituted or substituted $C_{1-10}$ alkyl wherein said substituents are
unsubstituted or substituted $C_{4-8}$ cycloalkyl wherein said substituent is
hydroxy and
carboxy;
$C_{10-15}$ bi- and tricycloalkyl,
halogen,
hydroxy,
carboxy,
oxo,
oxime,
$C_{1-10}$ alkylthio,
$C_{1-10}$ alkylsulfinyl,
$C_{1-10}$ alkylsulfonyl,
$C_{1-10}$ alkoxycarbonyl;
unsubstituted and substituted 4, 5, 6 and 7 membered heterocyclic rings having 1, 2, 3or 4 hetero atoms or moieties wherein said hetero atoms or moieties are N, S, SO, $SO_2$ and O, wherein said substituents are
$C_{1-10}$ alkyl,
$C_{1-10}$ aralkyl,
$C_{1-10}$ aralkoxy,
$C_{1-10}$ alkaryl,
amino,
$C_{1-10}$ alkylamino,
oxo,
oxime,
fused phenyl,
$C_{1-10}$ alkoxycarbonyl,

$C_{1-10}$ alkyl carbonate,
$C_{1-10}$ alkyl ureide,
$C_{1-10}$ alkyl carbamate and
unsubstituted or substituted 5, 6 and 7 membered heterocyclic rings having 1, 2, 3 and 4 hetero atoms, wherein said hetero atoms are N, O and S and wherein said subsituents are
oxo and
fused phenyl;
unsubstituted or substituted phenyl, whereinsaid substituents are
halogen,
hydroxy,
carboxy,
$C_{1-10}$ alkyl,
$C_{1-10}$ carboxyalkyl,
$C_{1-10}$ alkoxy,
sulfate and
5, 6 and 7 membered heterocyclic rings having 1, 2, 3 or 4 hetero atoms, wherein said hetero atoms are N, O and S;
amino,
aminocarbonyl,
$C_{1-10}$ dialkylaminocarbonyl,
$C_{1-10}$ alkylamino,
$C_{1-10}$ dialkylamino,
$C_{1-10}$ alkylcarbamate,
$C_{1-10}$ alkylcarbonate,
$C_{1-10}$ alkylureide,
$C_{1-10}$ aralkylcarbamate,
unsubstituted or substituted aryloxy wherein said substituents are
amino,
$C_{1-10}$ alkyl and
$C_{1-10}$ aminoalkyl;
$C_{1-10}$ aralkyloxy,
unsubstituted or substituted $C_{1-10}$ alkaryloxy wherein said substituent is
$C_{1-10}$ alkyl carbamate,
N, N-$C_{1-10}$ alkyl -$C_{1-10}$ carboxyalkyl and
N, N, N-$C_{1-10}$ dialkyl -$C_{1-10}$
alkoxycarbonylalkyl;
unsubstituted or substituted $C_{3-8}$ cycloalkyl wherein said substituents are
$C_{1-10}$ alkyl and
carboxy;
N, N-$C_{1-10}$ dialkyl,
unsubstituted or substituted $C_{5-15}$ bicycloalkyl and tricycloalkyl wherein said substituent is
carboxy;
$C_{1-10}$ alkoxy,
unsubstituted or substituted phenyl, wherein said substituents are
amino,
halogen,
$C_{1-10}$ alkyl,
$C_{1-10}$ alkoxy,
nitro,
phenyl carbonyl and
unsubstituted or substituted 5, 6 and 7 membered heterocyclic rings containing 1, 2, 3 or 4 heterocyclic atoms, wherein said hetero atoms are O, N and S and wherein said substituent is
oxo;
unsubstituted or substituted 4, 5, 6, 7 or 8 membered mono and bicyclic heterocyclic rings containing 1, 2, 3, or 4 hetero atoms or moieties wherein said hetero atoms or moieties are N, O, S, SO and $SO_2$ and whe-

rein said substituents are

oxo,
hydroxy,
carboxy,
amino,
$C_{1-10}$ carboxyalkyl,
$C_{1-10}$ alkyl,
$C_{1-10}$ alkoxy,
$C_{1-10}$ aralkoxy,
$C_{1-10}$ alkaryloxy,
$C_{1-10}$ alkoxycarbonyl,
$C_{1-10}$ alkoxycarbonylamino,
$C_{1-10}$ alkoxycarbonylalkyl,
$C_{1-10}$ aralkoxycarbonyl and
substituted or unsubstituted aryl
wherein said substituents are

$C_{1-5}$ alkyl,
carboxy and
halogen;

further wherein $R^7$ and $R^8$ are joined to form 5, 6 and 7 membered heterocyclic rings containing 2, 3 or 4 hetero atoms wherein said hetero atoms are N, O and S, and

$-(CH_2)_r-CO-NH-R^{13}$, wherein $R^{13}$ is

$C_{1-10}$ alkyl,
$C_{1-10}$ aminoalkyl,
$C_{1-10}$ alkoxycarbonylalkyl,
$C_{1-10}$ carboxyalkyl,
8 membered bicyclic heterocyclic rings containing 1 or 2 hetero atoms wherein said hetero atoms are N,
$-(CH_2)_rNH-CO-NH-R_{14}$ where $R^{14}$ is

hydrogen,

substituted or unsubstituted $C_{1-10}$ alkyl and $C_{7-15}$ bi- and tricycloalkyl wherein said substituents are

carboxy,
$C_{1-10}$ alkoxycarbonyl,
aminocarbonyl and
$C_{1-10}$ carboxyalkyl; and

substituted and unsubstituted 5, 6 and 7 membered heterocyclic rings containing 1 or 2 hetero atoms or moieties wherein said hetero atoms or moieties are N, O, S, SO and $SO_2$ and wherein said substituents are

oxo,
carboxy,
amino,
$C_{1-10}$ carboxyalkyl,
$C_{1-10}$ alkyl and
$C_{1-10}$ alkoxycarbonylamino.

Salts encompassed within the term "pharmaceutically acceptable salts" refer to non-toxic salts of the compounds of this invention which are generally prepared by reacting the free base with a suitable organic or inorganic acid. Representative salts include the following salts:

| | |
|---|---|
| Acetate | Lactobionate |
| Benzenesulfonate | Laurate |
| Benzoate | Malate |
| Bicarbonate | Maleate |
| Bisulfate | Mandelate |
| Bitartrate | Mesylate |
| Borate | Methylbromide |
| Bromide | Methylnitrate |
| Calcium Edetate | Methylsulfate |
| Camsylate | Mucate |
| Carbonate | Napsylate |
| Chloride | Nitrate |
| Clavulanate | Oleate |
| Citrate | Oxalate |
| Dihydrochloride | Pamoate (Embonate) |
| Edetate | Palmitate |
| Edisylate | Pantothenate |
| Estolate | Phosphate/diphosphate |
| Esylate | Polygalacturonate |
| Fumarate | Salicylate |
| Gluceptate | Stearate |
| Gluconate | Subacetate |
| Glutamate | Succinate |
| Glycollylarsanilate | Tannate |
| Hexylresorcinate | Tartrate |
| Hydrabamine | Teoclate |
| Hydrobromide | Tosylate |
| Hydrocloride | Triethiodide |
| Hydroxynaphthoate | Valerate |
| Iodide | |
| Isethionate | |
| Lactate | |

The term "pharmacologically effective amount" shall mean that amount of a drug or pharmaceutical agent that will elicit the biological or medical response of a tissue, system, animal or human that is being sought by a researcher or clinician.

The term "alkyl" shall mean straight or branched chain alkanes, alkenes and alkynes with one or more degrees of unsaturation at any position on the chain.

The term "aryl" shall mean phenyl.

The term "cycloalkyl" shall mean cyclic rings of alkanes, alkenes or alkynes with one or more degrees of unsaturation at any position of the ring.

Whenever the terms "alkyl" or "aryl" or either of their prefix roots appear in a name of a substituent (e.g. aralkoxyaryloxy) they shall be interpreted as including those limitations given above for "alkyl" and "aryl". Designated numbers of carbon atoms (e.g. $C_{1-10}$) shall refer independently to the number of carbon atoms in an alkyl or cyclic alkyl moiety or to the alkyl portion of a larger substituent in which alkyl appears as a prefix root.

The term "oxo" shall refer to the substituent =O.

The term "halogen" shall include iodine, bromine, chlorine and fluorine.

The term "preterm labor" shall mean expulsion from the uterus of a viable infant before the normal end of gestation, or more particularly, onset of labor with effacement and dilation of the cervix before the 37th week of gestation. It may or may not be associated with vaginal bleeding or rupture of the membranes.

The term "dysmenorrhea" shall mean painful menstruation.

The term "cesarean delivery" shall mean incision through the abdominal and uterine walls for delivery of a fetus.

As used herein, the definition of each expression, when it occurs more than once in any structure, can be independent of its definition elsewhere in the same structure.

The ability of the compounds of formula I to antagonize oxytocin makes these compounds useful as pharmacologic agents for mammals, especially for humans, for the treatment and prevention of disorders wherein

oxytocin may be involved. Examples of such disorders include preterm labor and especially dysmenorrhea. These compounds may also find use-fulness for stoppage of labor preparatory to Cesarean delivery. Because of the known relationship of vasopressin to oxytocin, the compounds of the present invention are also useful as vasopressin antagonists. They are useful in the treatment or prevention of disease states involving vasopressin disorders, including their use as diuretics and their use in congestive heart failure.

The compounds of the present invention can be administered in such oral dosage forms as tablets, capsules (each including timed release and sustained release formulations), pills, powders, granules, elixers, tinctures, suspensions, syrups and emulsions. Likewise, they may also be administered in intravenous (both bolus and infusion), intraperitoneal, subcutaneous or intramuscular form, all using forms well known to those of ordinary skill in the pharmaceutical arts. An effective but non-toxic amount of the compound desired can be employed as a tocolytic agent.

The dosage regimen utilizing the compounds of the present invention is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter or arrest the progress of the condition.

Oral dosages of the present invention, when used for the indicated effects, will range between about 0.3-6.0 gm/day orally. Intravenously, the most preferred doses will range from 0.1 to about 10 mg/minute during a constant rate infusion. Advantageously, compounds of the present invention may be administered in a single daily dose, or the total daily dosage may be administered in divided doses of two, three or four times daily. Furthermore, preferred compounds for the present invention can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittant throughout the dosage regimen.

In the methods of the present invention, the compounds herein described in detail can form the active ingredient, and are typically administered in admixture with suitable pharmaceutical diluents, excipients or carriers (collectively referred to herein as "carrier" materials) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, zanthan gum and the like.

The compounds of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Compounds of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamide-phenol, polyhydroxyethylaspartamide-phenol, or polyethyleneoxidepolylysine substituted with palmitoyl residues. Furthermore, the compounds of the present invention may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

The compounds of formula I can be prepared readily according to the following reaction Schemes and Examples or modifications thereof using readily available starting materials, reagents and conventional synthesis procedures. In these reactions, it is also possible to make use of variants which are themselves known to those of ordinary skill in this art, but are not mentioned in greater detail. Reference can also be made to applicants co-pending application, U.S. Serial No. 488343, corresponding to EP-A-0450761, the entire disclosure of which is incorporated herein by reference. Additional reference can be made to the method of Matier, et al., J. Org. Chem., Vol. 36, No. 5, 650-654 (1971), the entire disclosure of which is incorporated herein by reference, on elaboration of indenes and their 2-oxo derivatives to spiropiperidine analogs. Also incorporated herein by

reference is a varient of this procedure described in Matier, et al., J. Org. Chem., Vol. 36, No. 5, 650-654 (1971).

The most preferred compounds of the invention are any or all of those specifically set forth in these examples. These compounds are not, however, to be construed as forming the only genus that is considered as the invention, and any combination of the compounds or their moieties may itself form a genus. The following examples further illustrate details for the preparation of the compounds of the present invention. Those skilled in the art will readily understand that known variations of the conditions and processes of the following preparative procedures can be used to prepare these compounds. All temperatures are degrees Celsius unless noted otherwise.

## EXAMPLE 1

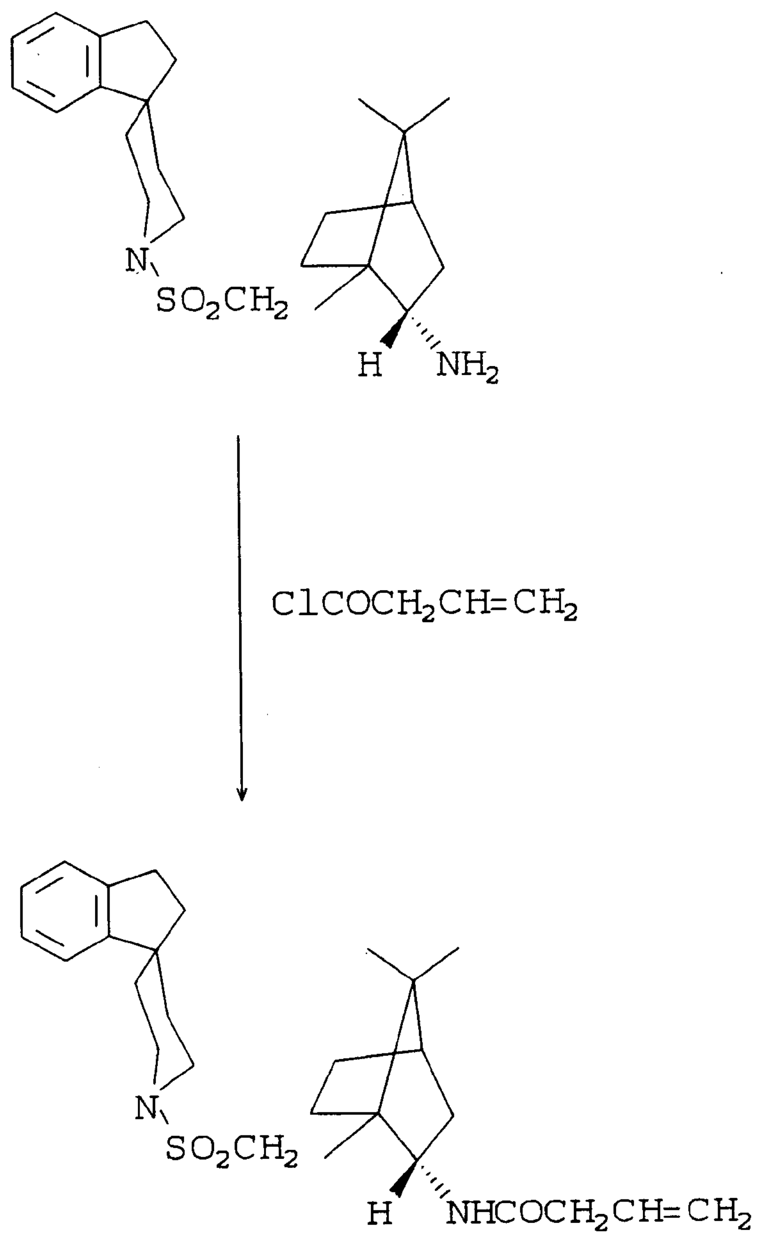

EXAMPLE 2

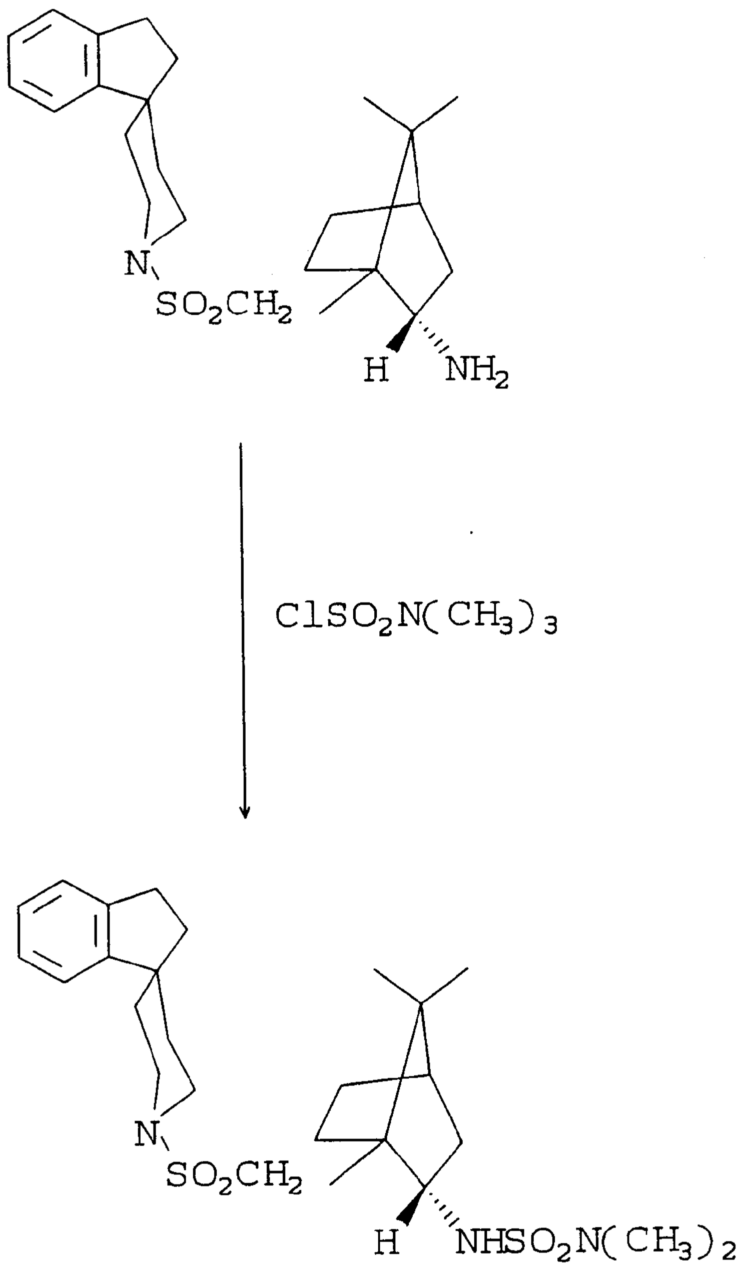

## EXAMPLE 3

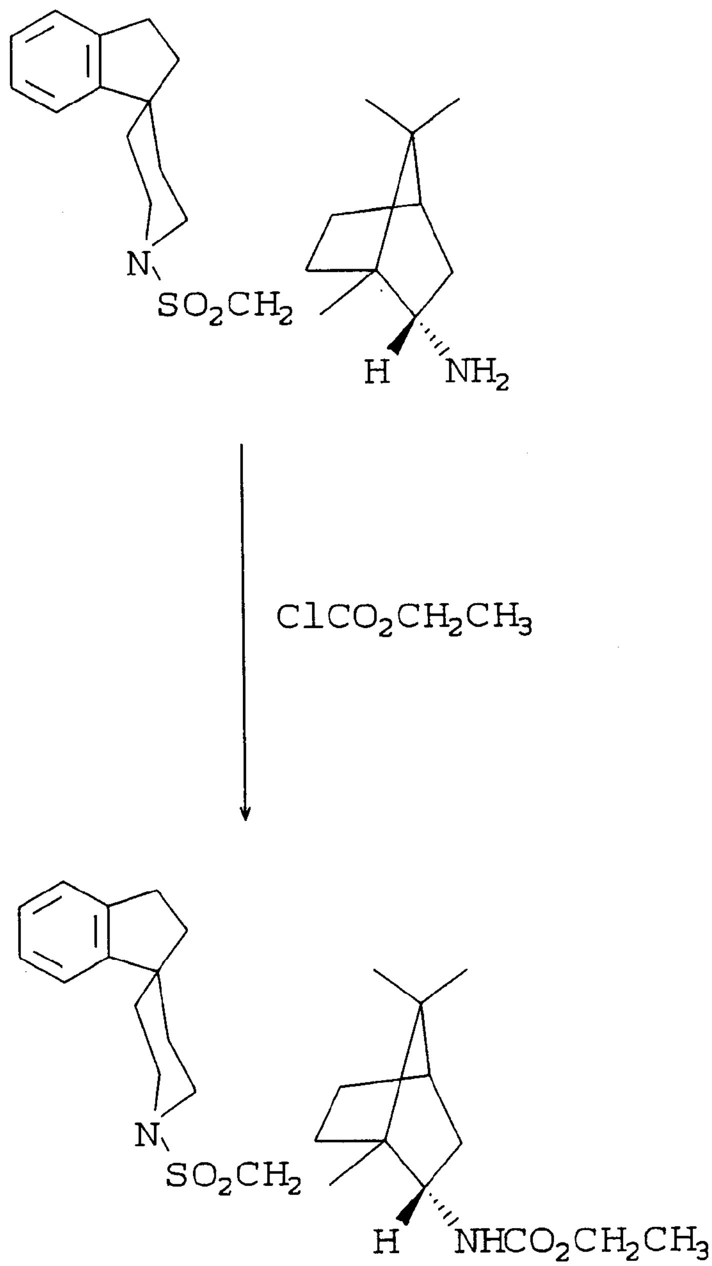

EXAMPLE 4

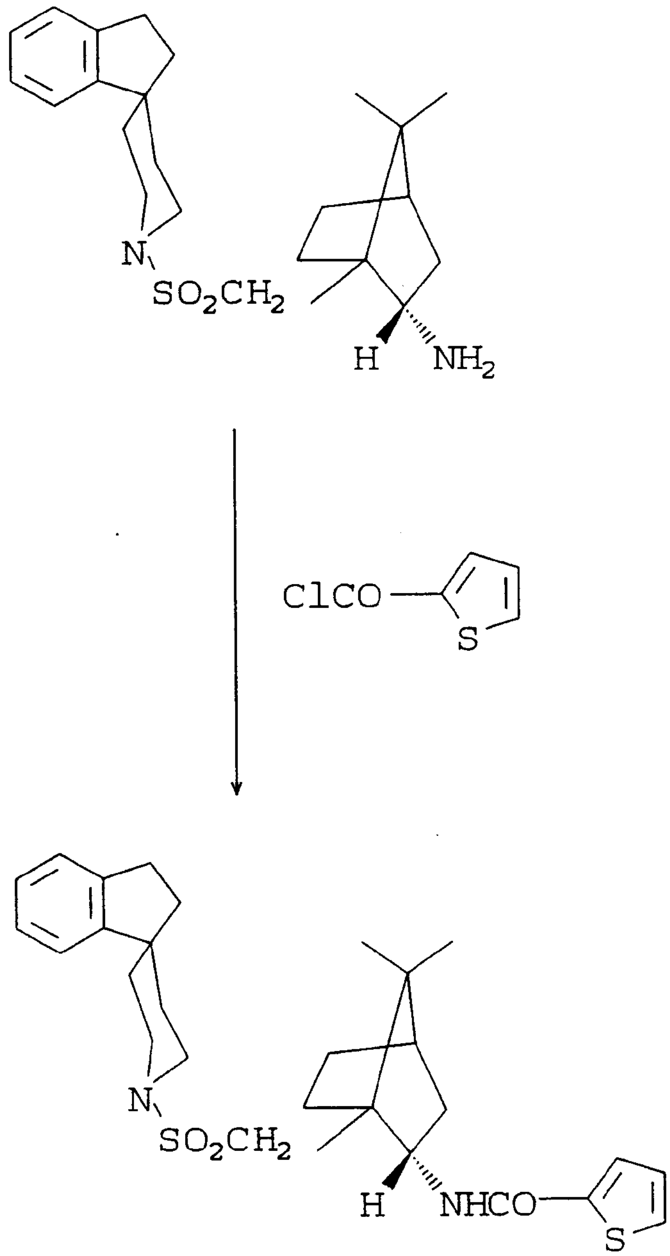

EXAMPLE 5

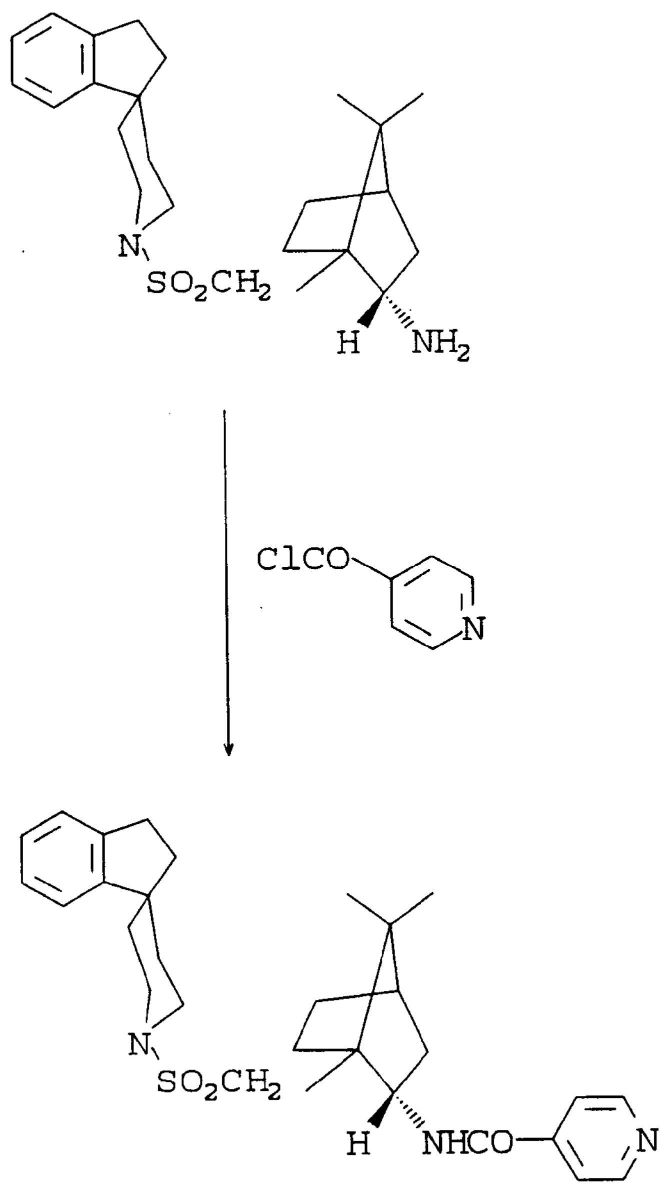

EXAMPLE 6

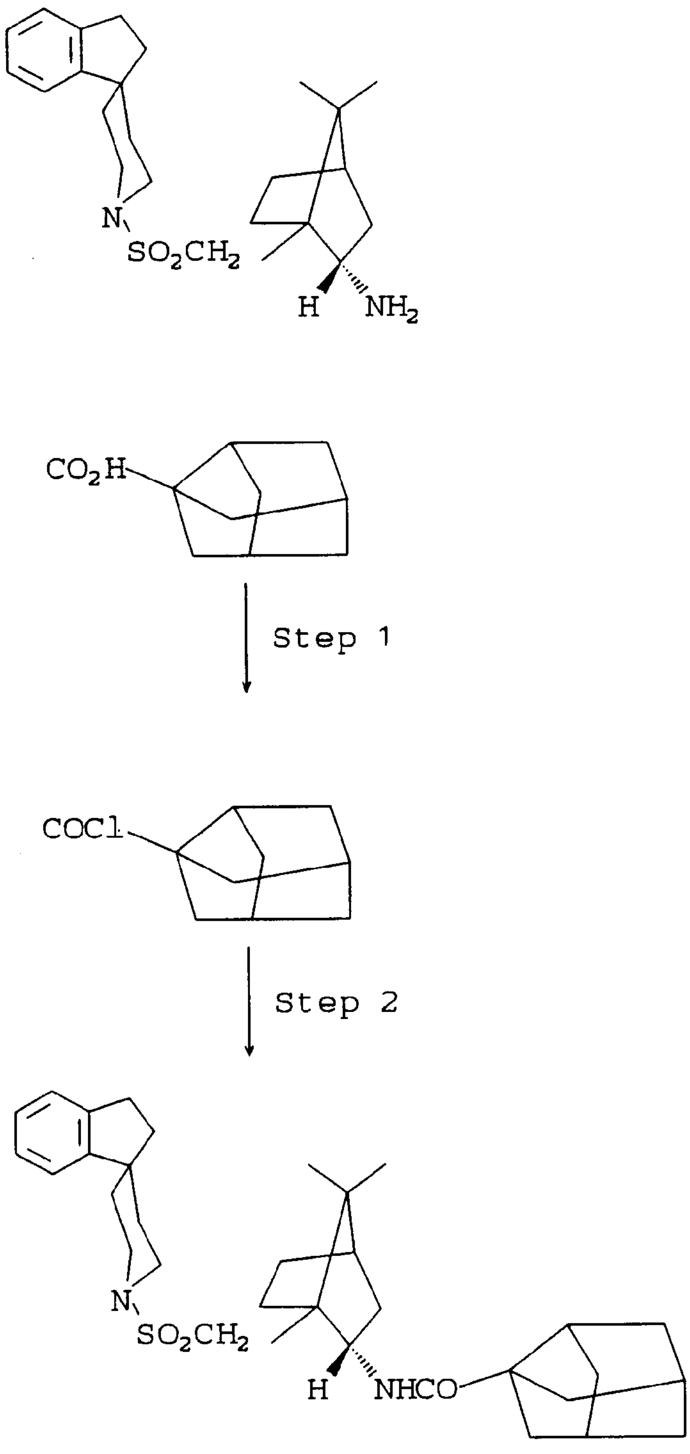

## EXAMPLE 8

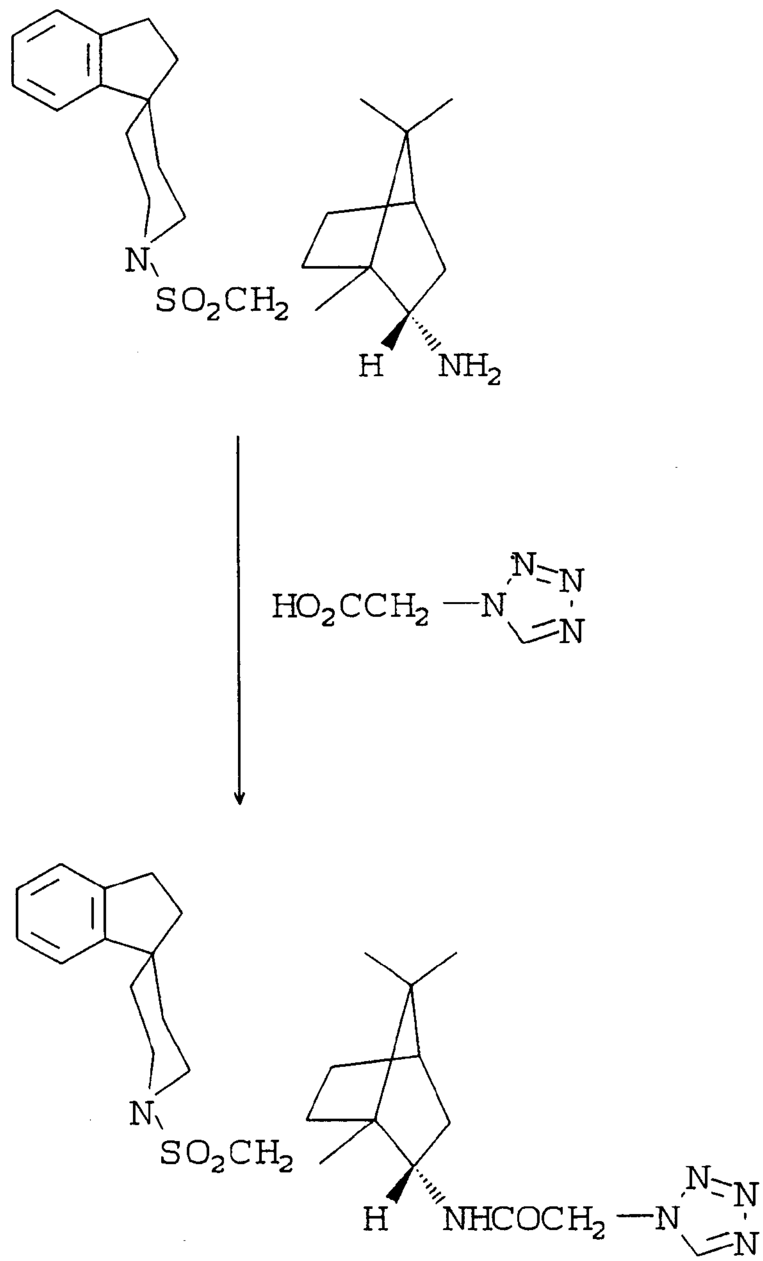

## REACTION SCHEMES, PART 2

EXAMPLE 9

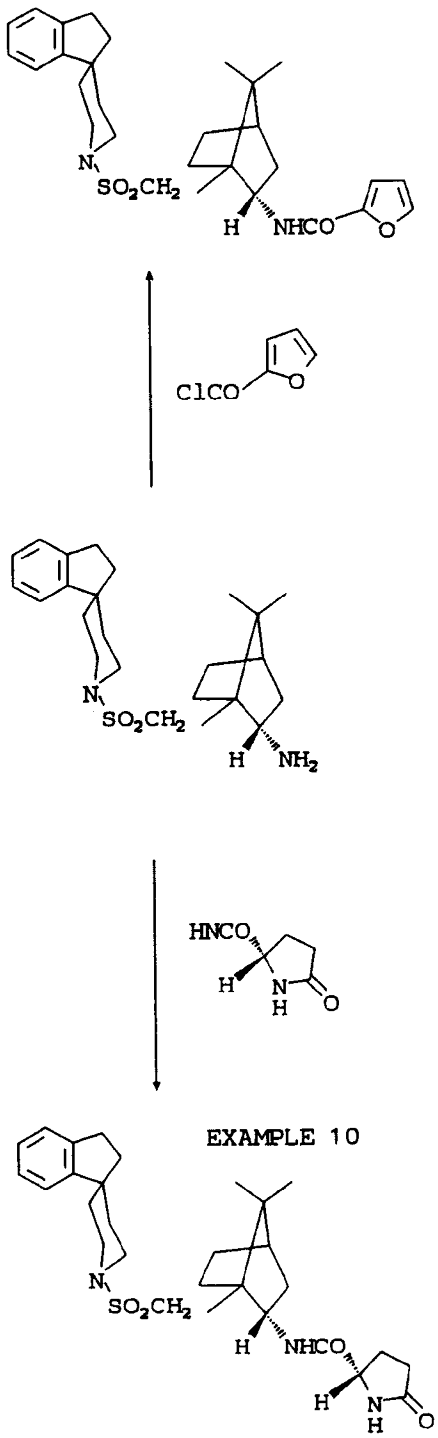

EXAMPLE 10

REACTION SCHEMES, PART 3

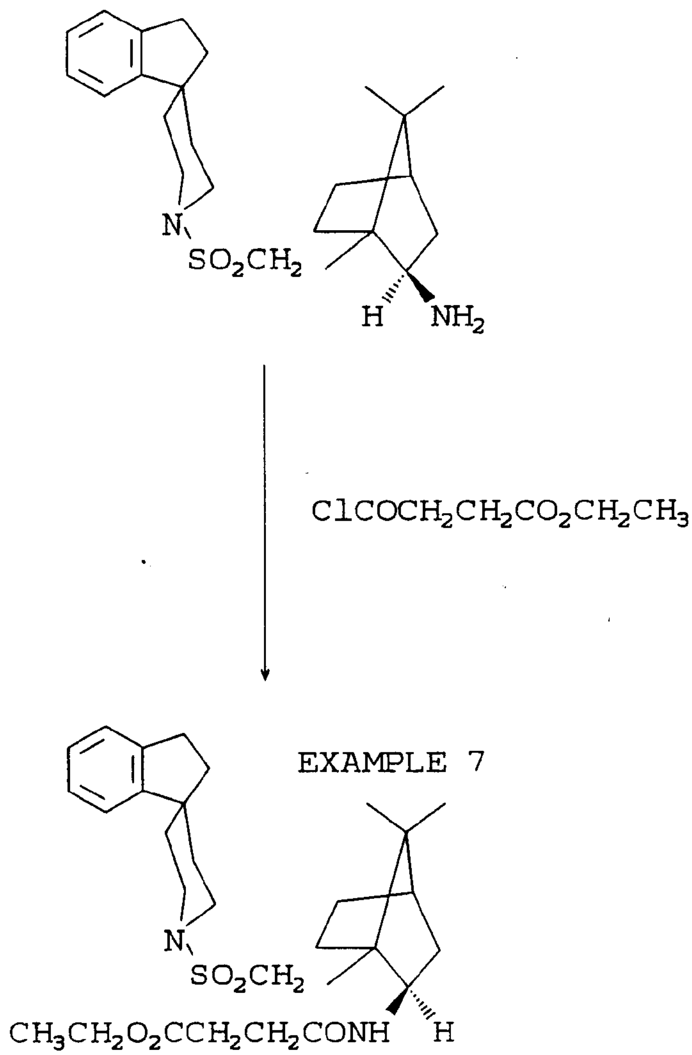


EXAMPLE 7

REACTION SCHEMES, PART 3 cont'd

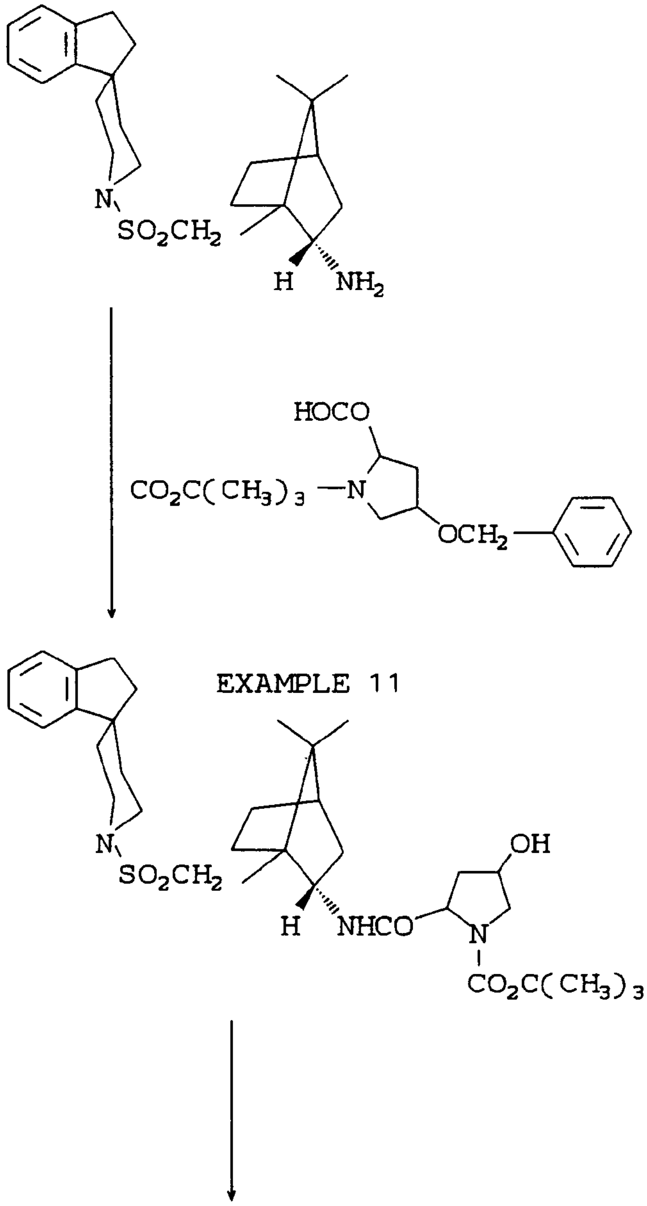

EXAMPLE 11

REACTION SCHEMES, PART 3 cont'd

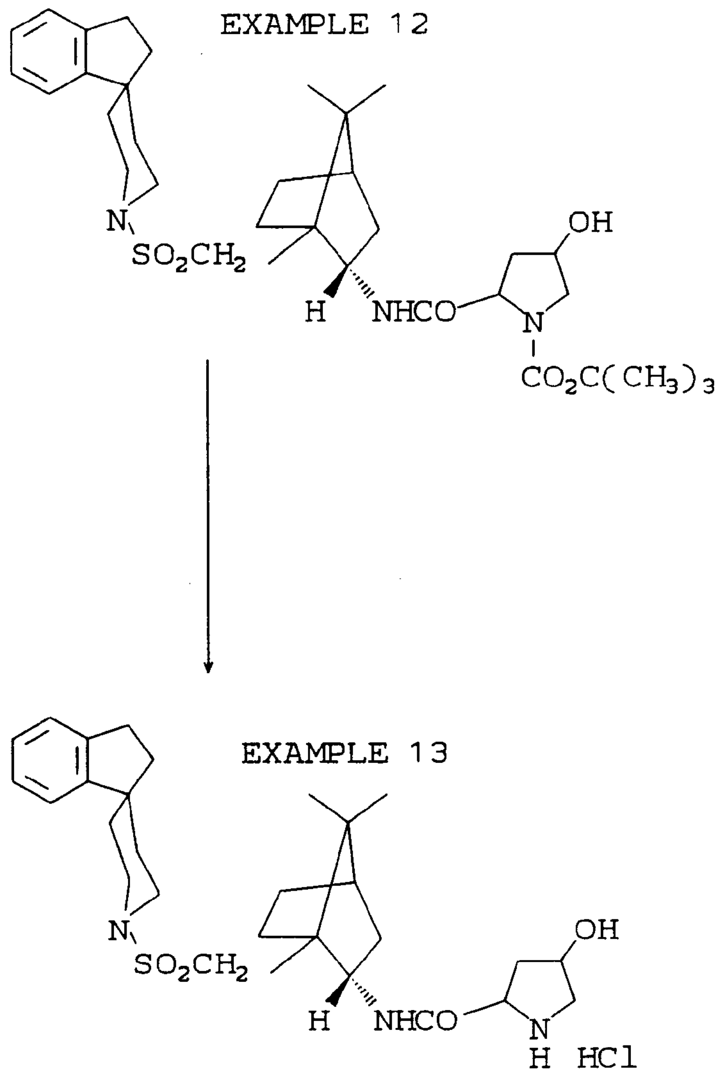

REACTION SCHEMES, PART 3 cont'd

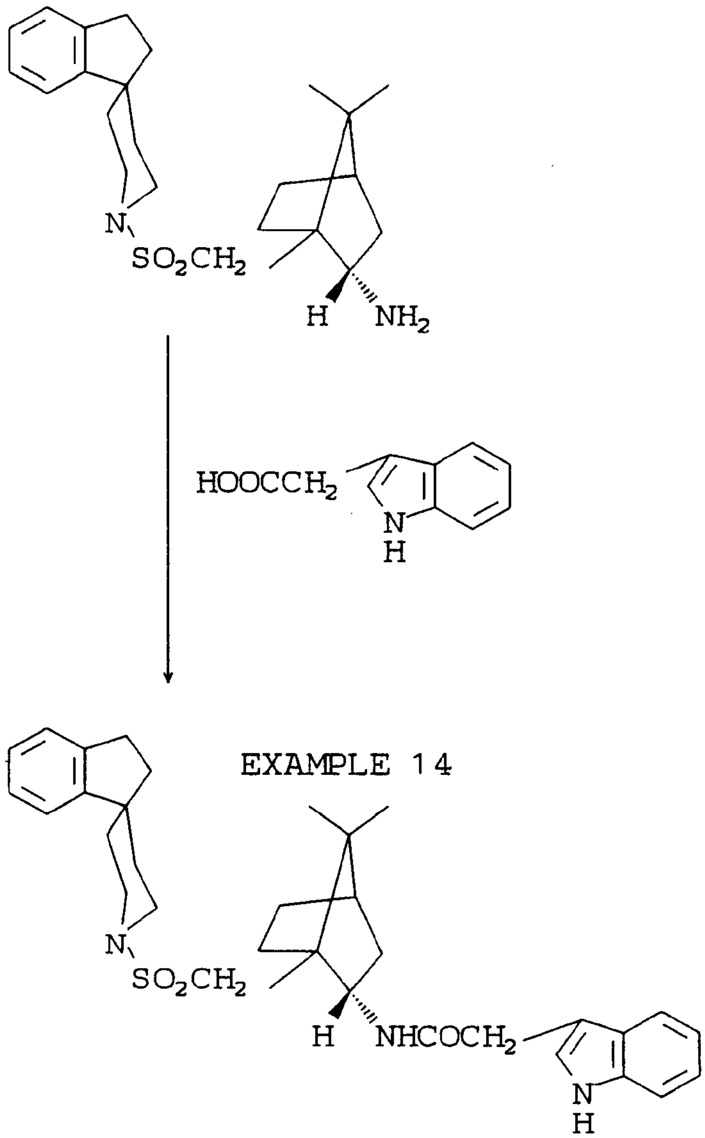

EXAMPLE 14

REACTION SCHEMES, PART 4

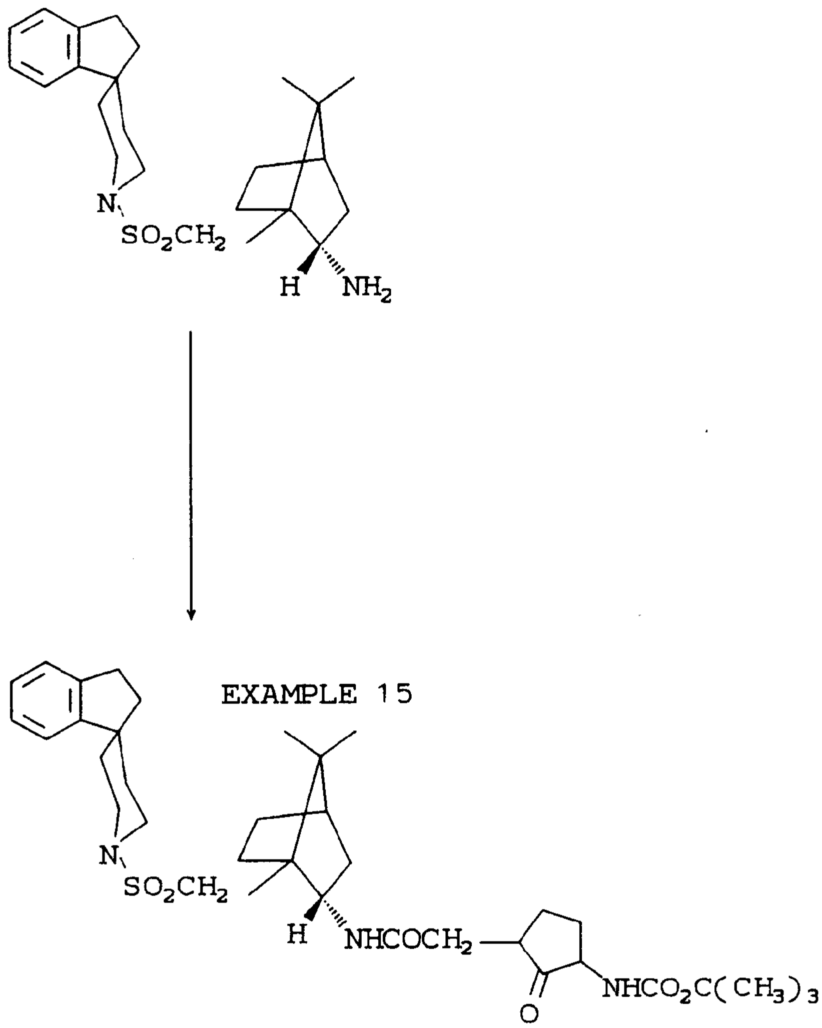

EXAMPLE 15

REACTION SCHEMES, PART 4 cont'd

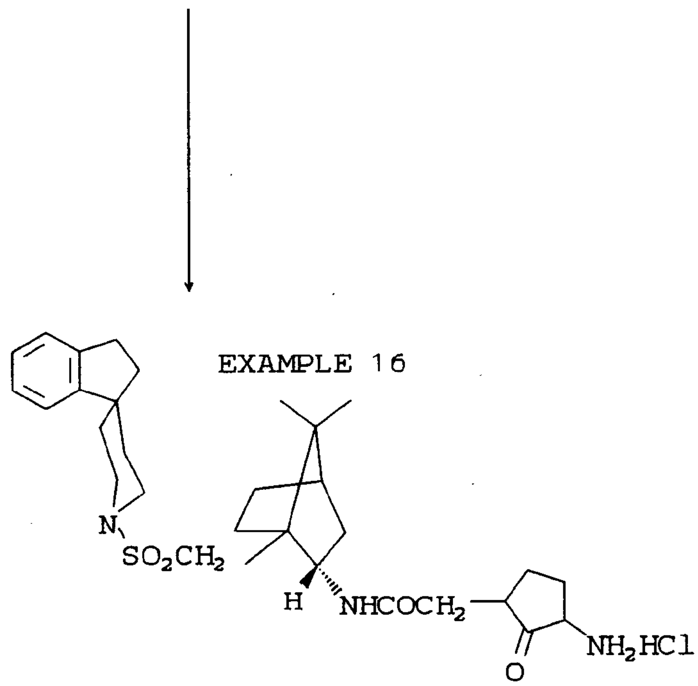

EXAMPLE 16

## REACTION SCHEMES, PART 5

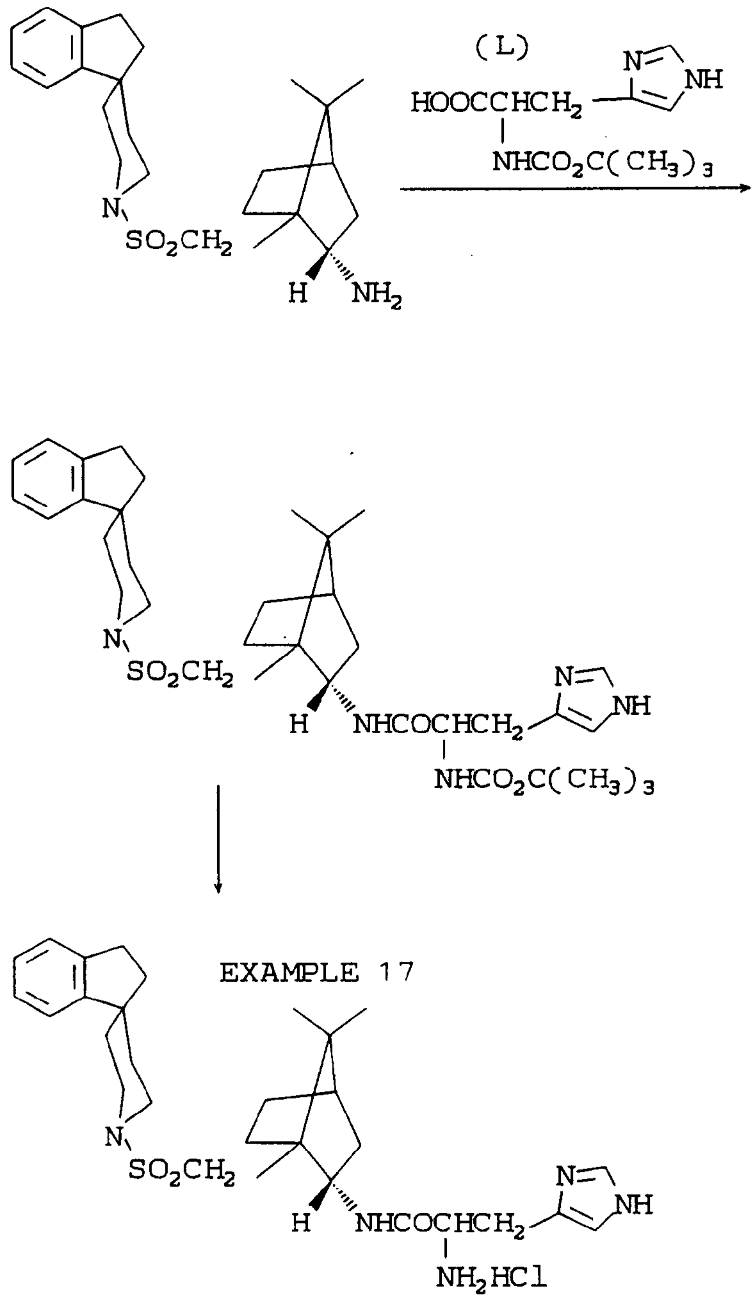


EXAMPLE 17

REACTION SCHEMES, PART 5 cont'd

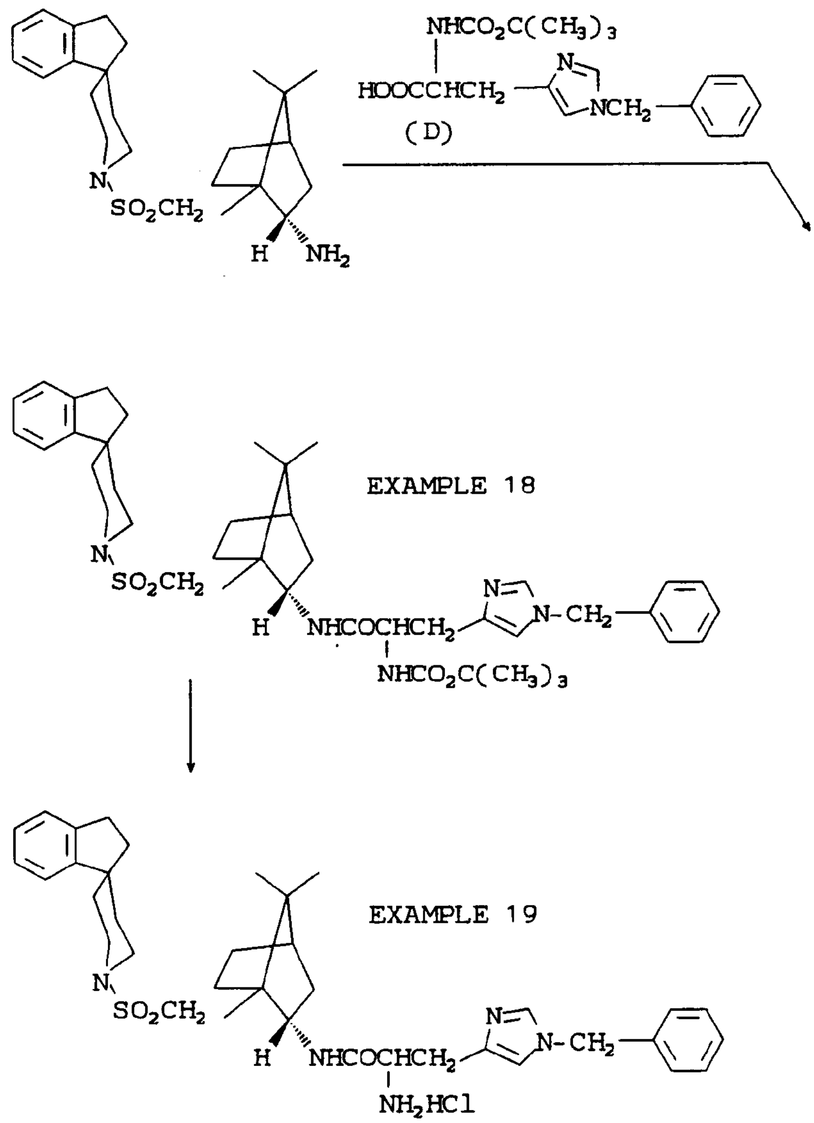

## REACTION SCHEMES, PART 6

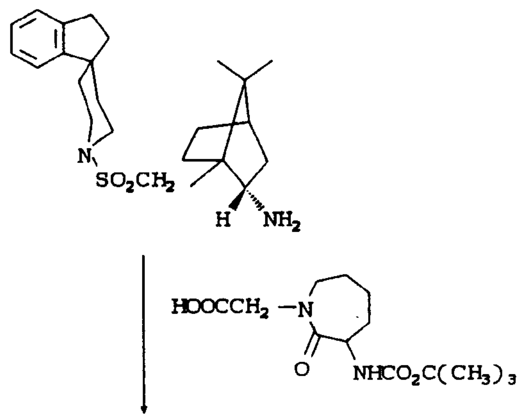

EXAMPLE 20

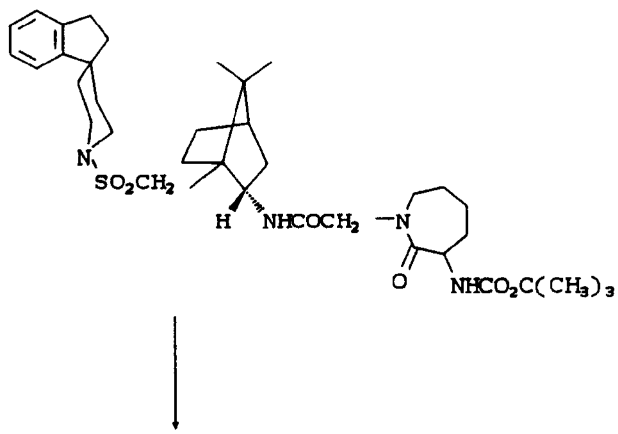

EXAMPLE 21

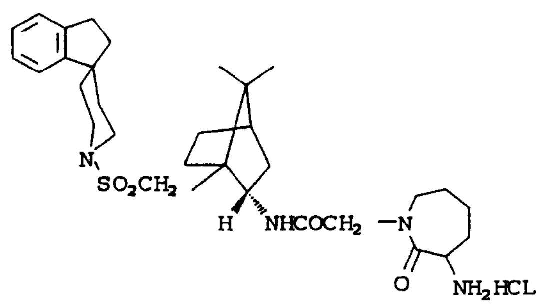

## REACTION SCHEMES, PART 7

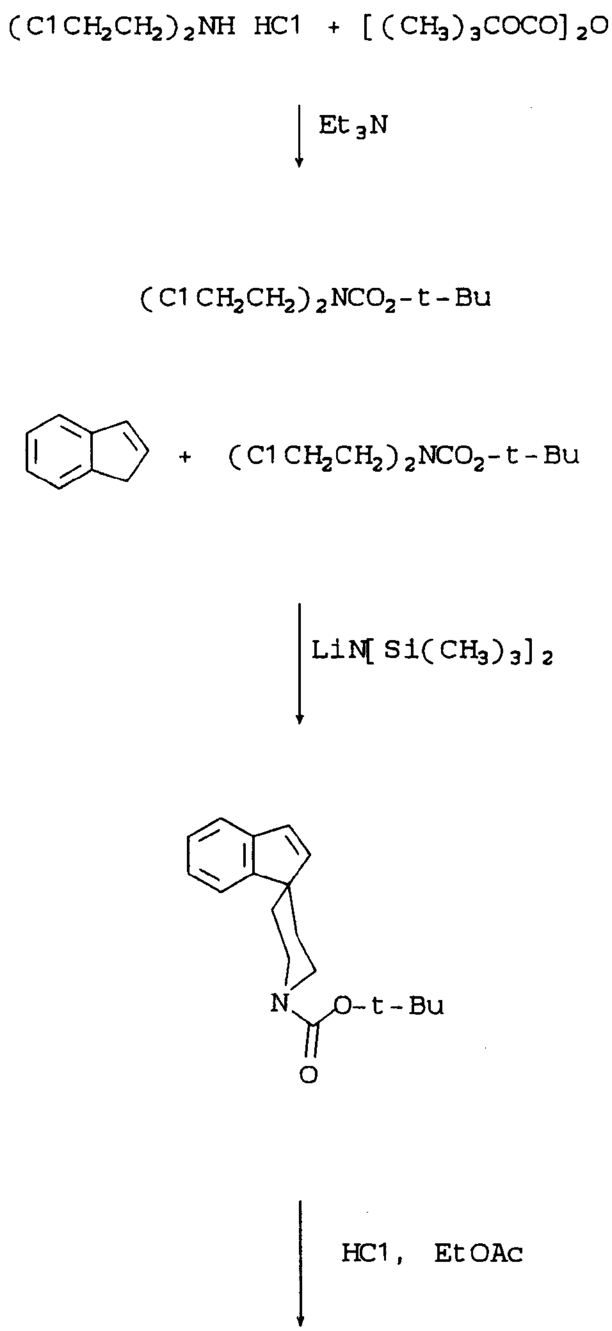

REACTION SCHEMES, PART 7 cont'd

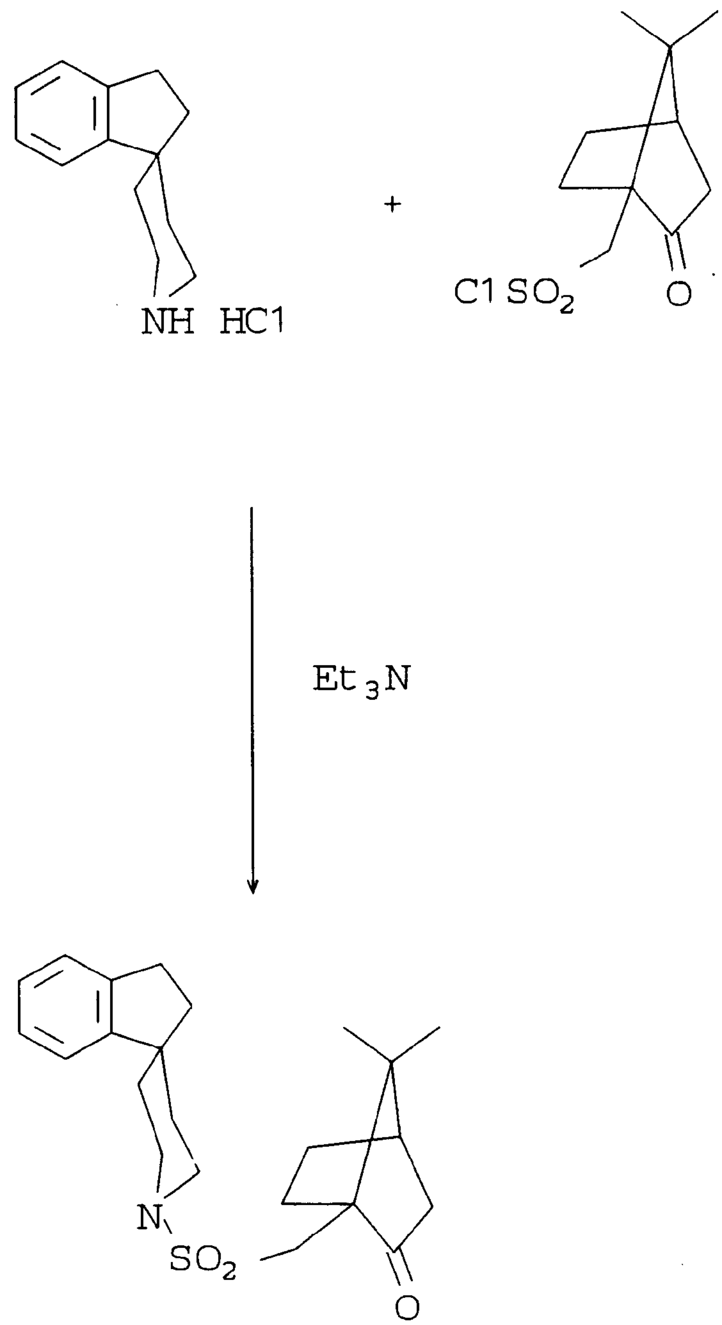

REACTION SCHEMES, PART 7 cont'd

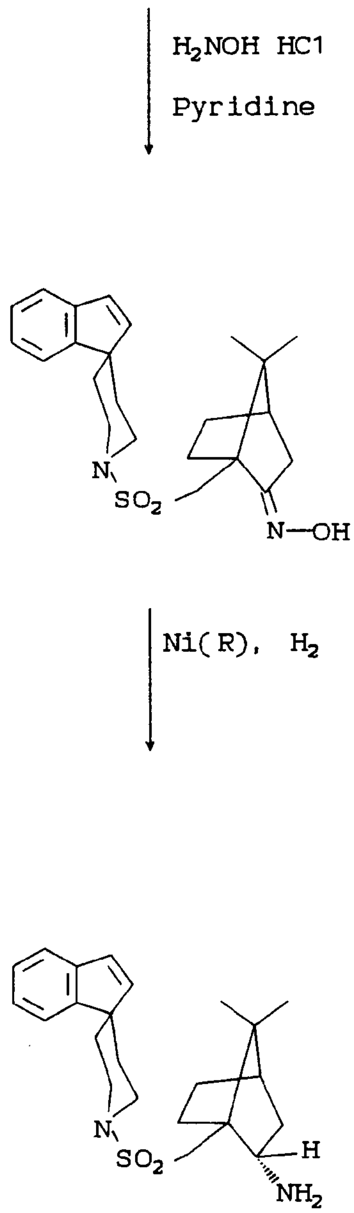


+

Endo Isomer

REACTION SCHEMES, PART 7 cont'd

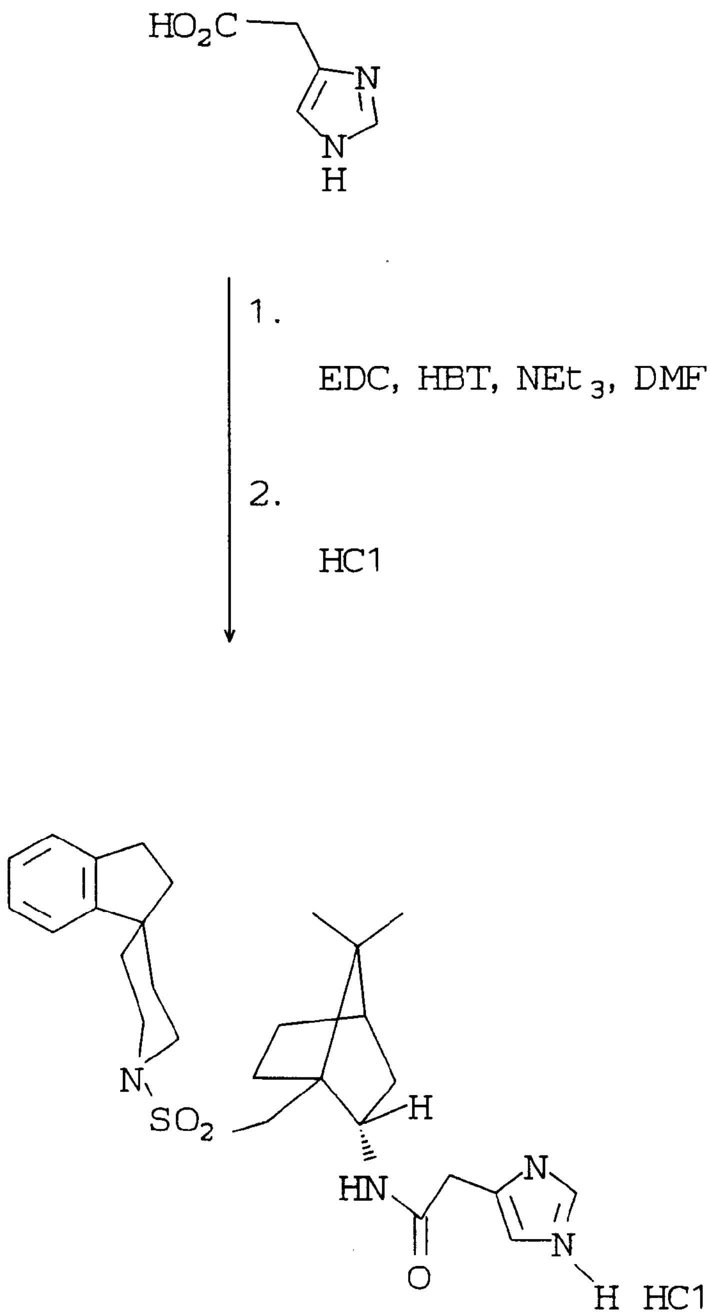

REACTION SCHEMES, PART 8

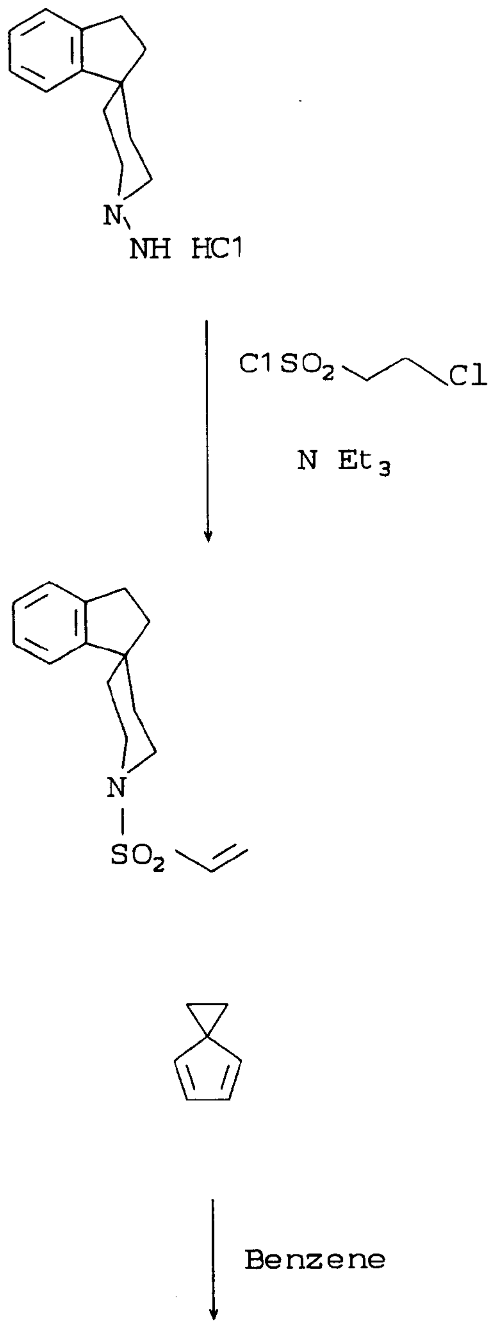

REACTION SCHEMES, PART 8 cont'd

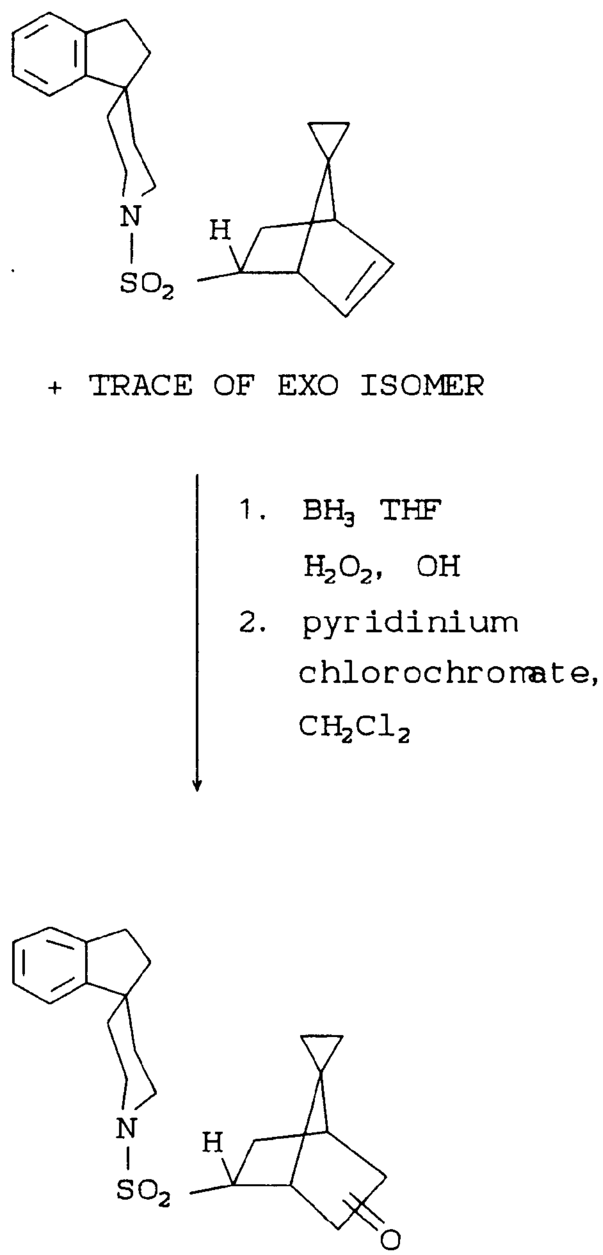


Example 31

REACTION SCHEMES, PART 8 cont'd

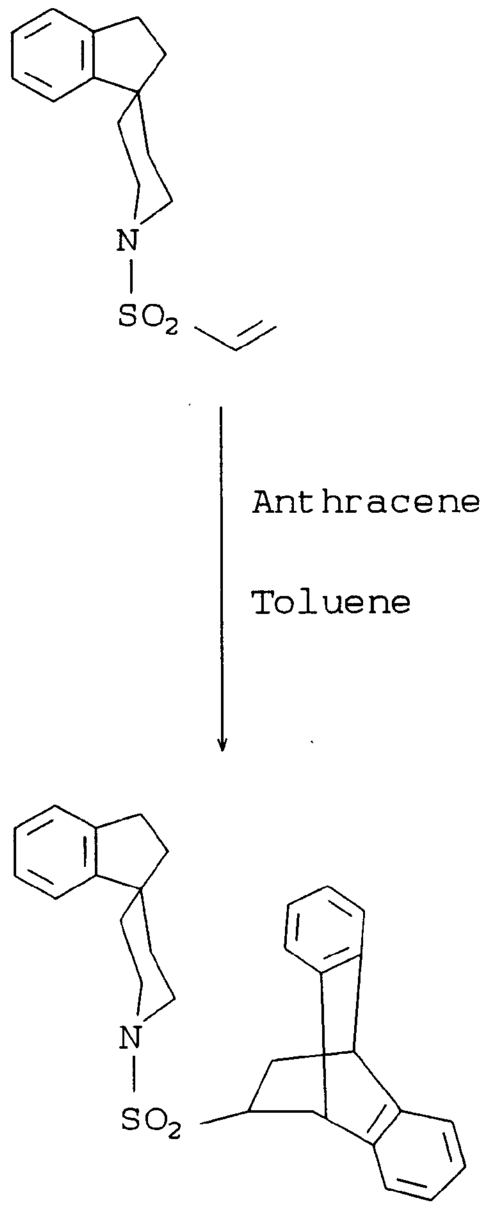


Example 32

REACTION SCHEMES, PART 8 cont'd

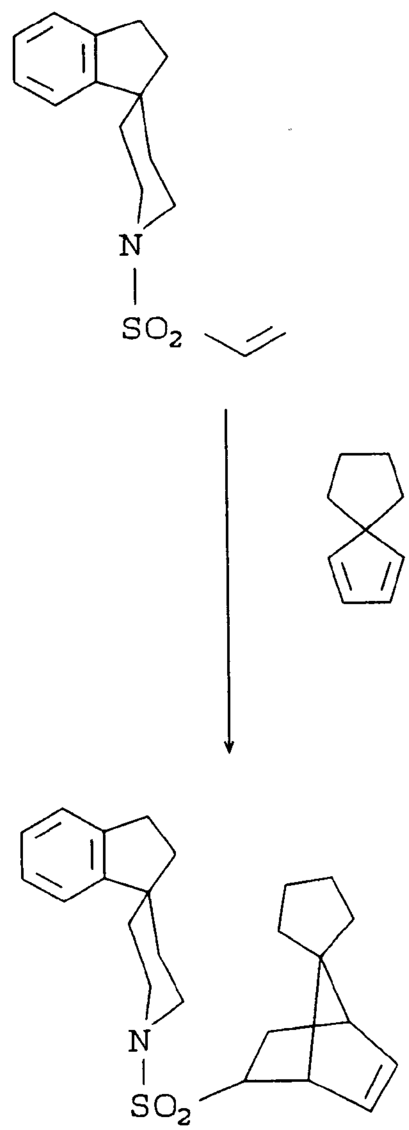

Example 33

REACTION SCHEMES, PART 8 cont'd

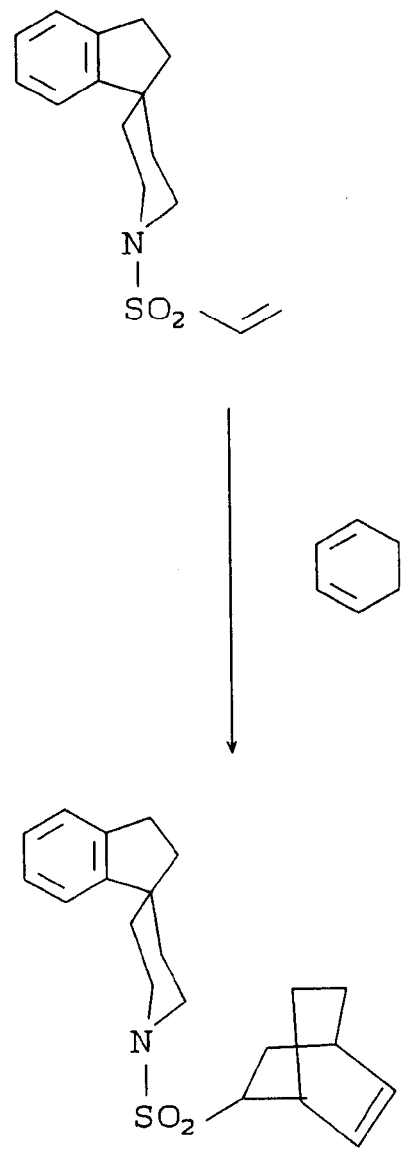

Example 34

REACTION SCHEMES, PART 9

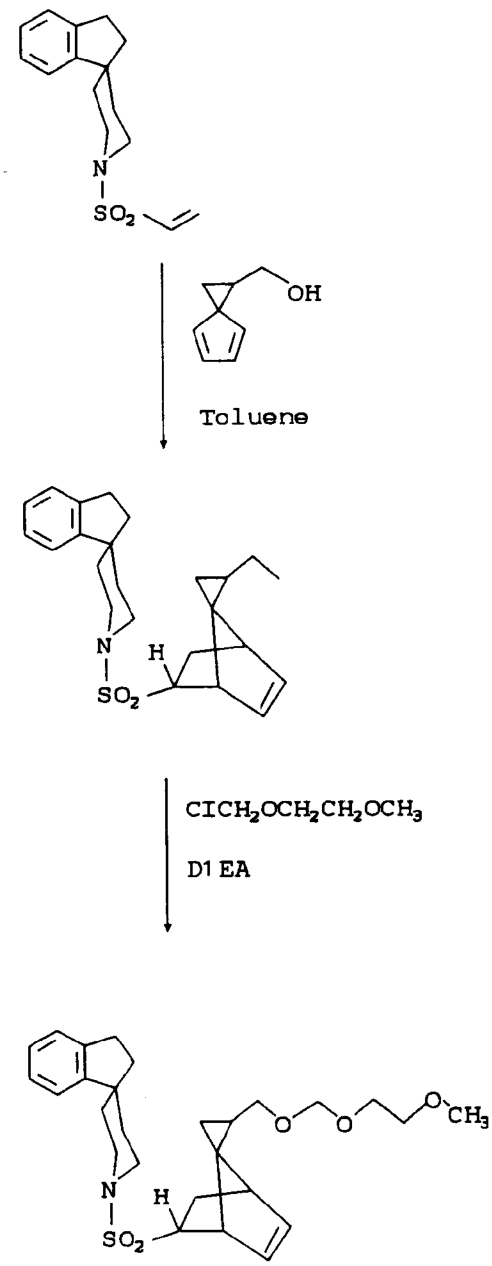


Example 35

REACTION SCHEMES, PART 10

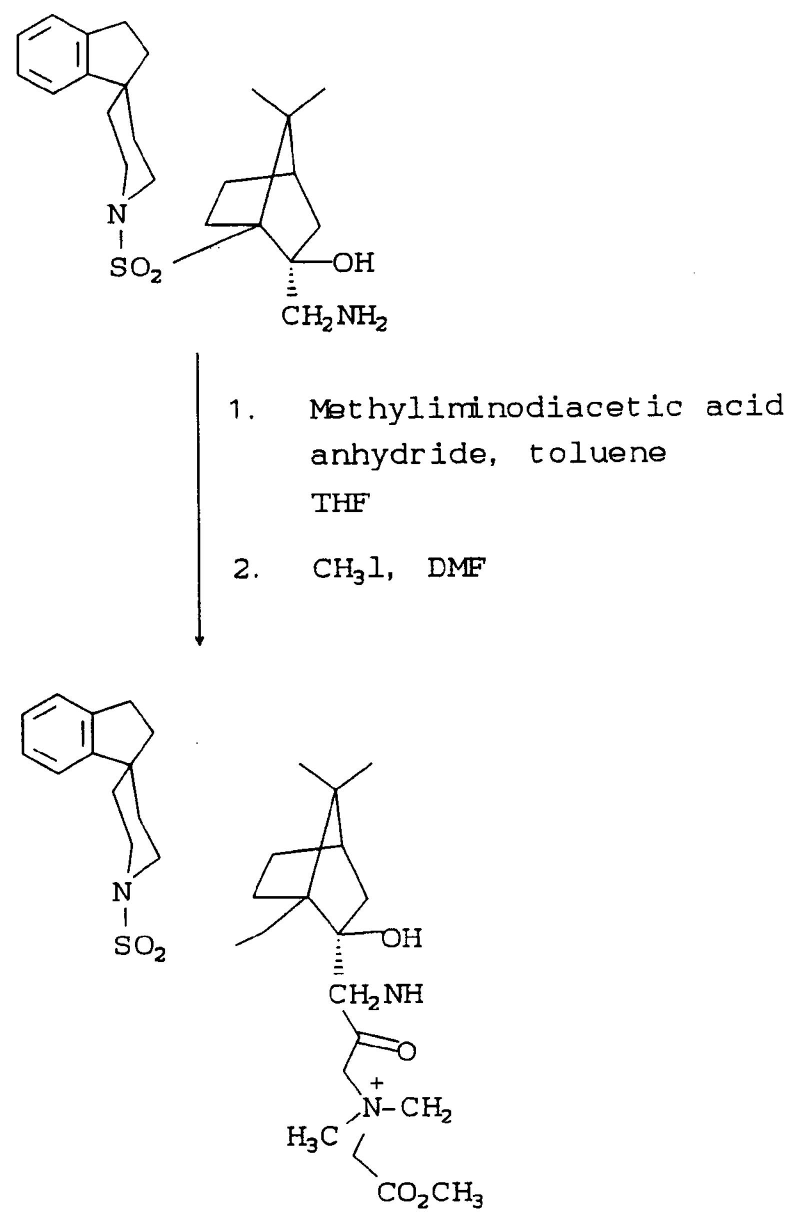


Example 36

## REACTION SCHEMES, PART 11

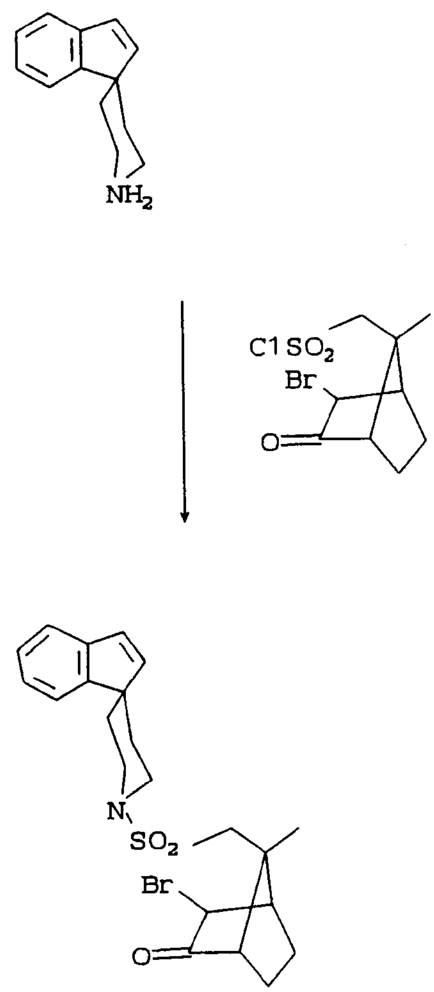

Example 37

REACTION SCHEMES, PART 11 cont'd

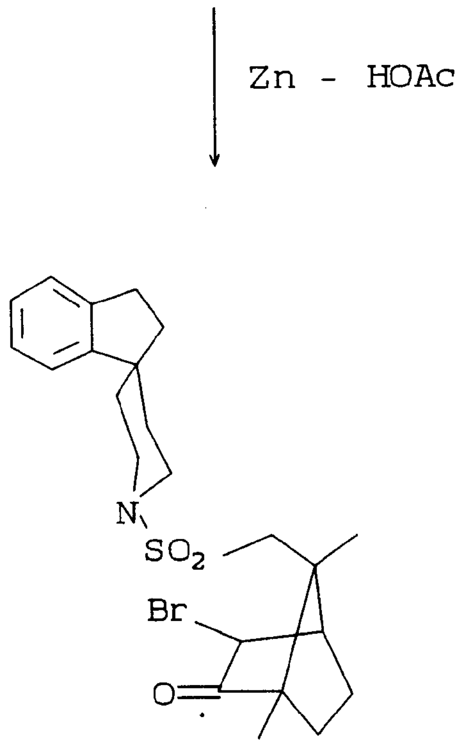


Example 38

EXAMPLE A

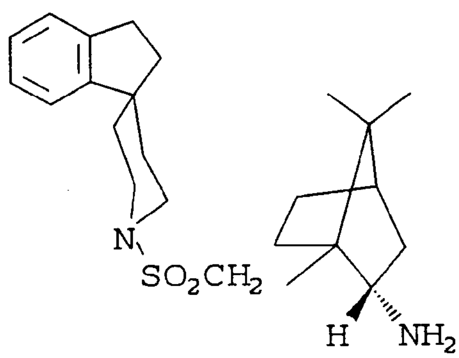

Endo-(1S)-1'(((2-amino-7,7-dimethylbicyclo(2.2.1)-hept-1-yl)-methyl)-sulfonyl)spiro(1H-indan-1,4'-piperidine)

Di-t-butyl dicarbonate (31g, 0.14 mole available from Aldrich) and bis(2-chloroethyl) amine hydrochloride (21.6g, 0.12 mole Aldrich) were combined in $CH_2Cl_2$ (250 ml) stirred at ambient temperature and treated with

triethylamine (12.8 g, 0.127 mole) added dropwise over 15 minutes. After 1 hour, another 1.5 ml of triethylamine was added. After a total of 2.5 hours, the mixture was poured onto a silica gel column packed with $CH_2Cl_2$-:hexane (1:1), and eluted with $CH_2Cl_2$. The combined product fractions were evaporated to dryness in vacuo to give N,N-bis(2-chloroethyl)-t-butyl-carbamate.

To a solution of indene (10.3 g, 89 mmole) in dry tetrahydrofuran (THF, 18 ml) cooled in an ice bath and maintained under a nitrogen blanket was added lithium bis(trimethylsilyl)amide (Aldrich, 177 ml of a 1.0M solution in THF; 177 mmole) over 15 minutes. The mixture was stirred in the cold for 30 minutes, then added over 15 minutes to a solution of N,N-bis(2-chloroethyl)-t-butylcarbamate (21.2 g, 88 mmole) stirred in an ice bath. The mixture was stirred for 2 hours in the cold and for 30 minutes at ambient temperature under nitrogen, then evaporated in vacuo to a foam. $CH_2Cl_2$ was added and the resulting mixture poured onto a silica gel column packed with 40% hexane in $CH_2Cl_2$. The column was eluted with 40% hexane in $CH_2Cl_2$ followed by $CH_2Cl_2$, and the product fractions were evaporated to dryness in vacuo to provide 1′-(t-butyloxycarbonyl)-spiro(indene-1,4′-piperidine).

1′-(t-Butyloxycarbonyl)spiro(indene-1,4′-piperidine) (16 g, 56 mmole) in ethyl acetate (250 ml) was stirred in an ice bath and saturated with HCl(g) for 30 minutes. The mixture was evaporated to dryness. Ethyl acetate was added and removed in vacuo three times, and the residue was triturated with diethyl ether and filtered to provide spiro(1H-indene-1,4′-piperidine) hydrochloride. The free base was obtained by slurrying the hydrochloride in aqueous sodium bicarbonate solution and extracting with $CH_2Cl_2$. The organic layer was separated, dried over sodium sulfate, filtered, and evaporated to dryness in vacuo to provide spiro(1H-indene-1,4′piperidine.

Spiro(1H-indene-1,4′piperidine) (308 mg, 1.66 mmol) and (+)-10-camphorsulfonyl chloride (418 mg, 1.66 mmol) were combined in $CH_2Cl_2$ and treated with triethylamine (0.23 ml). The mixture was stirred at ambient temperature for 15 minutes, then poured onto a silica gel column and eluted with 1:1 $CH_2Cl_2$:hexane. The product fractions were combined and evaporated to dryness in vacuo to provide (1S)-1′-(((7,7-dimethyl-2-oxobicicylo-(2.2.1) hept-1-yl)-methyl)sulfonyl)spiro(1H-indene-1.4′-piperidine) as a solid which was recrystallized from petroleum ether and dried overnight in vacuo at ambient temperature.

(1S)-1′-(((7,7-dimethyl-2-oxobicyclo(2.2.1)hept-1-yl)methyl)sulfonyl)spiro(1H-indene-1,4′-piperidine) (30 g, 0.075 mole) in pyridine (500 mL) was heated in an oil bath to 70°C (internal). Hydroxylamine hydrochloride (30 g) was added in three portions over ca. 20 minutes. After 2 hours, an additional 10 g of hydroxylamine hydrochloride was added (over 10 minutes). At 30, 40, and 50 minutes additional elapsed time, further 3 g lots of hydroxylamine hydrochloride were added. After another 30 minutes, the mixture was poured into water (2 L) and extracted 3 times with ethyl acetate (300 mL portions). The organic layers were combined, washed with 1N HCl (600 mL total), dried over sodium sulfate, filtered, and evaporated to dryness in vacuo. EtOH (abs; ca. 250 mL) was added to the resulting thick syrup and the solution allowed to stand at ambient temperature overnight. The mixture was filtered and the filtrate boiled down to ca. 80 mL. After standing, the mixture was again filtered and boiled down to ca. 20 mL. After a third filtration, the filtered solids were combined to give (1S)-1′-(((7,7-dimethyl-2-oximinobicyclo(2.2.1)hept-1-yl)-methyl) sulfonyl)spiro(1H-indene-1,4′-piperidine) (28 g).

Freshly prepared, activated Raney Nickel catalyst (ca. 30 g) in water was allowed to settle and the water decanted. Abs. ethanol (300 mL) was added, and the mixture swirled and again allowed to settle. The solvent was decanted. Two more wash-decant cycles with 150 mL of ethanol were similarly carried out. (1S)-1′-(((7,7-dimethyl-2-oximinobicyclo (2.2.1)hept-1-yl)methyl)sulfonyl)-spiro(1H-indene-1,4′-piperidine) (30 g) was stirred in a mixture of abs. ethanol (450 mL) and 2-methoxyethanol (900 mL), nitrogen was bubbled through the suspension/solution, and the Raney Nickel catalyst was added. The mixture was hydrogenated under 50 psi overnight. TLC (9:1 $CH_2Cl_2$MeOH, silica gel) showed the reaction to be complete. The catalyst was removed by filtration, and the filtrate evaporated to dryness in vacuo. The crude solid (27 g) was divided into 7 g batches, and each batch was dissolved in methylene chloride (ca. 200 mL) and flash chromatographed on silica (700 g in a 100 mm column, packed and eluted with 8% (v/v) methanol in methylene chloride), taking 200 mL fractions. The exo isomer of the title amine was obtained in fractions ca. 5-7, and the desired endo isomer in fractions ca. 8-16. TLC was on silica, eluted with 8% methanol-methylene chloride, phosphomolybdic acid stain. The combined product fractions were evaporated to dryness to provide the title compound (4.5 g from each 7 g lot, ca. 18 g total) as a colorless solid.

EXAMPLE 1

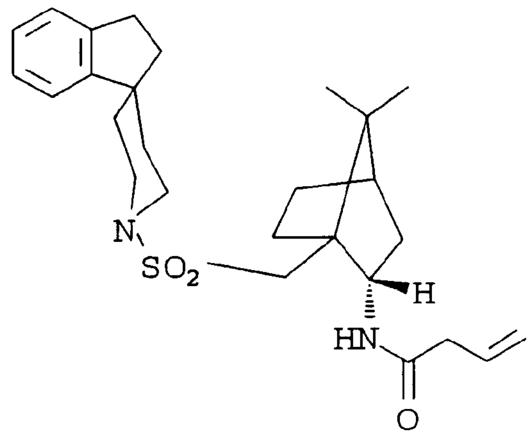

Endo-(1S)-1′-(((2-(but-3ene-1-ylamino)7,7-dimethyl-bicyclo(2.2.1)-hept-1-yl)-methyl)sulfonyl)spiro(1H-indan-1,4′-piperidine)

To a solution of endo-1S-1′-(((2-amino-7,7-dimethylbicyclo(2.2.1.)-hept-1-yl)-methyl)-sulfonyl) spiro(1H-indan-1,4′-piperidine) (50 mg, 0.000125 m) in methylene chloride (3 ml) in an atmosphere of nitrogen was added crotonyl chloride (13.1 mg, 14 ml, 0.000125 M) via a syringe. After two minutes, triethylamine (50 ml) was added to make the solution basic (ph=9). After 1 hour, the reaction mixture was concentrated and the oil was chromatographed on a silica "flash" column using 40% ethyl acetate in hexane as solvent. Fractions 27-40 contained a fluorescent spot. These were concentrated and ether-hexane was added and concentrated to yield the title compound as a white foam.

NMR ($CDCl_3$) was consistent with structure.
HPLC: 210-97.181% 254-99.386%
Mass Spectra: Calculated, m/e=470.679;
Found, m/e=471.3
Analysis for $C_{27}H_{38}N_2O_3S$:
Cal'd: C, 68.90; H, 8.14; N, 5.95
Found: C, 68.99; H, 8.19; N, 5.7

EXAMPLE 2

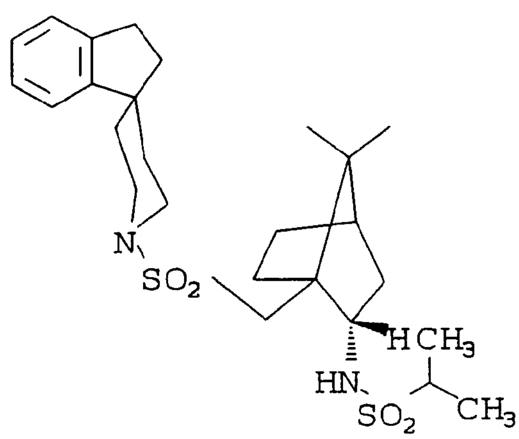

Endo-(1S)-1′-(((2-(dimethylaminosulfonylamino)-7,7-dimethylbicyclo(2.2.1)-hept-1-yl)-methyl)-sulfonyl)spiro(1H-indan-1,4-piperidine)

In an oven-dried flask (50 ml) under nitrogen was dissolved endo-1S-1′-(((2-amino-7,7-dimethylbicyclo(2.2.1.)-hept-1-yl)-methyl)-sulfonyl)-spiro(1H-indan-1,4′-piperidine)(50 mg, 0.000125 M) in methylene chloride (3 ml). Dimethylaminosulfonyl chloride (17.9 mg, 0.000125 M) was added, and after two minutes,

triethylamine (50 ml) was added. After one hour, the reaction mixture was concentrated under reduced pressure (20 mm). A silica "flash" column using methylene chloride(9)-methanol(1) as solvents was used to purify the title compound as a tan solid after adding and concentrating with hexane.
NMR ($CDCl_3$) was consistent with structure.
HPLC: 210-96.47% 254-100%
Mass Spectra: Calculated, 509.736; Found, 510.3
Analysis for $C_{25}H_{39}N_3O_4S_2$:
Cal'd: C, 59.51; H, 7.93; N, 8.04
Found: C, 59.58; H, 7.71; N, 7.87

EXAMPLE 3

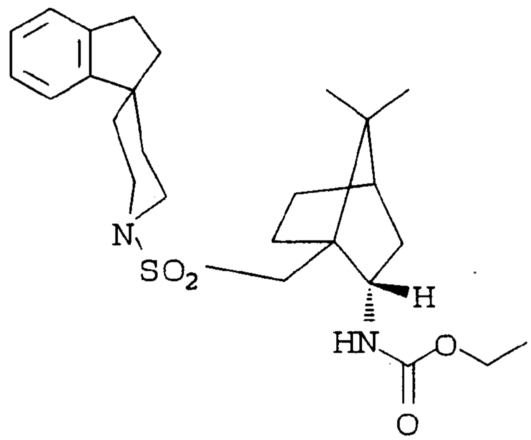

Endo-(1S)-1′-(((2-(ethoxycarbonylamino)-7,7-dimethyl-bicyclo(2.2.1)-hept-1-yl)-methyl)-sulfonyl)spiro(1H-indan-1,4-piperidine)

In an oven-dried flask (50 ml) under nitrogen was dissolved endo-1S-1′-(((2-amino-7,7-dimethyl-bicyclo(2.2.1.)-hept-1-yl)-methyl)-sulfonyl)-spiro(1H-indan-1,4′-piperidine)(50 mg, 0.000125 M) in methylene chloride (3 ml). Ethyl chloroformate (13.6 mg, 12 ml, 0.000125 M) was added via syringe. After two minutes, triethylamine (50 ml) was added to make the solution basic (pH=9). After 30 minutes, the reaction mixture was concentrated under reduced pressure (20 mm). The oil was chromatographed on a silica "flash" column with 40% ethyl acetate in hexane as eluent. Fractions 12-20 contained the product. These fractions were concentrated under reduced pressure (20 mm). Hexane-ether was added to the oil and removed under reduced pressure to yield 17 mg of the title compound as a white powder.
NMR ($CDCl_3$) was consistent with structure.
HPLC 210-99.817% 254-100%
Mass Spectra calculated 474.668 found 475.3
Analysis for $C_{26}H_{38}N_2O_4S$ :
Cal'd: C, 65.79; H, 8.07; N, 5.90
Found: C, 66.05; H, 8.22; N, 5.72

EXAMPLE 4

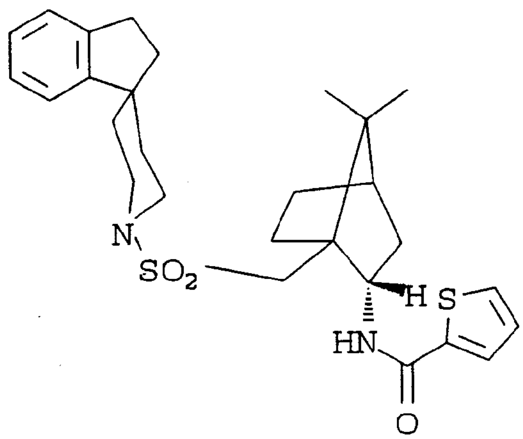

Endo-(1S)-1'-(((2-(2-thiophenecarbonylamino)-7,7-dimethylbicyclo(2.2.1)-hept-1-yl)-methyl)-sulfonyl)spiro(1H-indan-1,4-piperidine)

In an oven-dried flask (50 ml) under nitrogen was dissolved endo-1S-1'-(((2-amino-7,7-dimethylbicyclo(2.2.1.)-hept-1-yl)-methyl)-sulfonyl)-spiro(1H-indan-1,4'-piperidine)(50 mg, 0.000125 m) in methylene chloride (3 ml). The 2-thiophene carbonyl chloride (18 mg, 13.4 ml, 0.000125 m) was added via syringe. Triethylamine (50 ml) was added to make the mixture basic. The reaction mixture was concentrated to oil and chromatographed using a silica "flash" column with 40% ethyl acetate in hexane as eluent. Fractions 12-20 contained the product. Fractions 11-18 contained the desired product and these were concentrated to an oil. Ether-hexane was added and removed to yield 27 mg of the title compound as a white amorphous powder.
HPLC 210-98.873% 254-97/931%
Mass Spectra: Calculated, 512.738; Found, 513.3
Analysis for $C_{28}H_{36}N_2O_3S_2$ 0.72 hexane:
Cal'd: C, 63.97; H, 7.11; N, 5.33
Found: C, 63.59; H, 6.78; N, 5.08

EXAMPLE 5

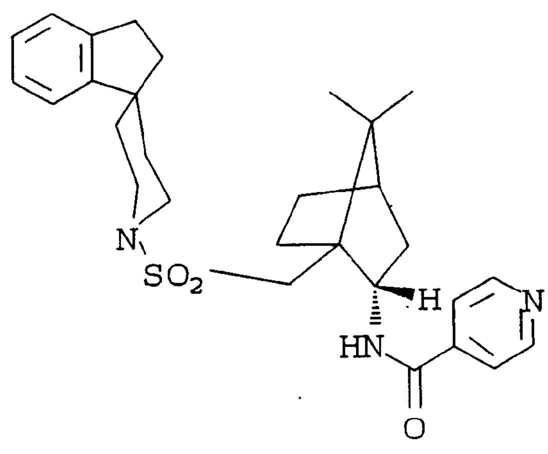

Endo-(1S)-1'-(((2-(isonicotinylamino)-7,7-dimethyl-bicyclo(2.2.1)-hept-1-yl)-methyl)-sulfonyl)spiro(1H-indan-1,4-piperidine)

In an oven-dried flask (50 ml) under nitrogen was dissolved endo-1S-1'-(((2-amino-7,7-dimethylbicyclo(2.2.1.)-hept-1-yl)-methyl)-sulfonyl)-spiro(1H-indan-1,4'-piperidine)(50 mg, 0.000125 m) in methylene chloride (3 ml). The isonicotinoyl chloride dihydrochloride (22.38 mg, 0.000125 m) was added. After 2 minutes, triethylamine (50 ml) was added so the pH equaled 9. The solvent was removed under reduced pressure and

the product was purified by a "flash" silica column using methylene chloride (9)-methanol (1) as solvents. The product was contained in fractions 6-10 which were concentrated. The oil was treated with hexane-ether which was removed under reduced pressure. This gave 18 mg of the title compound as a white foam.
NMR ($CDCl_3$) was consistent with structure.
HPLC 210-95.84% 254-98.04%
Mass Spectra: Calculated, 507.7; Found, 508.3
Analysis for $C_{29}H_{37}N_3O_3S$ :
Cal'd: C, 68.61; H, 7.35; N, 8.28
Found: C, 68.46; H, 6.99; N, 7.90

EXAMPLE 6

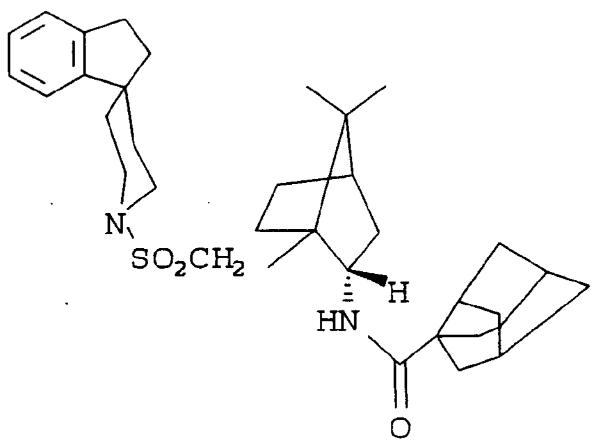

Endo-(1S)-1′-(((2-(noradamantyl-1-carbonylamino)-7,7-dimethylbicyclo(2.2.1)-hept-1-yl)-methyl)-sulfonyl)spiro(1H-indan-1,4-piperidine)

Step 1: Noradamantyl-1-carbonyl chloride

To a solution of noradamantylcarboxylate (20.8 mg, 0.000125 M) in methylene chloride (4 ml) in a round bottom flask (50 ml) under an atmosphere of nitrogen at 0°C was added oxalyl chloride (120 ml) via syringe. Dimethylformamide (7 ml) was added and the solution was stirred at 0°C for 15 minutes. The reaction mixture was allowed to warm to room temperature and stir for 45 minutes. The solvent was removed under reduced pressure (20 mm). Twice additional methylene chloride was added and removed under reduced pressure. The acid chloride of the title compound was kept under high vacuum (0.05 mm) for one hour before using in the next step.

Step 2: Endo-(1S)-1′-(((2-(noradamantyl-1-carbonylamino)-7,7-dimethylbicyclo (2.2.1)-hept-1-yl)-methyl)sulfonyl) spiro(1H-indan-1,4′-piperidine)

Adamantyl-1-carbonyl chloride was dissolved in methylene chloride (4 ml) and endo-1S-1′-(((2-amino-7,7-dimethylbicyclo(2.2.1)-hept-1-yl)-methyl)-sulfonyl)spiro(1H-indan-1,4′-piperidine) (50 mg, 0.000125 m) was added. After two minutes, triethylamine (100 ml) was introduced via syringe. The solvent was removed under reduced pressure and the oil was chromatographed on silica with 40% ethyl acetate in hexane as elutant. The product fractions were collected and concentrated to an oil, which became an amorphous white solid upon addition and removal of ether-hexane. This gave 10 mg of the title compound.
NMR ($CDCl_3$) was consistent with structure.
HPLC 210-98.427% 254-100%
Mass Spectra: Calculated, m/e 550.81; Found, m/e 551.3
Analysis for $C_{33}H_{46}N_2O_3S$ :
Cal'd: C, 71.96; H, 8.42; N, 5.09
Found: C, 71.57; H, 8.71; N, 4.77

EXAMPLE 7

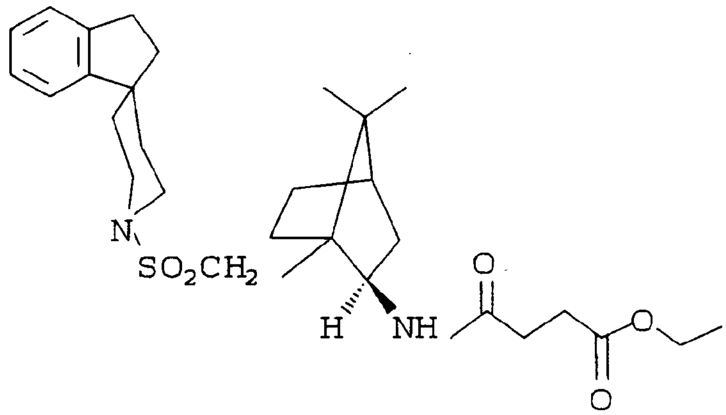

Exo-(1S)-1'-(((2-(ethyl succinoylamino)-7,7-dimethyl-bicyclo(2.2.1)-hept-1-yl)-methyl)-sulfonyl)spiro(1H-indan-1,4'-piperidine)

To a solution of exo-(1S)-1'-(((2-(amino)-7,7-dimethylbicyclo(2.2.1)-hept-1-yl)methyl)-sulfonyl) spiro(1H-indan-1,4'-piperidine) (50 mg, 0.000125 M) in methylene chloride (3 ml) under a blanket of nitrogen was added ethyl succinoyl chloride (20.6 mg, 0.000125 M). After two minutes, triethylamine (50 ml) was added with a syringe. The pH of the solution was 9. After 15 minutes, a new spot was observed on the tlc (silica-40% ethyl acetate in hexane). The reaction mixture was concentrated and the oil was chromatographed on a silica "flash" column with 40% ethyl acetate as solvent. Fractions 24-48 contained the product. These fractions were collected and concentrated. Ether-hexane was added and removed to yield 21.3 mg of the title compound as a white powder. NMR ($CDCl_3$) was consistent with structure.

HPLC 210-99.190% 254-96.914%

Mass Spectra: Calculated m/e, 530.732;
Found m/e, 531.5

Analysis for $C_{29}H_{42}N_2O_5S$ " 0.25 $H_2O$

Cal'd: C, 65.08; H, 8.00; N, 5.23

Found: C, 65.16; H, 8.03; N, 5.09

EXAMPLE 8

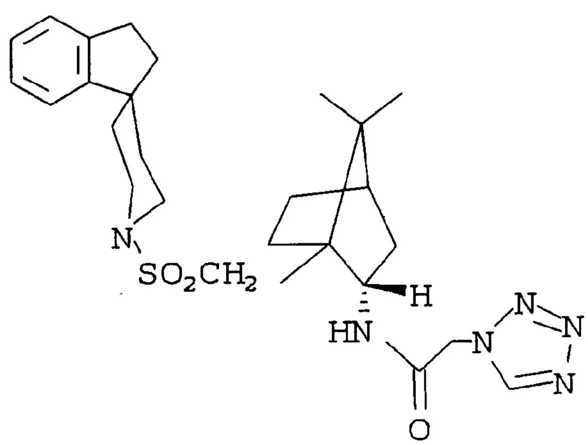

Endo-(1S)-1'-(((2-(tetrazole-1-acetylamino)-7,7-dimethylbicyclo(2.2.1)-hept-1-yl)-methyl)-sulfonyl)-spiro(1H-indan-1,4-piperidine)

In an oven-dried flask (50 ml) under nitrogen was dissolved endo-1S-1'-(((2-amino-7,7-dimethylbicyclo(2.2.1.)-hept-1-yl)-methyl)-sulfonyl)-spiro(1H-indan-1,4'-piperidine)(50 mg, 0.000125 M), tetrazole-1-acetic acid (16 mg, 0.000125 M), and benzotriazol-1-yloxytris(dimethylamino)phosphoniumhexafluorophos-

phate (Sequalog) (55 mg, 0.000125 M) in acetonitrile (3 ml). Diisopropylethylamine (50 ml) was added to make the solution basic. After tlc (silica-methylene chloride(9)-methanol(1)) showed a new spot, the reaction was concentrated to an oil. The oil was dissolved in methylene chloride-ether (1:3) and the solution was washed with water, sodium bicarbonate (sat., aqueous), water, 10% potassium bisulfate, and brine. After drying with sodium sulfate, the solution was filtered and concentrated to oil, which was chromatographed on a silica "flash" column with 10% methanol in methylene chloride. Fractions 5-10 contained the product and were collected and concentrated. Ether-hexane was added and removed to yield the title compound as a white powder.
NMR ($CDCl_3$) was consistent with structure.
HPLC: 210-98% 254-100%
Mass Spectra: Calculated, 513.679; Found, 513.3
Analysis for $C_{26}H_{36}N_6O_3S$ " 0.25 $H_2O$:
Cal'd: C, 60.37; H, 7.11; N, 16.25
Found: C, 60.28; H, 6.96; N, 16.18

EXAMPLE 9

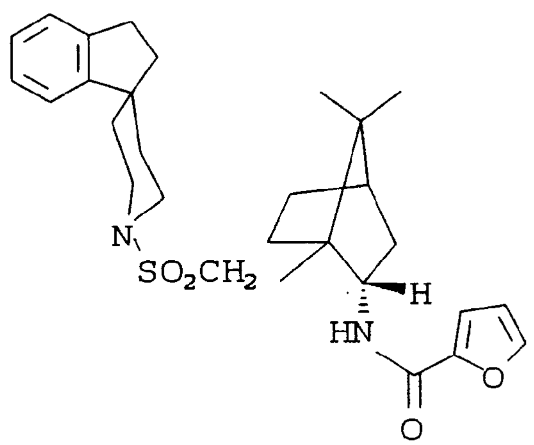

Endo-(1S)-1′-(((2-(2-furoylamino)-7,7-dimethylbicyclo-(2.2.1)-hept-1-yl)-methyl)sulfonyl)spiro(1H-indan-1,4-piperidine)

In an oven-dried flask (50 ml) under nitrogen was dissolved endo-1S-1′-(((2-amino-7,7-dimethylbicyclo(2.2.1.)-hept-1-yl)-methyl)-sulfonyl)-spiro(1H-indan-1,4′-piperidine)(50 mg, 0.000125 M) in methylene chloride (3 ml) and 2-furoyl chloride (16.3 mg, 0.000125 M) was added. After two minutes, triethylamine (150 ml) was added so the pH equaled 9. The solvent was removed under pressure and the product was purified by a silica "flash" column using 40% ethyl acetate in hexane as solvents. The product was contained in fractions 6-10 which were concentrated. The oil was treated with hexane-ether which was removed under reduced pressure.
NMR ($CDCl_3$) was consistent with structure.
HPLC: 210-99.341% 254-99.576%
Mass Spectra: Calculated, 496.674; Found, 497.3
Analysis for $C_{28}H_{36}N_2O_4S$:
Cal'd: C, 67.71; H, 7.31; N, 5.64
Found: C, 67.41; H, 7.39; N, 5.46

EXAMPLE 10

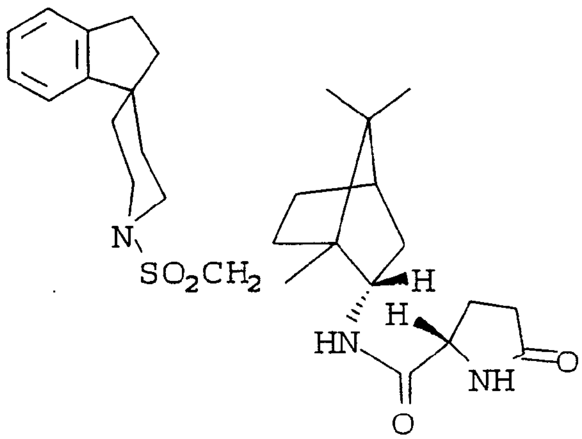

Endo-(1S)-1'-(((2-(S-(1)-2-oxo-pyrrolidin-5-yl-carbonyl)amino)-7,7-dimethylbicyclo(2.2.1)-hept-1-yl)-methyl)-sulfonyl)spiro(1H-indan-1,4-piperedine)

In an oven-dried flask (50 ml) under nitrogen was dissolved endo-1S-1'-(((2-amino-7,7-dimethylbicyclo(2.2.1.)-hept-1-yl)-methyl)-sulfonyl)-spiro(1H-indan-1,4'-piperidine)(50 mg, 0.000125 M), S-(1)-2-oxo-pyrrolidine-5-carboxylic acid (16.1 mg, 0.000125 M), and benzotriazol-1-yloxytris(dimethylamino)phosphonium-hexafluorophosphate (Sequalog) (55 mg, 0.000125 M) in acetonitrile (3 ml). Diisopropylethylamine (50 ml) was added to make the solution basic. After tlc(silica-methylene chloride(9)-methanol(1)) showed a new spot, the reaction was concentrated to an oil. The oil was dissolved in methylene chloride-ether(1:3) and the solution was washed with water, sodium bicarbonate (sat., aqueous), water, 10% potassium bisulfate, and brine. After drying with sodium sulfate, the solution was filtered and concentrated to an oil. Ether-hexane was added and removed under reduced pressure to give the title compound as a white solid.
NMR ($CDCl_3$) was consistent with structure.
HPLC: 210-93.102% 254-92.292%
Mass Spectra: Calculated, 513.705; Found, 514.3

EXAMPLE 11

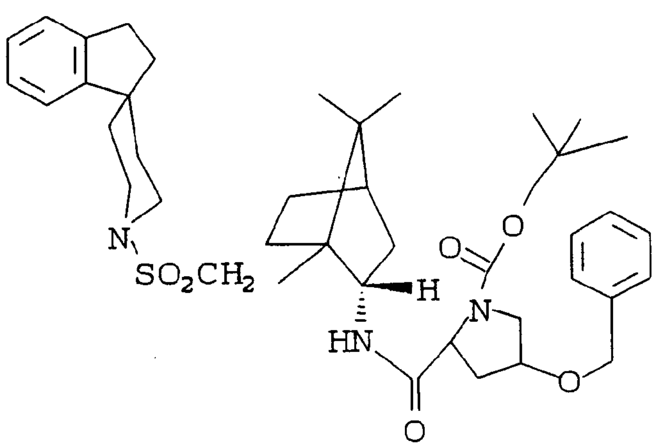

Endo-(1S)-1'-(((2-(L-N-(tert.-butoxycarbonyl)-4-benzyl-oxy-prolinoylamino)-7,7-dimethylbicyclo(2.2.1)-hept-1-yl)-methyl-sulfonyl)spiro(1H-indan-1,4-piperidine)

In an oven-dried flask (50 ml) under nitrogen was dissolved endo-1S-1'-(((2-amino-7,7-dimethylbicyclo(2.2.1.)-hept-1-yl)-methyl)-sulfonyl) spiro(1H-indan-1,4'-piperidine)(50 mg, 0.000125 M), L-N(tert.-butoxycarbonyl)-4-benzyloxy-proline (40.2 mg, 0.000125 M), and benzotriazol-1-yloxytris(dimethylamino)

phosphonium-hexafluorophosphate sequalog (55 mg, 0.000125 M) in acetonitrile (3 ml). Diisopropylethylamine (50 ml) was added to make the solution basic. After tlc(silica-methylene chloride(9)-methanol(1)) showed a new spot, the reaction was concentrated to an oil. The oil was dissolved in methylene chloride-ether(1:3) and the solution was washed with water, sodium bicarbonate (sat., aqueous), water, 10% potassium bisulfate, and brine. After drying with sodium sulfate, the solution was filtered and concentrated to an oil which was purified by "flash" silica chromatography with 40% ethyl acetate in hexane as solvent. Fractions 15-24 contained the product and they were collected and concentrated. Ether-hexane treatment provided a white solid which was the title compound. A 10 mg sample was used for characterization and testing with the remainder being used in subsequent steps.

NMR ($CDCl_3$) was consistent with structure.
HPLC: 210-99.003% 254-100%
Mass Spectra: Cal'd, 705.965;
Found, 706
Analysis for $C_{40}H_{55}N_3O_6S$ " 0.25 $H_2O$:
Cal'd: C, 67.62; H, 7.87; N, 5.91
Found: C, 67.56; H, 8.11; N, 5.59

EXAMPLE 12

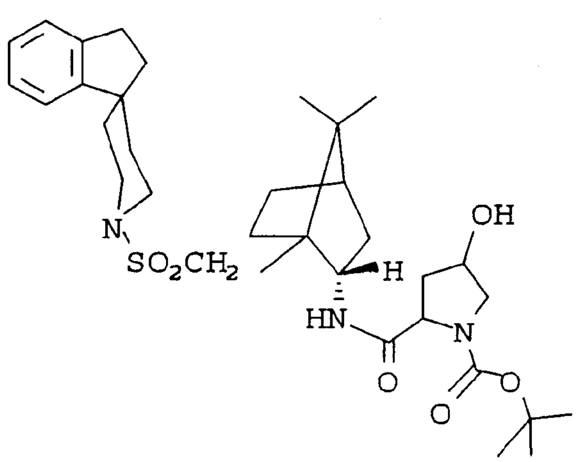

Endo-(1S)-1′-(((2-(L-N(tert.-butoxycarbonyl)-4-hydroxy-prolinoylamiio)-7,7-dimethylbicyclo(2.2.1)-hept-1-yl)methyl-sulfonyl)spiro(1H-indan-1,4-piperidine)

A mixture of Endo-(1S)-1′-(((2-(L-N(tert.-butoxycarbonyl)-4-benzyloxy-prolinoylamino)-7,7-dimethyl)bicyclo(2.2.1)-hept-1-yl)-methyl)-sulfonyl) spiro(1H-indan-1,4-piperidine), ethanol (5 ml), and $Pd(OH)_2$ (60 mg) was hydrogenated on the Parr apparatus at 50 psi for 18 hours. The catalyst was removed by filtration and the solvent was removed under reduced pressure to yield the title compound as a white solid.

NMR ($CDCl_3$) was consistent with structure.
HPLC: 210-93.5% 254-(7.381%
Mass Spectra: Calculated, 615.840; Found, 616
Analysis for $C_{33}H_{49}N_{36}O_6S$ " 1.5 $H_2O$:
Cal'd: C, 61.65; H, 8.15; N, 6.53
Found: C, 61.46; H, 7.88; N, 6.90

EXAMPLE 13

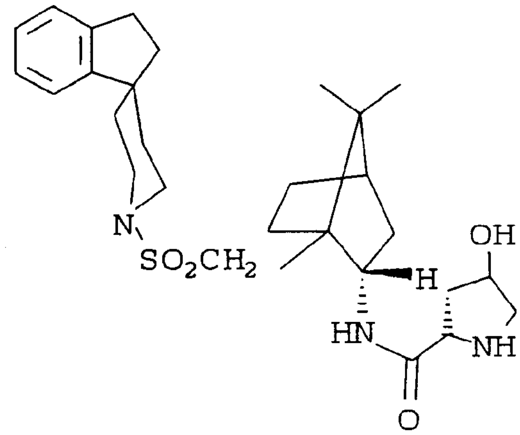

Endo-(1S)-1'-(((2-(L-4-hydroxyprolinoylamino)-7,7-dimethylbicyclo(2.2.1)-hept-1-yl)-methyl)-sulfonyl)spiro(1H-indan-1,4-piperidine)hydrochloride

A mixture of Endo-(1S)-1'-(((2-(L-N(tert.-butoxycarbonyl)-4-hydroxy-prolinoylamino)-7,7-dimethyl)bicyclo(2.2.1)-hept-1-yl)-methyl)-sulfonyl)spiro (1H-indan-1,4-piperidine), was dissolved in ethyl acetate (5 ml) and cooled to -5°C. This was placed under a nitrogen atmosphere and hydrogen chloride gas was bubbled in for 10 minutes. The solvent was removed under reduced pressure. Ether was added and removed under reduced pressure to give 15.48 mg of the title compound as a white solid.

NMR ($CDCl_3$) was consistent with structure.

HPLC: 210-93.50% 254-93.812%

Mass Spectra: Calculated, 515.721 (free base);
Found, 516.4

Analysis for $C_{28}H_{41}N_3O_4S$ HCl 0.25 $H_2O$:

Cal'd: C, 60.41; H, 7.69; N, 7.54

Found: C, 60.08; H, 7.73; N, 7.35

EXAMPLE 14

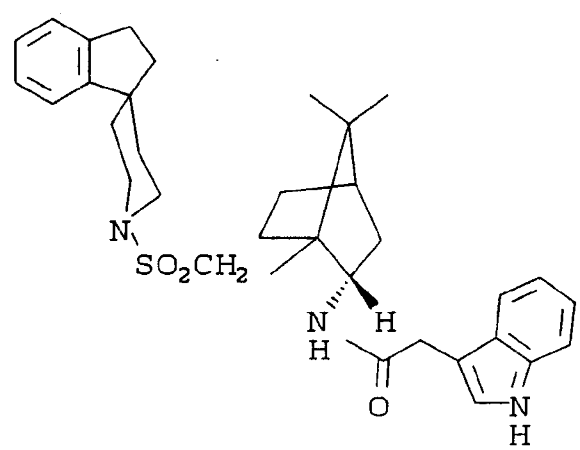

Endo-(1S)-1'-(((2-(3-indolylacetylamino)7,7-dimethyl-bicyclo(2.2.1)-hept-1-yl)methyl-sulfonyl)spiro(1H-indan-1,4-piperidine)

In an oven-dried flask (50 ml) under nitrogen was dissolved endo-1S-1'-(((2-amino-7,7-dimethylbicyclo(2.2.1.)-hept-1-yl)-methyl)-sulfonyl) spiro(1H-indan-1,4'-piperidine)(50 mg, 0.000125 M), 3-indolyl acetic acid (21.9 mg, 0.000125 M), and benzotriazol-1-yloxytris(dimethylamino)phosphoniumhexafluorophosphate (Sequalog) (55 mg, 0.000125 M) in acetonitrile (3 ml). Diisopropylethylamine (50 ml) was added to make the solution basic. After tlc(silica-methylene chloride(9)-methanol(1)) showed a new spot, the reaction was concentrated to an oil. The oil was dissolved in methylene chloride-ether (1:3) and the solution was washed with water, sodium bicarbonate (sat., aqueous), water, 10% potassium bisulfate, and brine. After drying with sodium sulfate, the solution was filtered and concentrated to an oil which was purified by "flash" silica chromatography with 10% methanol in methylene chloride as solvent. Fractions 12-19 contained the product and they were collected and concentrated. Ether-hexane treatment provided a white solid which was the title compound.

MR ($CDCl_3$) was consistent with structure.

HPLC: 210-99.829% 254-92.699%

Mass Spectra: Calculated, 559.777; Found, 560.3

Analysis for $C_{33}H_{41}N_3O_3S$ " 0.5 $H_2O$:

Cal'd: C, 69.69; H, 7.44; N, 7.51

Found: C, 69.70; H, 7.57; N, 7.20

EXAMPLE 15

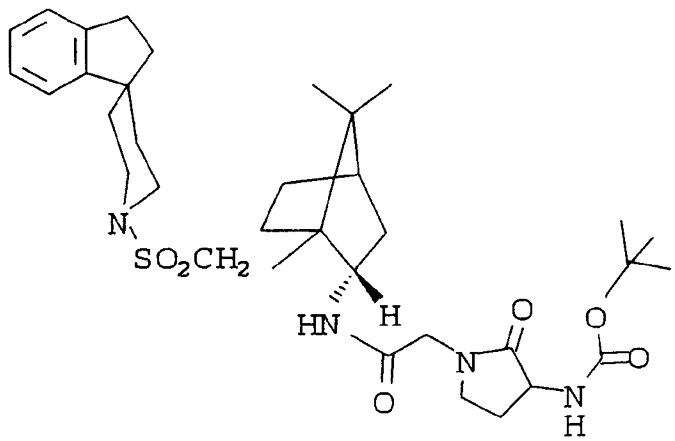

Endo-(1S)-1'-(((2-(S-3-(tert.-butoxycarbonyl)amino)-2-oxo-1-pyrrolidineacetylamino)7,7-dimethylbicyclo-(2.2.1)hept-1-yl)-methyl-sulfonyl)spiro(1H-indan-1,4-piperidine)

In an oven-dried flask (50 ml) under nitrogen was dissolved endo-1S-1'-(((2-amino-7,7-dimethylbicyclo(2.2.1.)-hept-1-yl)-methyl)-sulfonyl) spiro(1H-indan-1,4'-piperidine)(50 mg, 0.000125 M), S-3-((tert.-butoxycarbonyl)amino)-2-oxo-1-pyrrolidine acetic acid (32.2 mg, 0.000125 M), and benzotriazol-1-yloxytris (dimethylamino)phosphoniumhexafluorophosphate (Sequalog) (55 mg, 0.000125 M) in acetonitrile (3 ml). Diisopropylethylamine (50 ml) was added to make the solution basic. After tlc(silicamethylene chloride(9)-methanol(1)) showed a new spot, the reaction was concentrated to an oil. The oil was dissolved in methylene chloride-ether(1:3) and the solution was washed with water, sodium bicarbonate (sat., aqueous), water, 10% potassium bisulfate, and brine. After drying with sodium sulfate, the solution was filtered and concentrated to an oil which was purified by "flash" silica chromatography with 10% methanol in methylene chloride as solvent. Fractions 15-28 contained the product and they were collected and concentrated. Ether-hexane treatment provided a white solid which was the title compound.

NMR ($CDCl_3$) was consistent with structure.

HPLC: 210-94.005% 254-96.33%

Mass Spectra: Calculated m/e, 642.859; Found m/e, 643.4

Analysis for $C_{34}H_{50}N_4O_6S$ " 0.1 hexane:

Cal'd: C, 63.79; H, 7.95; N, 8.59

Found: C, 63.66; H, 8.32; N, 8.29

EXAMPLE 16

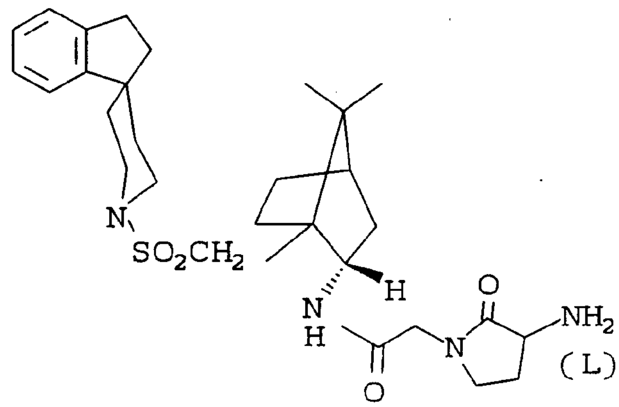

Endo-(1S)-1′-(((2-(S-3-amino)-2-oxo-1-pyrrolidine-acetylamino)7,7-dimethylbicyclo(2.2.1)-hept-1-yl)-methyl)-sulfonyl)spiro(1H-indan-1,4-piperidine) hydrochloride

Endo-1S-1′-(((2-(S-3-(tert.-butoxycarbonyl) amino)-2-oxo-1-pyrrolidineacetylamino-)-7,7-dimethyl-bicyclo(2.2.1.)hept-1-yl)-methyl)-sulfonyl)spiro (1H-indan-1,4′-piperidine) was dissolved in ethyl acetate (5 ml) and cooled to -5°C. This was placed under a nitrogen atmosphere and hydrogen chloride gas was bubbled in for 10 minutes. The solvent was removed under reduced pressure. Ether was added and removed under reduced pressure to give 23.7 mg of the title compound as a white solid.

NMR ($CDCl_3$) was consistent with structure.

HPLC: 210-96.562% 254-95.493%

Mass Spectra: Calculated for free base m/e, 542;
Found m/e, 543.3

Analysis for $C_{29}H_{42}N_4O_4S$ " $H_2O$ " HCl:

Cal'd: C, 58.32; H, 7.59; N, 9.38

Found: C, 58.14; H, 7.97; N, 9.21

EXAMPLE 17

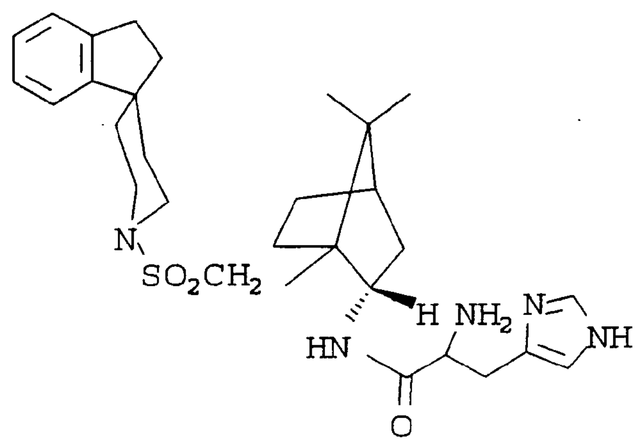

Step 1: Endo-(1S)-1′-(((2-(L-N-tertbutoxycarbonylhistidinoylamino)-7,7-dimethylbicyclo(2.2.1)-hept-1-yl)-methyl-sulfonyl)spiro(1H-indan-1,4-piperidine)

In an oven dried flask (50 ml) under nitrogen were dissolved endo-1S-1′-(((2-amino-7,7-dimethylbicyclo(2.2.1.)hept-1-yl)-methyl)-sulfonyl)spiro(1H-indan-1,4′-piperidine) (50 mg, 0.000125 M), L-N-tert, butoxycarbonyl histidine (29.9 mg, 0.000125 M), and benzotriazol-1-yloxytris(dimethylamino)phosphoniumhexafluorophosphate (Sequalog) (55 mg, 0.000125 M) in acetonitrile (3 ml). Diisopropylethylamine (50 ml) was added to make the solution basic. After tlc(silica-methylene chloride(9)-methanol(1)) showed a new spot, the reaction was concentrated to an oil. The oil was dissolved in methylene chloride-ether (1:3) and the

solution was washed with water, sodium bicarbonate (sat., aqueous), water, 10% potassium bisulfate, and brine. After drying with sodium sulfate, the solution was filtered and concentrated to an oil, which was purified by "flash" silica chromatography with 10% methanol in methylene chloride as solvent. Fractions 15-28 contained the product and they were collected and concentrated. Ether-hexane treatment provided a white solid which as the title compound.

NMR ($CD_3OD$) was consistent with structure.
HPLC: 210-97.822% 254-100%
Mass Spectra: Calculated m/e, 639.859;
Found m/e, 640.3

Step 2: Endo-(1S)-1′-(((2-(L-N-histidinoylamino)-7,7-dimethylbicyclo(2.2.1)-hept-1-yl)-methylsulfonyl)spiro(1H-indan-1,4-piperidine)

Endo-1S-1′-(((2-(L-N-tert.butoxycarbonylhistidinoylamino)-7,7-dimethylbicyclo(2.2.1.)-hept-1-yl)-methyl)-sulfonyl)spiro(1H-indan-1,4′-piperidine) (50 mg, 0.000125 M) was dissolved in ethyl acetate (5 ml) and cooled to -5°C. This was placed under a nitrogen atmosphere and hydrogen chloride gas was bubbled in for 10 minutes. The solvent was removed under reduced pressure. Ether was added and removed under reduced pressure to give 27.5 mg of the title compound as a white solid.

NMR ($CD_3OD$) was consistent with structure.
HPLC: 210-98.652% 254-100%
Mass Spectra: Calculated for free base m/e, 539.7;
Found m/e, 540.3
Analysis for $C_{29}H_{41}N_5O_3S$ 2HCl $2H_2O$:
Cal'd: C, 53.69; H, 7.30; N, 10.79
Found: C, 53.58; H, 7.45; N, 11.00

EXAMPLE 18

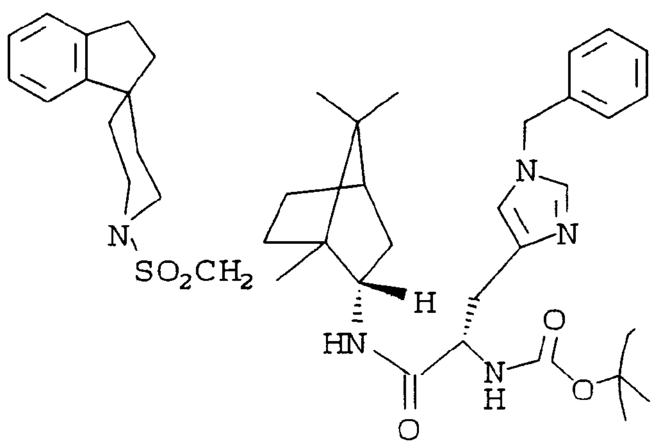

Endo-(1S)-1′-(((2-(D-N-(tert.butoxycarbonyl)-im-benzyl histidinoylamino)-7,7-dimethylbicyclo(2.2.1)-hept-1-yl)-methyl)sulfonyl)spiro(1H-indan-1,4′-piperidine)

In an oven-dried flask (50 ml) under nitrogen were dissolved endo-1S-1′-(((2-amino-7,7-dimethylbicyclo(2.2.1.)-hept-1-yl)-methyl)-sulfonyl) spiro(1H-indan-1,4′-piperidine) (50 mg, 0.000125 M), D-N-tert. butoxycarbonylim-benzyl-histidine (43 mg, 0.000125 M), and benzotriazol-1-yloxytris(dimethylamino)phosphoniumhexafluorophosphate (Sequalog) (55 mg, 0.000125 M) in acetonitrile (3 ml). Diisopropylethylamine (50 ml) was added to make the solution basic. After tlc(silica-methylene chloride(9)-methanol(1)) showed a new spot, the reaction was concentrated to an oil. The oil was dissolved in methylene chloride-ether (1:3) and the solution was washed with water, sodium bicarbonate (sat., aqueous), water, 10% potassium bisulfate, and brine. After drying with sodium sulfate, the solution was filtered and concentrated. Ether-hexane provided a white solid which was the title compound.

NMR ($CDCl_3$) was consistent with structure.
HPLC: 210-99.551%
Mass Spectra: Calculated, m/e 730.062

Found, m/e 730.6
Analysis for $C_{41}H_{55}N_5O_5S$: 0.65 $H_2O$ 0.4 hexane:
Cal'd: C, 67.15; H, 8.04; N, 9.02
Found: C, 67.18; H, 7.73; N, 9.02

EXAMPLE 19

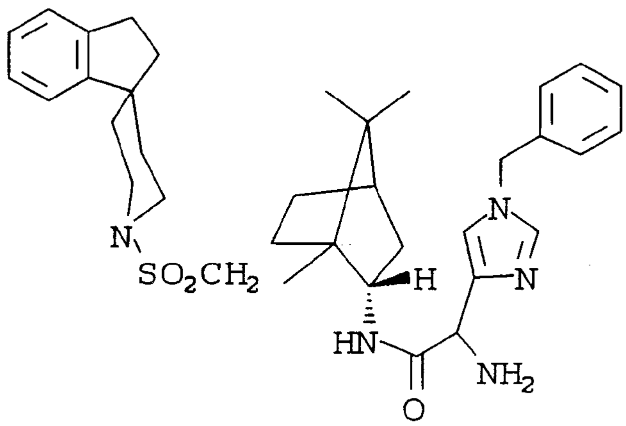

Endo-(1S)-1'-(((2-(D-im-benzylhistidinoylamino)-7,7-dimethylbicyclo(2.2.1)-hept-1-yl)-methyl)-sulfonyl)spiro(1H-indan-1,4-piperidine)hydrochloride

Endo-(1S)-1'-(((2-(D-N-(tert.butoxycarbonyl)-im-benzylhistidinoylamino)-7,7-dimethylbicyclo(2.2.1.)-hept -1-yl)-methyl)-sulfonyl)spiro(1H-indan-1,4'-piperidine) was dissolved in ethyl acetate (5 ml) and cooled to -5°C. This was placed under a nitrogen atmosphere and hydrogen chloride gas was bubbled in for 10 minutes. The solvent was removed under reduced pressure. Ether was added and removed under reduced pressure to give the title compound as a white solid.

NMR ($CDCl_3$) was consistent with structure.
HPLC: 210-100%
Mass Spectra: Calculated m/e, 629.879; Found m/e, 630
Analysis for $C_{36}H_{47}N_5O_3S$
HCl 0.1 ethyl acetate 1.4 $H_2O$:
Cal'd: C, 59.33; H, 7.20; N, 9.51
Found: C, 59.36; H, 7.03; N, 9.50

EXAMPLE 20

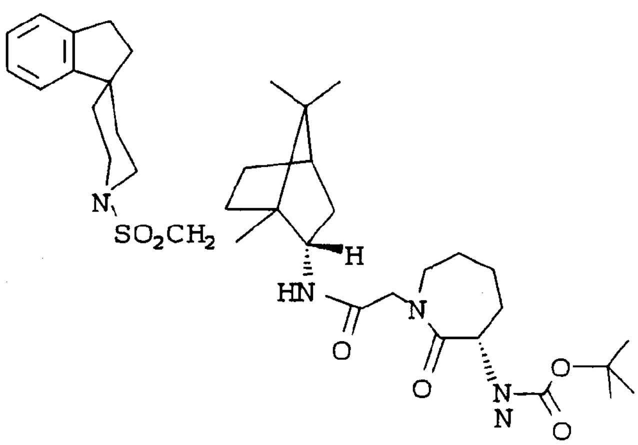

Endo-(1S)-1′(((2-(S-3-tert.butoxycarbonylamino-2-oxo-1-azepineacetylamino)-7,7-dimethylbicyclo(2.2.1)-hept-1-yl)-methyl-sulfonyl)spiro(1H-indan-1,4-piperidine)

In an oven-dried flask (50 ml) under nitrogen was dissolved endo-1S-1′-(((2-amino-7,7-dimethyl-bicyclo(2.2.1.)-hept-1-yl)-methyl)-sulfonyl) spiro(1H-indan-1,4′-piperidine)(50 mg, 0.000125 M), S-3-tert.butoxycarbonylamino-2-oxo-1-azepine acetic acid (32.8 mg), and benzotriazol-1-yloxytris (dimethylamino)phosphonium-hexafluorophosphate sequalog (55 mg, 0.000125 M) in acetonitrile (3 ml). Diisopropylethylamine (50 ml) was added to make the solution basic. After tlc(silica-methylene chloride(9)-methanol(1)) showed a new spot, the reaction was concentrated to an oil. The oil was dissolved in methylene chloride-ether(1.3) and the solution was washed with water, sodium bicarbonate (sat., aqueous), water, 10% potassium bisulfate, and brine. After drying with sodium sulfate, the solution was filtered and concentrated to an oil, which was purified by "flash" silica chromatography with 10% methanol in methylene chloride as solvent. The fractions which contained the product were collected and concentrated. Ether-hexane treatment provided a white solid which was the title compound. A 10 mg sample was saved for characterization and testing.

NMR ($CDCl_3$) was consistent with structure.
HPLC 210-100%
Mass Spectra calculated m/e 670.918 found m/e 671
Analysis for $C_{36}H_{54}N_4O_6S$ :

| | |
|---|---|
| Cal'd: | C, 64.44; H, 8.11; N, 8.35 |
| Found: | C, 64.12; H, 8.23; N, 8.03 |

EXAMPLE 21

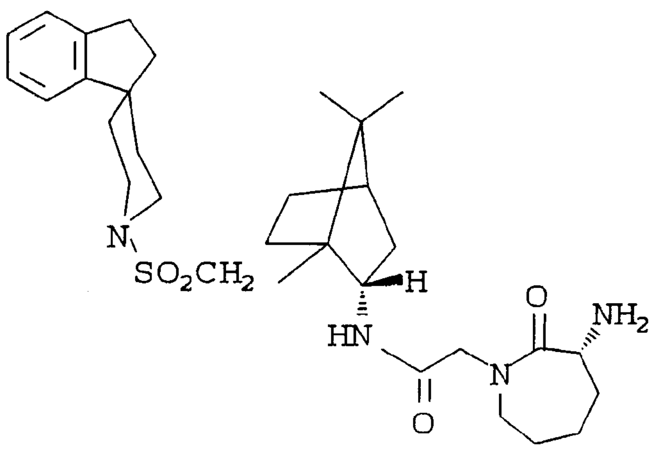

Endo-(1S)-1′-(((2-(S-3-amino-2-oxo-1-azepine acetylamino)-7,7-dimethylbicyclo(2.2.1)-hept-1-yl-methyl)-sulfonyl)spiro(1H-indan-1,4′-piperidine) hydrochloride

Endo(1S)-1′-(((2-(S-3-tert-butoxycarbonylamino-2-oxo-1-azepine acetylamino)-7,7-dimethylbicylo-(2.2.1.)-hept-1-yl)-methyl)-sulfonyl)spiro(1H-indan-1,4′-piperidine) was dissolved in ethyl acetate (5 ml) and cooled to 5°C. This was placed under a nitrogen atmosphere and hydrogen chloride gas was bubbled in for 10 minutes. The solvent was removed under reduced pressure. Ether was added and removed under reduced pressure to give the title compound as a white solid.

NMR($DCDl_3$) was consistent with structure.
HPLC 210-100%
Mass Spectra: Calculated m/e, 570.802; Found m/e, 571
Analysis for $C_{31}H_{46}N_4O_4S$ " HCl 1.10 $H_2O$ " 0.40 ethyl acetate:

| | |
|---|---|
| Cal'd: | C, 59.11; H, 7.97; N, 8.46 |
| Found: | C, 59.09; H, 8.00; N, 8.45 |

EXAMPLE 22

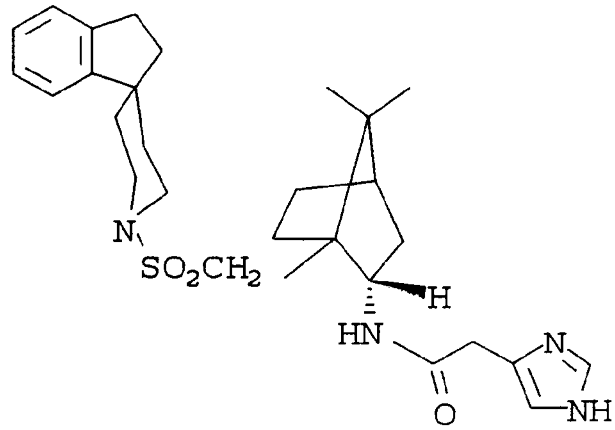

Endo-(1S)-1′-(((2-(4-imidazoleacetylamino)-7,7-dimethylbicyclo(2.2.1)-hept-1-yl)-methyl)-sulfonyl) spiro(1H-indene-1,4′-piperidine)hydrocloride

N,N-bis(2-chloroethyl)-t-butylcarbamate

Di-t-butyldicarbonate (62 g, 0.28 mole, available from Aldrich) and bis(2-chloroethyl)amine hydrochloride (55 g, 0.21 mole ,available from Aldrich) were stirred together in methylene chloride (400 mL). Triethylamine (42 mL, 0.3 mole) was added dropwise but briskly to the stirred suspension. After 10 minutes, additional triethylamine (ca. 5-6 mL) was added to adjust the pH of the mixture (as determined by spotting a sample on E. Merck pH 5-10 colorpHast sticks, moistened with water) to 9-9.5. The mixture was stirred for 1 hour at ambient temperature, then filtered. The filtrate was divided in half, and each half (ca. 300 mL) was flash chromatographed on a separate 15″, 2″diameter silica column packed with 3:2 (v:v) methylene chloride:hexane and eluted with 9:1 methylene chloride:hexane. 100 mL fractions were taken, and the product was obtained in fractions ca. 6-12. Assay was by TLC on silica gel plates, eluted with methylene chloride and visualized with phosphomolybdic acid stain ($R_f$ ca. 0.75). Evaporation of the combined product fractions from both columns in vacuo provided the title compound (70 g) as a colorless oil. The oil was twice dissolved in dry THF and evaporated in vacuo to remove methylene chloride.

1′-(t-Butyloxycarbonyl)spiro(1H-indene-1,4′-piperidine)

To a solution of indene (36.2 g, 310 mmole) in dry tetrahydrofuran (THF, 40 mL) cooled in an ice bath and maintained under a nitrogen blanket was added (dropped funnel) lithium bis(trimethylsilyl)amide (620 mL of a 1.0 M solution in THF; 620 mmole, available from Aldrich) over 30 minutes. The mixture was stirred in the cold for 30 minutes, then transferred by cannula over 20 minutes to a solution of N,N-bis(2-chloroethyl)-t-butylcarbamate (70 g, 290 mmole) in THF (40 mL), stirred in an ice bath. The mixture was stirred for 2 hours in the cold and for 30 minutes at ambient temperature under nitrogen, then evaporated in vacuo to a foam. $CH_2Cl_2$ (400 mL) was added and the resulting mixture divided in half. Each half was poured onto a silica gel column (15″, 2″ id) packed and eluted with 1:1 hexane:$CH_2Cl_2$ (2 L) followed by 1:4 hexane: $CH_2Cl_2$. The product fractions (fractions 3-9 of 200 mL fractions) were evaporated to dryness in vacuo to provide 1′-(t-butyloxycarbonyl)spiro (indene-1,4′piperidine), 90 g total, as a crude yellow solid. The solid was taken up in boiling hexane (400 mL), cooled, and the crystallized solid filtered. The filtrate was repeatedly boiled down to 1/2 its volume, cooled and filtered to obtain successive crops, providing a total of 54 g of pure product. The residue was rechromatographed to provide another 6.7 g (60.7 g total).

Spiro(1H-indene-1,4′-piperidine) hydrochloride

1′-(t-Butyloxycarbonyl)spiro(indene-1,4′-piperidine) (60.7 g) in ethyl acetate (700 mL) was stirred in an ice bath and saturated with HCl (g) for 30 minutes, keeping the internal temperature ≤ 12°C. The mixture was stirred in the cold an additional 30 minutes, then evaporated to dryness. Ethyl acetate was added and removed in vacuo three times, and the residue was triturated with diethyl ether and filtered to provide spiro(1H-indene-1,4′-piperidine) hydrochloride.

(1S)-1′-(((7,7-dimethyl-2-oxobicyclo(2.2.1)hept-1-)methyl)-sulfonyl)spiro(1H-indene-1,4′-piperidine)

Spiro(1H-indene-1,4′piperidine) hydrochloride (45.4 g, 0.2 mole) and (+)-10-camphorsulfonyl chloride (62.5 g, 0.25 mole, available from Aldrich) were combined in $CH_2Cl_2$ (700 mL) and treated with triethylamine (68.5 mL, 0.5 mole). Additional triethylamine was added as needed to adjust the pH of the mixture to 9-9.5 (moistened E. Merck colorpHast sticks). The mixture was stirred at ambient temperature for 1 hour, then poured onto a silica gel column (10″, 2″ id) packed with $CH_2CL_2$ and eluted with 1:1 $Et_2O$:$CH_2Cl_2$. The product fractions were combined and evaporated to dryness in vacuo to provide the title compound as a solid which was recrystallized from petrolium ether and dried 6 hours in vacuo at ambient temperature: (m.p. 146-147°C).

TLC: Rf=0.44 silica gel ($CH_2Cl_2$).
NMR: Consistent with structure.
HPLC: >99.7% pure.
MS: Molecular ion at m/e - 399
Analysis for $C_{23}H_{29}NO_3S$:

| | |
|---|---|
| Cal'd: | C, 69.14; H, 7.32; N, 3.51 |
| Found: | C, 68.97; H, 7.2; N, 3.38 |

(1S)-1′-(((7,7-dimethyl-2-oximinobicyclo(2.2.1)hept-1-yl)-methyl)sulfonyl)spiro(1H-indene-1,4′-piperidine)

(1S)-1′-(((7,t-dimethyl-2-oxobicyclo (2.2.1)hept-1-yl)methyl)sulfonyl)spiro(1H-indene-1,4′piperidine) (30 g, 0.075 mole) in pyridine (500 mL) was heated in an oil bath to 70°C (internal). Hydroxylamine hydrochloride (30 g) was added in three portions over ca. 20 minutes. After 2 hours, an additional 10 g of hydroxylamine hydrochloride was added (over 10 minutes). At 30, 40 and 50 minutes additional elapsed time, further 3 g lots of hydroxylamine hydrochloride were added. After another 30 minutes, the mixture was poured into water (2 L) and extracted 3X with ethyl acetate (300 mL portions). The organic layers were combined, washed with 1N HCl (600 mL total), dried over sodium sulfate, filtered, and evaporated to dryness in vacuo. EtOH (abs; ca. 250 mL) was added to the resulting thick syrup and the solution allowed to stand at ambient temperature overnight. The mixture was filtered and the filtrate boiled down to ca. 80 mL. After standing, the mixture was again filtered and boiled down to ca. 20 mL. After a third filtration, the filtered solids were combined to give the title compound (28 g).

Endo-(1S)-1′(((2-amino-7,7-dimethylbicyclo(2.2.1)hept-1-yl)-methyl)sulfonyl)spiro(1H-indene-1,4′-piperidine)

Freshly prepared, activated Raney Nickel catalyst (ca. 30 g) in water was allowed to settle and the water decanted. Abs. ethanol (300 mL) was added, and the mixture swirled and again allowed to settle. The solvent was decanted. Two more wash-decant cycles with 150 mL of ethanol were similarly carried out. (1S)-1′(((7,7-dimethyl-2-oximinobicyclo (2.2.1)hept-1-yl)methyl)sulfonyl)spiro(1H-indene-1,4′-piperidine) (30 g) was stirred in a mixture of abs. ethanol (450 mL) and 2-methoxyethanol (900 mL), nitrogen was bubbled through the suspension/solution, and the Raney Nickel catalyst was added. The mixture was hydrogenated under 50 psi overnight. TLC (9:1 $CH_2Cl_2$:-MeOH, silica gel) showed the reaction to be complete. The catalyst was removed by filtration, and the filtrate evaporated to dryness in vacuo. The crude solid (27 g) was divided into 7 g batches, and each batch was dissolved in methylene chloride (ca. 200 mL) and flash chromatographed on silica (700 g in a 100 mm column, packed and eluted with 8% (v/v) methanol in methylene chloride), taking 200 mL fractions. The exo isomer of the title amine was obtained in fractions ca. 5-7, and the desired endo isomer in fractions ca. 8-16. TLC was on silica, eluted with 8% methanol/methylene chloride, phosphomolybdic acid stain. The combined product fractions were evaporated to dryness to provide the title compound (4.5 g from each 7 g lot, ca 18 g total) as a colorless solid.

Endo-(1S)-1′(((2-(4-imidazoleacetylamino)-7,7-dimethylbicyclo-(2.2.1)-hept-1-yl)methyl)sulfonyl) spiro(1H-indene-1,4′-piperidine hydrochloride

4.56 g (11.3 mmols) of Endo-(1S)-1′(((2-amino-7,7-dimethylbicyclo-(2.2.1)hept-1-yl)methyl) sulfonyl)spiro(1H-indene-1,4′-piperidine) was dissolved in 50 mL DMF and the solution treated with 2.30 g (14.1 mmols) of 4-imidazole acetic acid, 1.9 g (14.1 mmols) of 1-hydroxybenzotriazole hydrate (HBT), and 2.7 g (14.1 mmols) of 1-ethyl-3-(3-dimethylaminopropyl) carbodiimide HCl (EDC). The pH of the suspension was adjusted to 9.5 with 4.65 mL (33.4 mmols) of triethylamine and the reaction mixture stirred at 25°C for 18 hours.

DMF was removed in vacuo and the crude purplish residue treated with water and extracted with EtOAc (3X). The organics were combined, washed with $H_2O$ (1X), brine (1X), dried over $Na_2SO_4$, filtered and stripped

to dryness in vacuo. Flash chromatography of the crude product on silica gel (114/10/1 of $CH_2Cl_2$/MeOH/conc. $NH_4OH$) gave 5.29 g (92%) of desired product as a white foam.

5.17 g (10.1 mmols) of this product was dissolved in 100 mL EtOAc. While stirring vigorously, a solution of HCl (g) in EtOAc was added dropwise until precipatation ceased. The slightly gummy mixture was stirred 15 minutes at 25°C, then evaporated to dryness in vacuo. The residue was restripped 3X from EtOAc, then 3X from $Et_2O$. The white solid was scraped from the walls of the flask, triturated with $Et_2O$ and 5.3 g of hydrochloride salt collected.

M.P.: 93-167°C (slow foam)
HPLC: 99.4%
PMR: Consistent with structure, plus 0.30 ethyl acetate, 0.05 ether and $H_2O$.
M.S.: M+H + 511 (FAB)
Analysis for $C_{28}H_{38}N_4O_3S$ " HCl " 0.30 $C_4H_8O_2$ " 0.05 $C_4H_{10}O$ " 0.4
$H_2O$. (M.W. 584.48):
Cal'd: C, 60-41; H, 7.36; N, 9.59
Found: C, 60.38; H, 7.46; N, 9.33

EXAMPLE 23

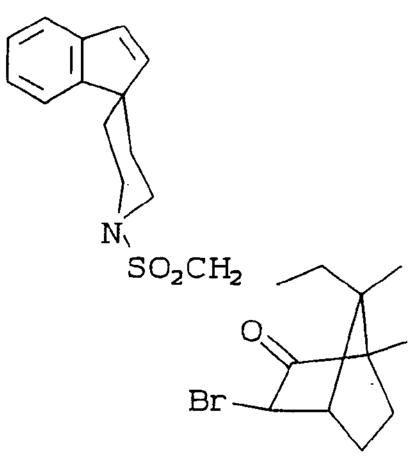

1-[[6-bromo-1,7-dimethyl-2-oxobicyclo[2.2.1]hept-7-yl)methyllsulfonyl]-spiro[1H-indene-1,4'-piperidine]

Dissolved 100 mg of (+)-3-Bromocamphorsulfonic acid ammonium salt (.304 mM) in 15 mL of DML. Added 5 eq of $SOCl_2$ (FW=118.97; d=1.631; 1.52 mM; 120 mL). The mixture was allowed to react overnight, then concentrated to obtain 92.7 mg of the crude sulfonyl chloride. The 92.7 mg of sulfonyl chloride was dissolved in 25 mL of dry THF. To this was added 1.1 eq of indene salt (68.59 mg) and 2 eq of TFA, which was allowed to react at room temperature for three hours. The product was concentrated and washed with HCl, $H_2O$ and brine. Flask chromatography in 20% EtOAc/petroleum ether.
HPLC 97.7% at 13.52 min.

EXAMPLE 24

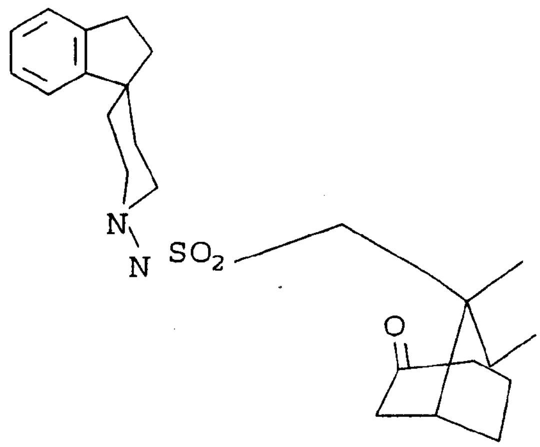

(1R-syn)-1'-[[(1,7-dimethyl-2-oxobicyclo[2.2.1]hept-7-yl)methyl)sulfonyl]-spiro[1H-indene-1,4'-piperidine]

To a solution of the product of Example 23 (107 mg, 0.300 mmol) in glacial acetic acid (10 mL) was added zinc dust (24 mg, 0.367 mmol). The temperature was then increased to reflux. After 30 minutes, the mixture was cooled to room temperature, filtered, then concentrated under reduced pressure. Purification by flash chromatography (35% ethyl acetate in petroleum ether as eluent) afforded 80 mg of the product as a white amorphous foam.
NMR (300 MHz, $CDCl_3$): Consistent with structure.
HPLC (Vydac C18 column, gradient from 95/5 to 0/100 $H_2O/CH_3CN$ with 0.1% TFA, 15 min. gradient, flow rate = 1.5 mL/min): purity = 98%, r.t. = 12.67 min.
FABMS: $[M+1]^+$] at 400.91
Analysis Cal'd for $C_{23}H_{29}NO_3S$:
Cal'd: C, 69.13; H, 7.32; N, 3.51
Found: C, 69.25; H, 7.30; N, 3.48

EXAMPLE 25

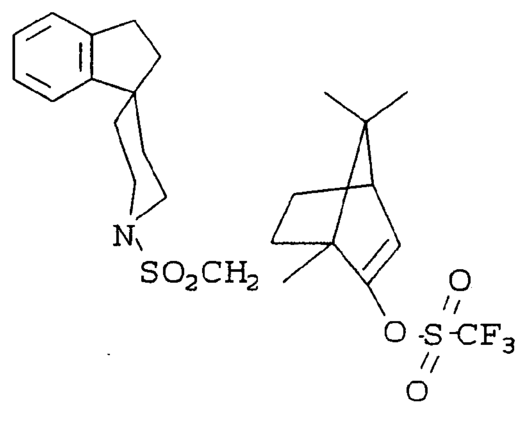

(1S)-1'(((2-(trifluoromethyl)sulfonyl)oxy)2,3-ene-7,7-dimethylbicyclo(2.2.1) hept-1-yl)methyl)sulfonyl)-spiro(1H-indene-1,4'-piperidine)

To a solution of (1S)-1(((7,7-dimethyl-2-bicyclo(2.2.1)hept-1-yl)methyl)sulfonyl)spiro(1H-indene-1,4'-piperidine) (2g, 5.0 mmol) and 2,6-ditert-butyl-4-methylpyridine (1.5 g, 7.5 mmol) in $CH_2Cl_2$ (25 ml) was added triflic anhydride (1.3 ml, 7.5 mmol) and the mixture was stirred at room temperature for 30 minutes. The mixture was then diluted with $CH_2Cl_2$ (30 ml) and filtered. The filtrate was then washed with 5% HCl (2x50 ml), saturated $NaHCO_3$ (2x50 ml), and brine, dried over $Na_2SO_4$, and evaporated.

The crude triflate was purified by flash chromatography eluting with 20% ethylacetate in hexanes to yield

1.7 g of the title product as a white foam (64%).
HPLC RT = 12.75 min.
NMR ($CDCl_3$) in agreement with title compound.
FAB MS: 532 (M+1)
Analysis Cal'd for $C_{42}H_{28}NS_2O_5F_3$:

| | |
|---|---|
| Cal'd: | N, 2.63, C, 54.22, H, 2.63 |
| Found: | N, 2.37; C, 54.42; H, 5.34 |

EXAMPLE 26

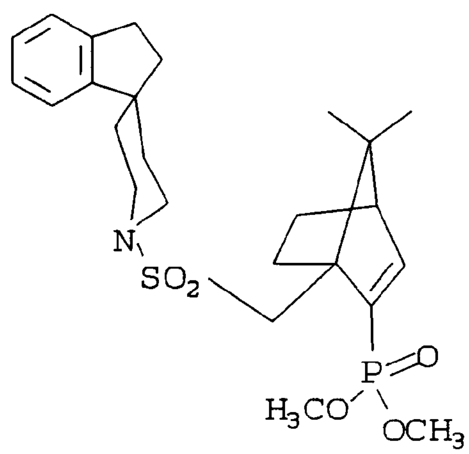

(1S)-1'-(((2-(dimethylphosphonyl)oxy)2,3-ene-7,7-dimethylbicyclo(2.2.1)hept-1-yl)sulfonyl)spiro(1H-indene-1,4'-piperidine)

A mixture of the triflate product of Example 25 (50 mg, 0.097 mmol), dimethyl phosphite (13 ml, 0.14 mmol), triethylamine (61 ml, 0.44 mmol), and tetrakis(triphenylphosphine)palladium (5 mg, 0.005 mmol) in DMF (3 ml) was stirred under an atmosphere of argon for 1 hour. The reaction was then diluted with $CHCl_3$ (25 ml). The chloroform was washed with 5% HCl (2x25 ml) and brine, dried over $Na_2SO_4$ and evaporated to dryness. Purification via flash chromatography (25% ethylacetate in hexanes) afforded 40 mg of the title compound as a white solid (86%).
PLC RT = 10.31 min.
NMR($CDCl_3$) in agreement with title compound
FAB MS: 492 (M+1)
Analysis Cal'd for $C_{25}H_{34}NSO_5P$ 0.5 $H_2O$

| | |
|---|---|
| Cal'd: | N, 2.80; C, 59.99; H, 7.05 |
| Found: | N, 2.71; C, 59.90; H, 7.49 |

EXAMPLE 27

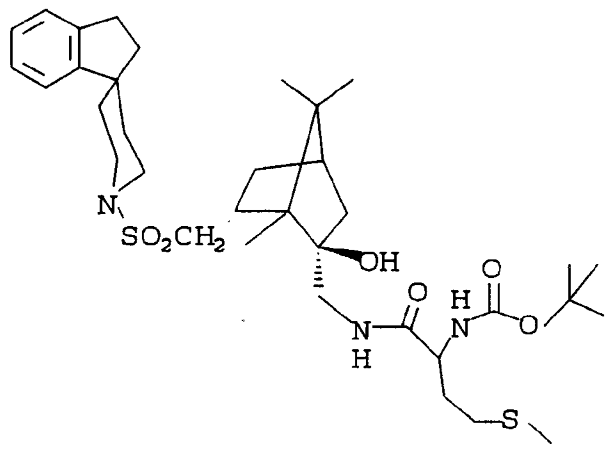

[1-[[[[exo-2-hydroxy-7,7-dimethyl-1-[spiro[1H-indene-1,4-piperidin]-1-yl-sulphonyl)methyl]bicyclo]2.2.1] hept-2-yl]methyl]amino]carbonyl]-3-(methylthio) propyl]-carbamic acid-1,1-dimethylethyl ester

To a stirred solution of (1S)-1′-(((exo-2-hydroxy-endo-2-aminomethyl-7,7-dimethylbicyclo (2.2.1)hept-1-yl)methyl)sulfonyl)spiro(1H-indene-1,4′-piperidine) (600 mg, 1.39 mmole) in 5 ml of dry, degassed N,N-dimethylformamide was added Boc-L-methionine (381 mg, 1.53 mmole) and 743 mg (1.68 mmole) of benzotriazol-1-yloxy tris (dimethylamino)phosphonium hexafluorophosphate (BOP) at room temperature. The resulting reaction mixture was protected from moisture and the pH was adjusted to 8-9 with diisopropylettiylamine. After one hour, all volatile components were removed under reduced pressure and the residue was dissolved in 250 ml of ethyl acetate. This solution was washed in succession with 10% aqueous citric acid, 50% sodium bicarbonate solution, and brine. The organic phase was dried (sodium sulfate) and concentrated. Chromatography of the crude reaction product on silica gel (1:1 ethyl acetate-hexane elution) afforded the title compound as an amorphous solid: m.p. 82-92°C.

HPLC: >92% pure at 214 nM

NMR: Consistent with structure and verified presence of solvent

FAB MS: 670 ($M^+$ + thioglycerol)

Analysis Cal'd for $C_{34}H_{51}N_3O_6S_2$â$0.5CHCl_3$:

| | |
|---|---|
| Cal'd: | C, 57.42; H, 7.19; N, 5.82 |
| Found: | C, 57.58; H, 7.50; N, 5.83 |

EXAMPLE 28

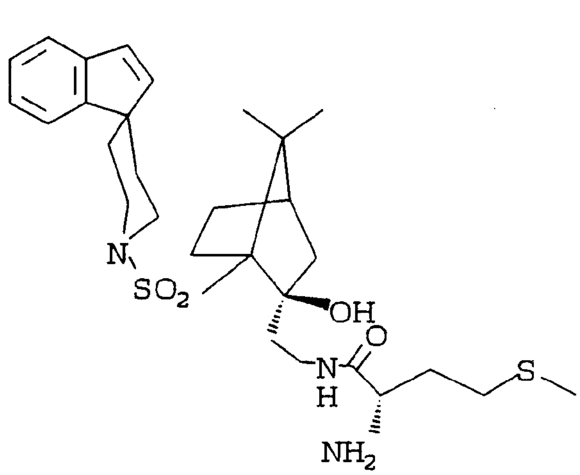

2-amino-N-[[exo-2-hydroxy-7,7-dimethyl-1-[(spiro[1H-indene-1,4′-piperidin]1′ylsulfonyl)methyl]bicyclo] 2.2.1]hept-2-yl]methyl]-4-methylthio)-butanamide

A continuous stream of dry HCl gas was passed for 5 minutes into an ice cold solution of ethyl acetate (2 ml) containing 20 mg of the product of Example 27. After 30 minutes at 0°C, the reaction mixture was concentrated and the residue was chromatographed on two 0.25 mm precoated silica gel plates (chloroform-methanol-ammonium hydroxide, 93:7:0.7 v/v elution). The title compound was obtained as a solid (10.7 mg): m.p. 78-80°C.

TLC: $R_f$=0.28 ($CHCl_3$-$CH_3OH$-$NH_4OH$, 95:5:0.5)
NMR: Consistent with structure and verifies presence of solvent
FAB MS: 562 ($M^+$+H)
Analysis Cal'd for $C_{29}H_{43}N_3O_4S_2$â0.75$CHCl_3$:
Cal'd: C, 54.86; H, 6.77; N, 6.45
Found: C, 54.52; H, 6.90; N, 6.32

EXAMPLE 29

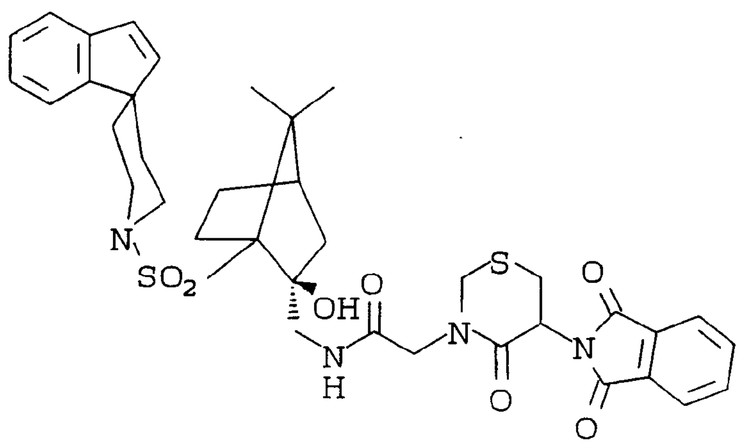

5-(1,3-dihydro-1,3-dioxo-2H-isoindol-2-yl)-N-[[2-hydroxy-7,7-dimethy-1-[(spiro[1H-indene-1,4′-piperidin]-1′-ylsulfonyl)methyl]bicyclo[2.2.1]hept-2-yl]-methyl]dihydro-4-oxo-2H-1,3-Thiazine-3(4H)-acetamide

To a stirred solution of (1S)-1′-(((exo-2-hydroxy-2-aminomethyl-7,7-dimethylbicyclo(2.2.1)hept-1-yl)-sulfonyl)spiro(1H-indene-1,4′-piperidine) (67 mg, 0.16 mmole) in 1.5 ml of dry, degassed N,N-dimethylformamide was added 4-oxo-5-phthalyl-1,3-thiazine-acetic acid (62 mg, 0.192 mmole) and 88 mg (0.20 mmole) of benzotriazol-1-yloxytris(dimethylamino)-phosphonium hexafluorophosphate (BOP) at room temperature. The resulting reaction mixture was protected from moisture and the pH was adjusted to 8-9 with diisopropylethylamine. After 24 hours all volatile components were removed under reduced pressure and the residue was suspended in 5 ml of toluene. Concentration of this suspension afforded the crude product which was chromatographed on silica gel (96:4:0.4 $CHCl_3$-$CH_3OH$-$NH_4OH$) to give the title compound (109 mg): m.p. 134-137° C.

NMR: Consistent with structure and verifies presence of solvent;
HPLC: >94% pure at 214 nM;
FAB MS: 733 ($M^+$+H)
Analysis Cal'd for $C_{38}H_{44}N_4O_7S_2$â1.1$CHCl_3$:
Cal'd: C, 54.34; H, 5.26; N, 6.48
Found: C, 54.12; H, 5.22; N, 6.42

EXAMPLE 30

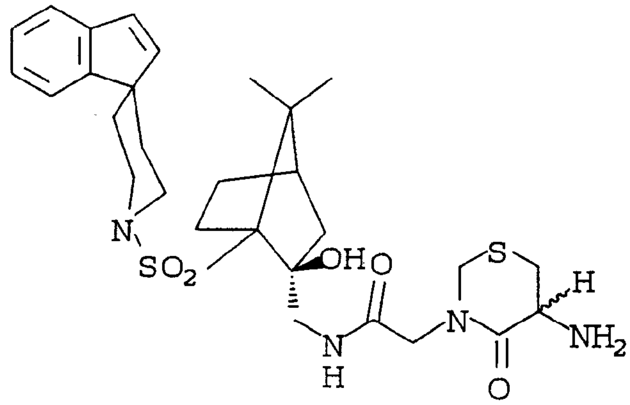

5-amino-N-[[1-[[spiro[1H-indene-1,4′-piperidin]-1-yl)-sulfonyl]methyl]-exo-2-hydroxy-7,7-dimethyl-bicyclo[1.1.1]hept-2-yl]methyl]dihydro-4-oxo-2H-1,3-Thiazine-3(4H)-acetamide

The product of Example 29 (60 mg) was dissolved in 3 ml of methanol and treated with 50 ml (1.59 mmole) of 95% hydrazine at 23°C. After 24 hours the solvent was removed under vacuum and the residue was suspended in toluene. Rotoevaporation of this suspension gave the crude product, which was chromatographed on four 0.5 mm procoated silica gel plates (chloroform-methanol-ammonium hydroxide, 92:8:0.8 v/v elution). The title compound was obtained as an amorphous solid (27 mg): m.p. 103-106°C;
NMR: Consistent with structure and verifies presence of solvent
HPLC: >97% pure at 214 nM;
FAB MS: 605 ($M^{+}$+H)
Analysis Cal'd for $C_{30}H_{44}N_4O_5S_2$â0.6$CHCl_3$:
Cal'd: C, 54.33; H, 6.64; N, 8.28
Found: C, 54.12; H, 6.60; N, 8.20

EXAMPLE 31

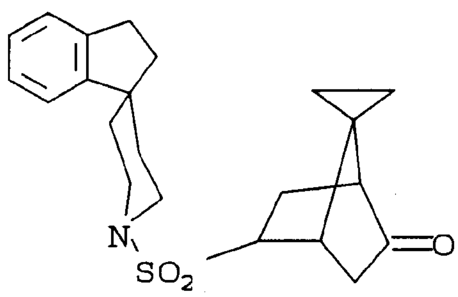

1′-[(5-oxospiro[bicyclo[2.2.1]heptane-7,1′-cyclopropan]-2-yl)sulfonyl]-spiro[1H-indane-1,4′-piperidine]

Spiro(1H-indane-1,4′-piperidine)hydrochloride (3 g, 13.5 mmole) was dissolved in 30 ml of methylene chloride. The resulting solution was cooled to 0°C and treated with 3.76 ml of triethylamine. This was followed by the dropwise addition of chloroethylsulfonylchloride (1.57 ml, 15 mmole). The reaction mixture was allowed to warm to room temperature over one hour and was filtered. The filtrate was washed with water, sodium bicarbonate solution, and brine. The dried organic washings were concentrated and the residue was column chromatographed on silica gel (98:2 methylene chloride-ether elution) to give 1.88 g of 1′-(vinylsulfonyl)spiro(1H-indane-1,4′piperidine) (m.p. 114-115°C).

1′-(Vinylsulfonyl)spiro(1H-indane-1,4′ piperidine) (185 mg, 0.67 mmole) was combined with 5.1 g (55 mmole) of spiroheptadiene in 4 ml of benzene and heated to reflux for 48 hours. The solvent and excess reagent were removed under reduced pressure and the residual oil was chromatographed on silica gel (4:1 hexane-ethyl acetate elution) to give 216 mg of a solid which was recrystallized from methanol to give white needles (m.p. 176-177°C). Of this material, 110.7 mg was dissolved in 5 ml of dry THF under nitrogen. This solution was

cooled to 0°C and treated with 0.1 ml of borane-THF complex. After stirring for one hour, an additional 0.1 ml of Borane-THF complex was added to the reaction mixture and stirring was continued at room temperature for 1 hour more. The reaction mixture was recooled to 0°C and quenched with the dropwise addition of water. After 10 minutes, 0.3 ml of 3N sodium hydroxide solution and 0.3 ml of 30% hydrogen peroxide solution was added. The resulting mixture was heated to 50°C for 1 hour and allowed to stand at 23°C overnight. The reaction mixture was partitioned between ethyl acetate and brine. The aqueous phase was extracted with ethyl acetate and the combined organic extracts were dried ($MgSO_4$) and concentrated. Purification of the crude reaction product by preparative TLC on silica gel (2:1 hexane-ethyl acetate) afforded two components. The more polar product (m.p. 232-233°C, from methanol) (0.15 mmole) was dissolved in 5 ml of methylene chloride and oxidized with pyridinium chlorochromate (2 mmole) at 23°C. The reaction mixture was filtered through celite and the filtrate was applied to 0.25 mm precoated silica gel plates. Elution with 2:1 hexane-ethyl acetate and recrystallization from methanol afforded the title compound as a white solid: m.p. 210-212°C;
NMR: Consistent with structure and verifies presence of solvent
HPLC: >96% pure at 214 nM;
FAB MS: 386 ($M^+$+H)
Analysis Cal'd for $C_{22}H_{27}NO_3S$â0.3$CH_2O$:
Cal'd: C, 67.59; H, 7.12; N, 3.58
Found: C, 67.66; H, 7.22; N, 3.53

EXAMPLE 32

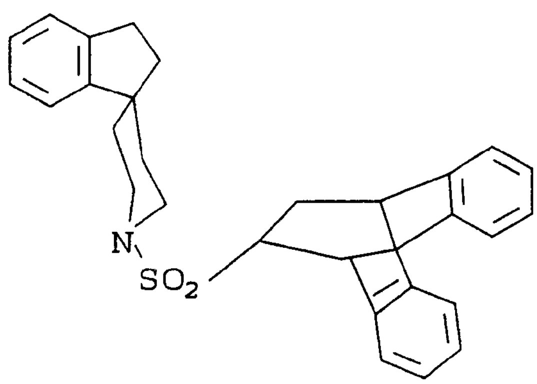

1'-[(9,10-dihydro-9,10-ethanoanthracen-1]-yl) sulfonyl]-spiro[1H-indane-1,4'-piperidine]

1'-(Vinylsulfonyl)spiro(1H-indane-1,4' piperidine) (152 mg) was combined with 350 of anthracene in 15 ml of toluene and heated to reflux for 8 days. The solvent and excess reagent was removed under reduced pressure and the residual oil was chromatographed on silica gel (4:1 hexane-ethyl acetate elution) to give the title compound as an amorphous solid.
NMR: Consistent with structure and verifies presence of solvent
HPLC: >99% pure at 214 nM;
FAB MS: 456 ($M^+$+H)
Analysis Cal'd for $C_{29}H_{29}NO_2S$â0.05C $H_2O$â0.25 $HCl_3$:
Cal'd: C, 72.23; H, 6.08; N, 2.88
Found: C, 72.23; H, 5.92; N, 2.67

EXAMPLE 33

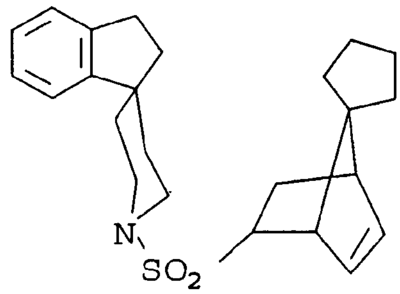

1′-(spiro[bicyclo[2.2.1]hept-5-ene-7,1′-cyclopentan]-2-ylsulfonyl)-spiro[1H-indane-1,4′-piperidine]

1′-(Vinylsulfonyl)spiro(1H-indane-1,4′ piperidine) (100 mg) was combined with 500 mg of spirononadiene in 3 ml of toluene and heated to reflux for 17 hours. The solvent and excess reagent were removed under reduced pressure and the residual oil was chromatographed on silica gel (4:1 hexane-ethyl acetate elution) to give the title compound as an amorphous solid: m.p. 115-119°C.

NMR: Consistent with structure and verifies presence of solvent
HPLC: >99% pure at 214 nM;
FAB MS: 398 ($M^{+}$+H)
Analysis Cal'd for $C_{24}H_{31}NO_{2}S$â0.05$CHCl_{3}$:
Cal'd: C, 71.57; H, 7.76; N, 3.47
Found: C, 71.94; H, 7.81; N, 3.42

EXAMPLE 34

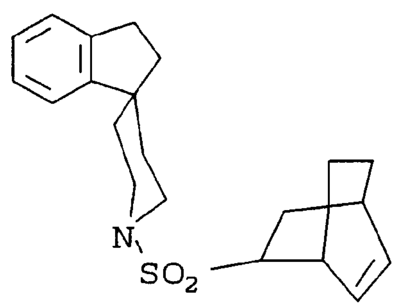

1′-(bicyclo[2.2.1]hept-5-en-2-ylsulfonyl)spiro[1H-indane-1,4′-piperidine]

1′-(Vinylsulfonyl)spiro(1H-indane-1,4′ piperidine) (100 mg) was combined with ten equivalents of cyclohexadiene in 10 ml of toluene and heated to reflux for 30 hours. The solvent and excess reagent were removed under reduced pressure and the residual oil was chromagraphed on silica gel (4:1 hexane-ethyl acetate elution) to give the title compound as an amorphous solid: m.p. 162-166°C.

NMR: Consistent with structure and verifies presence of solvent
HPLC: >99% pure at 214 nM;
FAB MS: 358 ($M^{+}$+H)
Analysis Cal'd for $C_{21}H_{27}NO_{2}S$â0.1$H_{2}0$:
Cal'd: C, 70.19; H, 7.63; N, 3.90
Found: C, 70.30; H, 8.03; N, 3.61

EXAMPLE 35

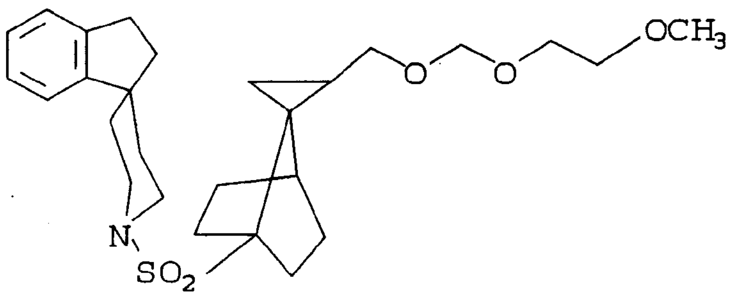

1'-[[2'-[[(2-methoxyethoxy)methoxy]methyl]spiro] bicyclo[2.2.1]hept-5-ene-7,1'-cyclopropan]-2-yl] sulfonyl]-spiro[1H-indane-1,4'-piperidine]

1'-(Vinylsulfonyl)spiro(1H-indane-1,4' piperidine) (2.45 g) was combined with 2.2 g of hydroxymethylspirocycloheptadiene and 3 mg of hydroquinone in 8 ml of toluene and heated to reflux for 17 hours. The solvent and excess reagent were removed under reduced pressure and the residual oil was chromatographed on silica gel (1:2 hexane-ethyl acetate elution) to give three components. The least polar of these products (80 mg) was dissolved in methylene chloride containing 78 mg of diisopropylethylamine and treated with 49 mg of 2-methoxyethoxymethyl chloride. The reaction mixture was protected from moisture and stirred at 23°C overnight. The reaction mixture was concentrated and the residual material was applied to 0.5 mm precoated silica gel plates. Elution with 1:1 hexane-ethyl acetate afforded the title compound.
NMR: Consistent with structure and verifies presence of solvent;
PLC: >90% pure at 214 nM;
FAB MS: 488 ($M^+$+H)

EXAMPLE 36

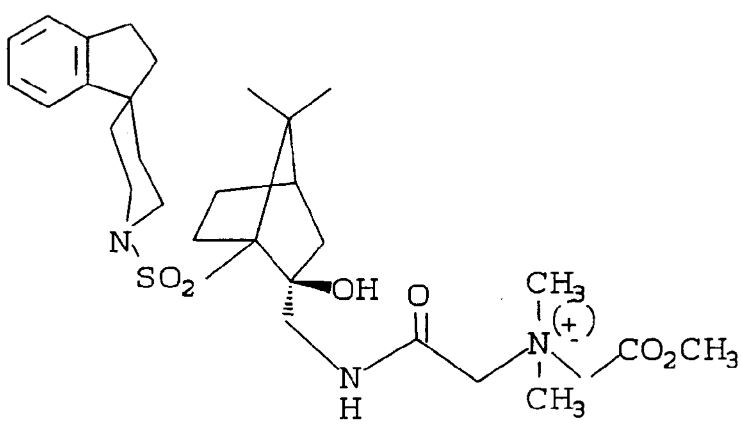

(1S-)-2-[[[exo-2-hydroxy-7,7-dimethyl-1-[(spiro [1H-indene-1,4'-piperidin]-1'-ylsulfonyl)methyl] bicyclo[2.2.1]hept-2-yl]methyl]amino]-N-(2-methoxy-2-oxoethyl)-N,N-dimethyl-2-oxo-ethanaminium salt with trifluoroacetic acid (1:1)

To a stirred solution of (1S)-1'-(((exo-2-hydroxy-endo-2-aminomethyl-7,7-dimethylbicyclo (2.2.1)hept-1-yl)methyl)sulfonyl)spiro(1H-indene-1,4'-piperidine) (108 mg, 0.25 mmole) in 5 ml of toluene was added 1 ml of toluene containing 0.5 mmole of methylimino- diacetic acid anhydride. Tetrahydrofuran (2 ml) was added and the white suspension was stirred at 23°C overnight. The reaction mixture was filtered and concentrated. The residue was dissolved in 5 ml of N,N-dimethylformamide and treated in succession with methyl iodide (0.5 ml) and diisopropylethylamine (0.2 ml). The resulting solution was protected from moisture and heated for 5 hours at 50°C. An additional 0.5 ml of methyl iodide was added and heating was continued for 5 hours more. The reaction mixture was concentrated and the residue was purified via reverse phase preparative HPLC (Vydac protein & Peptide C-18 column, mobile phase = 0.1% trifluoro- acetic acid (TFA) in water-acetonitrile). The fractions containing the title compound were pooled and concentrated. The residue was dissolved in dioxane and this solution was freeze-dried to yield the TFA salt of the title compound as a white powder:
NMR: Consistent with structure and verifies presence of solvent
HPLC: >99% pure at 214 nM;
FAB MS: 588 ($M^{+}$+H)
Analysis Cal'd for $C_{31}H_{46}N_3O_6S$â 1.8$CF_3CO_2H$â1.0 dioxane:
Cal'd: C, 52.61; H, 6.27; N, 4.77
Found: C, 52.58; H, 6.40; N, 4.76

EXAMPLE 37

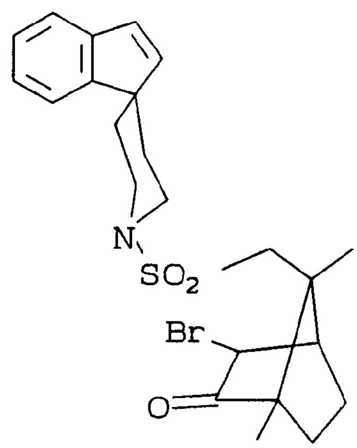

(1R-syn)-1′-[[(1,7-dimethyl-2-oxobicyclo[2.2.1]hept-7-yl)methyl]sulfonyl]-spiro[1H-indene-1,4′-piperidine]

Dissolved 100 mg (.304 mM; FW=328.23) of (-)-3-bromocamphosu1fonic acid ammonium salt in 15 mL of DCM. Added 5 eq of thionylchloride (FW=118.97; d=1.631; 1.52 mM; 120 mL) and allowed mixture to react at 0°C under a $N_2$ balloon for 1 hour. After 1 hour the mixture was concentrated to obtain 63.12 mg of crude acid chloride (.191 mM).

Dissolved 47.13 mg of indene HCl salt in 20 mL of THF. The acid chloride was added along with 2 eq of DIEA, and allowed to react at room temperature for 1 hour, then concentrated and washed with 2 x 200 mL of 1 N NCl, 2 x 200 mL $H_2O$ and 2 x 200 mL brine, then concentrated and dried over sodium sulfate. Flash chromatography in 25% EtOAc/petroleum ether.

EXAMPLE 38

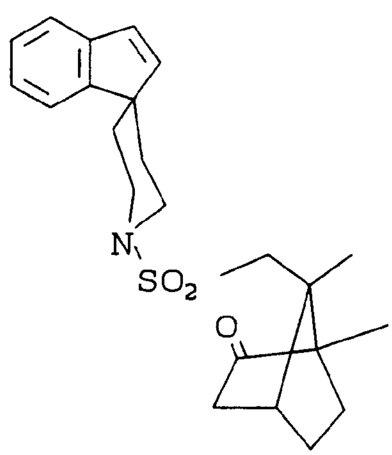

To a solution of the product of Example 37 (107 mg, 0.300 mmol) in glacial acetic acid (10 mL) was added

zinc dust (24 mg, 0.367 mmol). The temperature was then increased to reflux. After 30 minutes the mixture was cooled to room temperature, filtered, then concentrated under reduced pressure. Purification by flash chromatography (35% ethyl acetate in petroleum ether as eluent) afforded 80 mg of the product as a white amorphous foam.

NMR (300 MHz, $CDCl_3$): Consistent with structure;
HPLC: (Vydac C18 column, gradient from 95/5 to 0/100 $H_2O/CH_3CN$ with 0.1% TFA, 15 min. gradient, flow rate = 1.5 mL/min.):
Purity: 98%, r.t. = 12.67 min.
FAB MS: $[M + 1]^+$ at 400.91
Analysis Cal'd for $C_{23}H_{29}NO_3S$:

| | |
|---|---|
| Cal'd: | C, 69.13; H, 7.32; N, 3.51 |
| Found: | C, 69.25; H, 7.30; N, 3.48 |

EXAMPLE 39

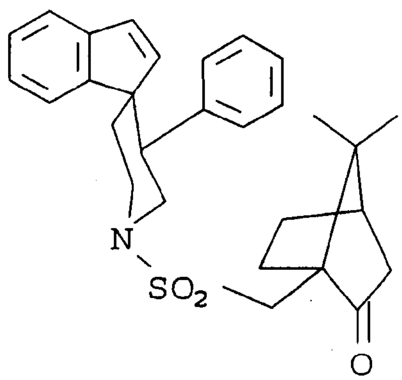

1'-[[(7,7-dimethyl-2-oxobicyclo[2.2.1]hept-1-yl)-methyl]sulfonyl]-3'-phenyl-spiro[1H-indene-1,4'-piperidine]

Ethanolamine (6.66 mL, 0.1103 mmol) was placed in a 3-neck round bottom flask and heated to reflux. Once at relux, styrene oxide (6.28 mL, 0.0557 mmol) was added dropwise to the reaction mixture over 15 minutes. The reaction mixture was refluxed an additional 2 hours and then allowed to cool. The product, N-(2-hydroxyethyl)-N-(2-hydroxy-2-phenylethyl)amine, was obtained as a clear oil by vacuum distillation.

N-(2-hydroxyethyl)-N-(2-hydroxy-2-phenyl-ethyl)amine (1.30 g, 7.16 mmol) was dissolved in chloroform and then while under a nitrogen blanket, thionyl chloride (1.05 mL, 14.32 mmol) was added dropwise over 10 minutes. The reaction mixture was then refluxed for 1 hour and allowed to cool to room temperature. Water was then added to the mixture and allowed to stir 1 hour. The reaction mixture was separated and the organic layer was washed three times with 3N HCl. The aqueous layers were combined and then basified with 40% sodium hydroxide. The aqueous layer was extracted 3 times with ether, the organics were combined, and dried over sodium sulfate. The organic layer was filtered and the filtrate concentrated to a yellow oil.

The yellow oil was dissolved in ether and then di-t-butyl dicarbonate (1.50 g, 6.87 mmol) was added along with triethylamine (500 ml, 3.59 mmol). The mixture was allowed to stir overnight and then it was washed with $H_2O$ (2x), 0.1N HCl (2x) and sodium bicarbonate (2x). The organic layer was dried over sodium sulfate, filtered, and the filtrate concentrated to a yellow oil. The yellow oil was dissolved in $CH_2Cl_2$ and poured onto a silica gel column. The column was eluted with 1:1 $CH_2Cl_2$/hexane. The product fractions were combined and concentrated to dryness to yield the product, N-(2-hydroxyethyl)-N-(2-hydroxy-2-phenylethyl)-t-butyl-carbamate.

To a solution of indene (268 ml, 2.30 mmol) in dry THF (2 mL) cooled in an ice bath and maintained under a nitrogen blanket was added lithium bis(trimethylsilyl)amide (1M solution in THF, 4.6 mL, 4.60 mmol) over 10 minutes. The mixture was stirred in the cold bath for 30 minutes, then added over 10 minutes to a solution of N-(2-hydroxethyl)-N-(2-hydroxy-2-phenylethyl)-t-butylcarbamate (694 mg, 2.30 mmol) stirred in an ice bath. The mixture was stirred for 2 hours in the cold and for 30 minutes at 25°C under nitrogen, then concentrated to an orange oil. 10% ethyl acetate in hexane was added and the resulting mixture poured onto a silica gel column packed with 10% ethyl acetate in hexane. Elution was with the same solvent and the product fraction were concentrated to provide 1'-(t-butyloxycarbonyl)-spiro-(indene-3'-phenyl-1,4'-piperidine.

1'-(t-Butyloxycarbonyl)spiro(indene-3-'phenyl-1,4'-piperidine) (290.9 mg, 0.842 mmol) in ethyl acetate was stirred in an ice bath and saturated with HCl (g) for 30 minutes. The mixture was concentrated to dryness and

reconcentrated from ether three times to yield spiro (1H-indene-3-phenyl-1,4′-piperidine)-hydrochloride.

Spiro(1H-indene-3′-phenyl-1,4′-piperidine) hydrochloride (205.2 mg, 0.690 mmol) and (+)-10-camphorsulfonyl chloride (196 mg, 0.782 mmol) were combined in THF and the pH was adjusted to 9 with triethylamine (196 mL, 1.41 mmol). The reaction mixture was stirred overnight at 25°C, then concentrated to dryness, and redissolved in $CH_2Cl_2$, then poured onto a silica gel column and eluted with $CH_2Cl_2$. The product fractions were combined and evaporated to dryness to provide the title compound which was crystallized from ether and dried in vacuo overnight.

m.p.: 183° - 215°C

NMR: Consistent with structure

HPLC: >95% pure

MS: M + H 476.3 (FAB)

Analysis Cal'd for $C_{29}H_{33}NO_3S$â$0.50H_2O$;

Cal'd: C, 71.86; H, 7.07; N, 2.89

Found: C, 71.88; H, 7.03; N, 2.87

EXAMPLE 40

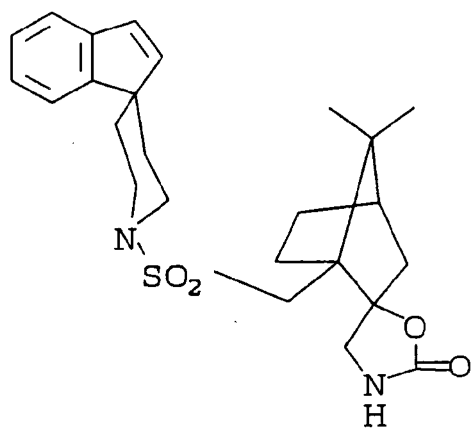

(1S(1a,2a,4a))-2-hydroxy-7,7-dimethyl-1-((spiro-(1H-indene,1,4′-piperidin)-1′-yl-sulfonyl)methyl)-bicyclo-(2.2.1)heptane-2-acetic acid

(100 mg, 0.217 mmol) diphenylphosphoryl azide (51.5 mL, 0.239 mmol), and triethylamine (66.2 mL, 0.471 mmol) were combined in DMF. The mixture was allowed to stir overnight and then it was concentrated to dryness. The resulting residue was dissolved in $CH_2Cl_2$, poured onto a silica gel column, and eluted with 5% methanol in $CH_2Cl_2$. The product fractions were combined and concentrated to dryness. The title compound was obtained as a white solid from ether and dried in vacuo overnight.

NMR: Consistent with structure

HPLC: >93% pure

MS: M + H 457.3 (FAB)

Analysis Cal'd for $C_{25}H_{32}N_2O_4S$;

Cal'd: C, 65.76; H, 7.06; N, 6.14

Found: C, 65.76; H, 7.42; N, 5.80

EXAMPLE 41

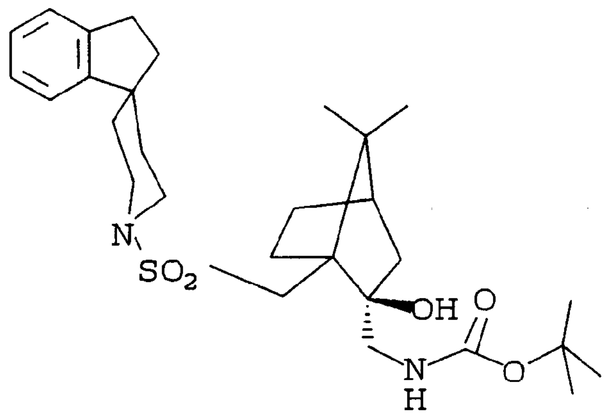

(1S-exo)-[[2--hydroxy-7,7-dimethyl-1-[(spiro[1H-indene-1,2′-piperidin)-1′-ylsulfonyl)methyl]bicyclo 2.2.1]hept-2-yl]carbamic acid 1,1-dimethylethyl ester

(1S)-1′-(((7,7-dimethyl-2-oxobicyclo-(2.2.1)-hept-1-yl)-methyl)sulfonyl)spiro(1H-indene-1,4′-piperidine) (1.92 g, 4.81 mmol) was combined with zinc iodide (47 mg, 0.15 mmol) in 2 mL of toluene. While under a nitrogen atmosphere, the mixture was treated with trimethylsilyl cyanide (960 mL, 7.21 mmol) dropwise. The reaction mixture was then heated to 100°C for 3.5 hours. The mixture was allowed to cool and diluted with 10 mL of dry THF. Then lithium aluminum hydride (1M in THF; 8.6 mL, 8.6 mmol) was added dropwise over 5 minutes and the mixture was allowed to stir at 25°C for 2 hours. The reaction mixture was then diluted with ether (N50 mL) and 10% sodium hydroxide solution was added dropwise until a gray precipitate stopped forming. The mixture was now filtered, the filtrate was washed with sodium bicarbonate and brine, the organic layer was separated and dried over sodium sulfate. The organic layer was filtered, the filtrate concentrated and the residue dissolved in $CH_2Cl_2$. This was poured onto a silica gel column, eluted with 97/3/0.3 of $CH_2Cl_2$/methanol/ammonium hydroxide and product fractions collected. The product fractions were combined and concentrated to dryness. The desired product, (1S)-1′-(((7,7-dimethyl-(2-endo-aminomethyl-2-exo-hydroxy)-bicyclo-(2.2.1)-hept-1-yl)-methyl)-sulfonyl)spiro(1H-indene-1,4′-piperidine), was obtained as a white foam from ether.

(1S)-1′-(((7,7-dimethyl-(2-endo-aminomethyl-2-exo-hydroxy)-bicycylo-(2.2.1)-hept-1-yl)-methyl)-sulfonyl )spiro(1H-indene-1,4′-piperidine) (44.9 mg, 0.104 mmol) and di-t-butyl dicarbonate (25 mg, 0.115 mmol) were combined in 5 mL of $CH_2Cl_2$. The reaction mixture was treated with triethylamine (18.1 mL, 0.13 mmol) and then stirred for 30 minutes at 25°C. The reaction mixture was poured onto a silica gel column and eluted with 15% ethyl acetate in hexane. The product fractions were combined and evaporated to dryness. The title compound was obtained as a white solid from $CH_2Cl_2$/hexane and was dried in vacuo overnight.

m.p.: 71°C-115°C

NMR: Consistent with structure

HPLC: >95% pure

Analysis Cal'd for $C_{29}H_{42}N_2O_5S$â0.45;

| | |
|---|---|
| Cal'd: | C, 66.14; H, 8.73; N, 5.03 |
| Found: | C, 66.21; H, 8.52; N, 4.75 |

EXAMPLE 42

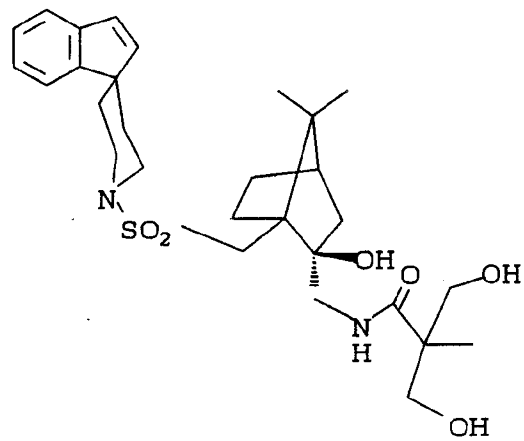

(1S-exo)-3-hydroxy-N-[[2-hydroxy-7,7-dimethyl-1-[(spiro[1H-indene-1,4′-piperidin]-1′-ylsulfonyl) methyl]bicyclo[2.2.1]hept-2-yl]methyl]-2-(hydroxy-methyl)-2-methyl-propanamide

(1S)-1′-(((7,7-dimethyl-(2-endo-aminomethyl-2-exo-hydroxy)-bicicylo-(2.2.1)-hept-1-yl)-methyl)-sulfonyl) spiro(1H-indene-1,4′-piperdine) (51 mg, 0.118 mmol), 2,2-bis(hydroxymethyl)-propionic acid (19 mg, 0.142 mmol), 1-hydroxybenzotriazole hydrate (HBT) (19 mg, 0.142 mmol), and 1-ethyl-3-(dimethylaminopropyl) carbodiimideâHCl(EDC) (27 mg, 0.142 mmol) were all combined in 5 mL of DMF. Then, triethylamine (46 mL, 0.33 mmol) was added to adjust the pH to 9, and the mixture was allowed to stir overnight at 25°C. The reaction mixture was concentrated to dryness and treated with 5% citric acid solution. The water layer was then basified with saturated sodium bicarbonate and extracted 3 times with ethyl acetate. The organics were combined and dried over sodium sulfate. The organic layer was filtered, the filtrate concentrated, and the resulting residue dissolved in $CH_2Cl_2$. This was then poured onto a silica gel column and eluted with 3% methanol in $CH_2Cl_2$. The product fractions were combined and evaporated to dryness. The title compound was obtained as a white solid from $CH_2Cl_2$/hexane and was dried in vacuo overnight.

m.p.: 167-170°C
NMR: Consistent with structure
HPLC: >99% pure
MS: M + H 547.3 (FAB)
Analysis Cal'd for $C_{29}H_{42}N_2O_6S$;
Cal'd: C, 63.71; H, 7.74; N, 5.12
Found: C, 63.65; H, 7.59; N, 4.88

EXAMPLE 43

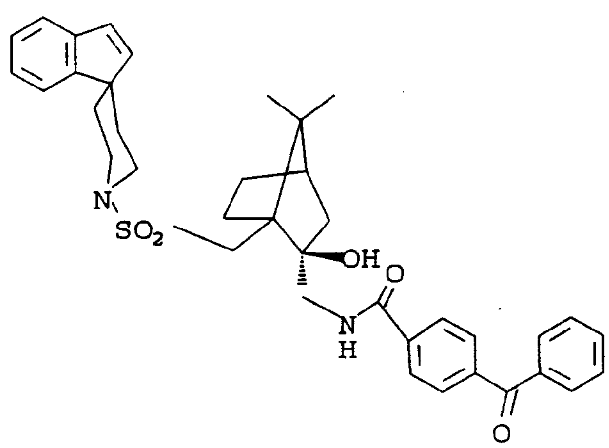

(1S-exo)-4-benzoyl-N-[[2-hydroxy-7,7-dimethyl-1-[(spiro[1H-indene-1,4′-piperidin]-1′-ylsulfonyl) methyl]bicyclo[2.2.1]hept-2-yl]methyl]-benzamide

The procedure of Example 42 was carried out using 49.6 mg, 0.115 mmol of (1S)-(((7,7-dimethyl-(2-endo-aminomethyl-2-exo-hydroxy)-bicicylo-(2.2.1)-hept-1-yl)-methyl)sulfonyl)spiro(1H-indene-1,4′-piperidine),26.5 mg, 0.138 mmol of EDC, 18.7 mg, 0.138 mmol of HBT, 48 mL, 0.345 mmol of triethylamine, and substituting 4-benzoylbenzoic acid (31.3 mg, 0.138 mmol) for 2,2-bis-(hydroxymethyl)-propionic acid. Chromatographic elution was with 2% methanol in $CH_2Cl_2$. The title compound was obtained and dried in vacuo, overnight.
m.p.: 10-135°C
NMR: Consistent with structure
HPLC: >94% pure
MS: M + H 639 (FAB)
Analysis Cal'd for $C_{38}H_{42}N_2O_5$Sâ0.40$C_6H_{14}$;
Cal'd: C, 72.06; H, 7.13; N, 4.16
Found: C, 72.01; H, 7.14; N, 4.18

EXAMPLE 44

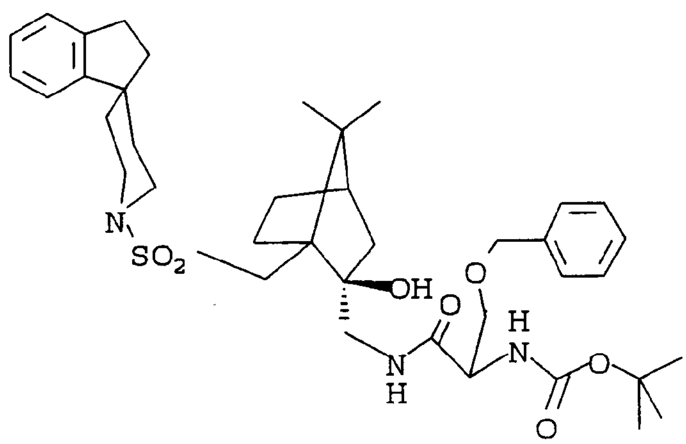

1′-(bicyclo[2.2.1]hept-5-en-2-ylsulfonyl)spiro[1H-indane-1,4′-piperidine]

The procedure of Example 42 was carried out using 94.5 mg, 0.22 mmol of (1S)-(((7,7-dimethyl-(2-endo-amino-methyl-2-exo-hydroxy)-bicicylo-(2.2.1)-hept-1-yl)-methyl)-sulfonyl)spiro(1H-indene-1,4′-piperidine), 49.8 mg, 0.26 mmol of EDC, 35.2 mg, 0.26 mmol of HBT, 90 mL, 0.66 mmol of triethylamine, and substituting t-boc-L-serine(bzl) (76.8 mg, 0.26 mmol) for 2,2-bis-(hydroxymethyl)-propionic acid. Chromatographic elution was with 98/2 of $CH_2Cl_2$/methanol/ammonium hydroxide. The title compound was obtained as a white solid from ether and dried in vacuo overnight.
m.p.: 75-95°C
NMR: Consistent with structure
HPLC: >98% pure
MS: M + H 639 (FAB)
Analysis Cal'd for $C_{39}H_{53}N_3O_7S$;
Cal'd: C, 66.16; H, 7.55; N, 5.94
Found: C, 66.04; H, 7.68;,N, 5.84

EXAMPLE 45

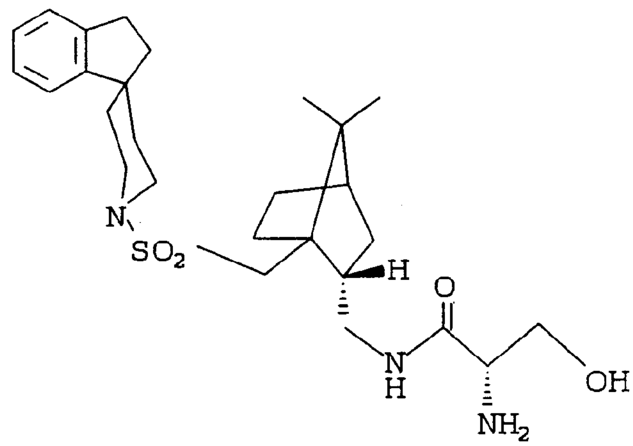

[1S-[1.alpha., 2 Beta., 2(R*), 4. beta.]]-2-amino-N[[1-[[(2,3-dihydrospiro[1H-indene-1,4'-piperidin]-yl)sulfonyl]methyl]-2-hydroxy-7,7-dimethylbicyclo[2.2.1]hept-2-ly]methyl]-3-hydroxy-propanamide

The product of Example 44 (120 mg, 0.17 mmol) was dissolved in 4% formic acid in methanol. Palladium hydroxide catalyst (25 mg) was added to the solution and it was placed on a PARR apparatus under 50 p.s.i. of hydrogen, overnight. The reaction mixture was filtered over solka floc and the filtrate was concentrated to a clear oil. This clear oil was dissolved in 2 mL of ethyl acetate and chilled to 0°C. At this point, 5 mL of prechilled sat'd HCl/ethyl acetate solution was added dropwise. The reaction mixture was allowed to stir for 1 hour and then concentrated to dryness. The resulting residue was partitioned between ethyl acetate and saturated sodium bicarbonate, and after extracting 3 times with ethyl acetate, the organics were combined and dried over sodium sulfate. The organic layer was filtered, the filtrate was concentrated, and the resulting residue was dissolved in $CH_2Cl_2$. This was then poured onto a silica gel column and eluted with 95/5/0.5 of $CH_2Cl_2$/methanol/ammonium hydroxide. The product fractions were combined and evaporated to dryness. The title compound was obtained as a white solid from ether and dried in vacuo overnight.

m.p.: 70-110°C

NMR: Consistent with structure

HPLC: >93% pure

MS: M + H 520.3 (FAB)

Analysis Cal'd for $C_{27}H_{41}N_3O_5S$â0.25 $C_4H_{10}$;

| | |
|---|---|
| Cal'd: | C, 61.31; H, 8.20; N, 7.67 |
| Found: | C, 61.35; H, 8.11; N, 7.58 |

EXAMPLE 46

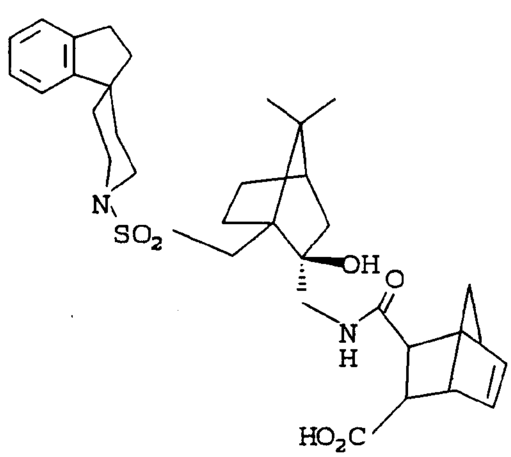

3-[[[[2-hydroxy-7,7-dimethyl-1-[(spiro[1H-indene-1,4′-piperidin]-1′-ylsulfonyl)methyl]bicyclo[2.2.1]-hept-2-yl]methyl]amino]carbonyl]bicyclo[2.2.1]hept-5-ene-2-carboxylic acid

(1S)-(((7,7-dimethyl-(2-endo-aminomethyl-2-exo-hydroxy)-bicicylo-(2.2.1)-hept-1-yl)-methyl) sulfonyl)spiro(1H-indene-1,4′-piperidine) (98.5 mg, 0.23 mmol) was combined with bicyclo (2.2.1)-5-heptene-2,3-dicarboxylic anhydride (43 mg, 0.26 mmol) in 5 mL of THF. Triethylamine (50 mL, 0.36 mmol) was added to the reaction dropwise to adjust the pH to 9. The reaction mixture was stirred overnight and then concentrated to dryness. The resulting residue was dissolved in $CH_2Cl_2$, poured onto a silica gel column, and eluted with 97/3/0.3 of chloroform/ methanol/acetic acid. The product fractions were combined and concentrated to dryness. The title compound was obtained as a white solid from ether and dried in vacuo overnight.

m.p.: 128-165°C
NMR: Consistent with structure
HPLC: >95% pure
MS: M + H 595 (FAB)
Analysis Cal'd for $C_{33}H_{42}N_2O_6S$â0.10 $CH_2Cl_2$;

| | |
|---|---|
| Cal'd: | C, 64.74; H, 7.12; N, 4.56 |
| Found: | C, 64.76; H, 7.07; N, 4.66 |

EXAMPLE 47

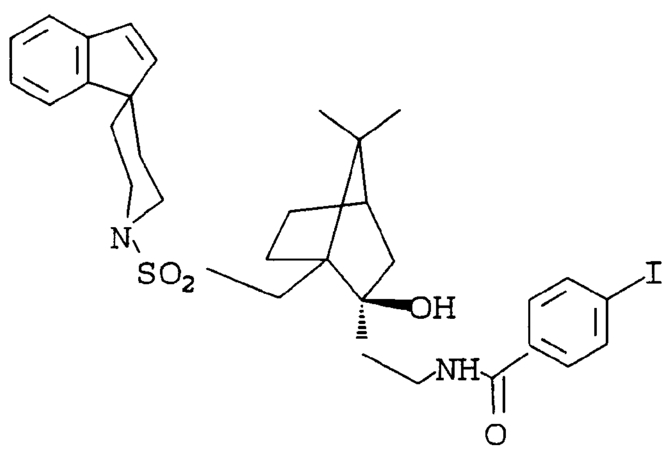

(1S)-1′-(((2-endo-cyanomethyl-7,7-dimethyl-exo-2-hydroxybicyclo-(2.2.1)-hept-1-yl)methyl)sulfonyl)-spiro(1H-indene-1,4′-piperidine)

Lithium bis(trimethylsilyl)amide (21.3 ml of a 1.0M THF solution, 21.3 mmol) was cooled to -78°C under $N_2$, treated with acetonitrile (1.07 ml, 20.4 mmol) and stirred 15 minutes. A THF solution (30 mL) of (1S)-1′-(((7,7-dimethyl-2-oxobicyclo(2.2.1)-hept-1-yl)methyl)sulfonyl)spiro(1H-indene-1,4′-piperidine) (40 gm, 10 mmol) was added dropwise and stirred 10 minutes after addition was complete. 6N HCl (2.8 ml) was added to the cold reaction all at once and the mixture was allowed to warm to 25°C.

The mixture was diluted with $H_2O$ (25 ml) and extracted with EtOAc (3 x 100 ml). The organic layers were combined, washed with $H_2O$ (25 ml) and brine (25 ml), dried over $Na_2SO_4$, filtered and concentrated to dryness in vacuo. Trituration with ether gave the title compound (4.17 gm, 93% yield) as a crystalline solid (m.p. 199-202°C).

TLC: $R_f$ = 0.69, Silica GF (7% $Et_2O$ in $CH_2Cl_2$)
PMR : Consistent with structure

(1S)-1′-(((2-endo-aminoethyl-7,7-dimethyl-2-exo-hydroxybicyclo(2.2.1)-hept-1-yl)methyl)sulfonyl)spiro(1H-indene-1,4′-piperidine)

Under a blanket of nitrogen, Lithium Aluminum Hydride (8.0 ml, 8.0 mmol, of a 1.0 M THF solution) was cooled to 0°C, treated dropwise with a THF solution (37 ml) of (1S)-1′-(((2-endo-cyanomethyl-7,7-dimethyl-2-hydroxybicyclo-(2.2.1)-hept-1-yl)methyl )-sulfonyl)spiro(1H-indene-1,4′-piperidine) (4.4 gm, 10.0 mmol) and the reaction stirred 10 minutes. With rapid stirring, the reaction was treated with $H_2O$ (0.37 ml), 20% NaOH

(aq) (0.40 ml) and $H_2O$ (1.46 ml) followed by extraction with EtOAc (3 x 150 ml). The organic layers were combined, washed with $H_2O$ (25 ml) and brine (25 ml), dried over $Na_2SO_4$, filtered and concentrated to dryness in vacuo.

Flash chromatography on silica gel (90/10/1/1 of $CH_2Cl_2$/MeOH/$H_2O$/HOAc) provided product fractions which were pooled and concentrated to dryness. The residue was treated with sat'd $Na_2CO_3$ (aq) and extracted with EtOAc (3 x 100 ml). The organic layers were combined, washed with $H_2O$ (25 ml) and brine (25 ml), dried over $Na_2SO_4$, filtered and concentrated to dryness to give the title compound (2.55 gm, 57% yield) as a white foam/solid.

TLC: $R_f$ = 0.27 Silica GF (90/10/1/1 of $CH_2Cl_2$/MeOH/$H_2O$/HOAc)
PMR: Consistent with structure

(1S)-1′-(((2-endo-aminoethyl-7,7-dimethyl-2-exo-hydroxybicyclo-(2.2.1)-hept-1-yl)methyl)sulfonyl)-spiro (1H-indene-1,4′-piperidine) (60 mg, 0.135 mmol) was dissolved in $CH_2Cl_2$ (1 ml), treated with 4-iodobenzoyl chloride (36.0 mg, 0.135 mmol) and $Et_3N$ to adjust the pH to 9.5. The reaction was stirred 30 minutes at 25°C and flash chromatographed on silica gel (8% $Et_2O$ in $CH_2Cl_2$) to give the title compound (45 mg, 50% yield) as a white foam from $Et_2O$ (m.p. 98-138°C, shrinks).

TLC: $R_f$ = 0.35 Silica GF (10% $Et_2O$ in $CH_2Cl_2$)
PMR: Consistent with structure
HPLC: 95.8% pure
M.S.: (FAB) M + H + Thioglycerol = 783

| | |
|---|---|
| Analysis Cal'd for | $C_{32}H_{39}IN_2O_4S$â0.30 $C_4H_{10}O$;â0.35 $H_2O$; |
| Cal'd: | C, 56.70; H, 6.12; N, 3.98 |
| Found: | C, 56.73; H, 6.11; N, 4.01 |

EXAMPLE 48

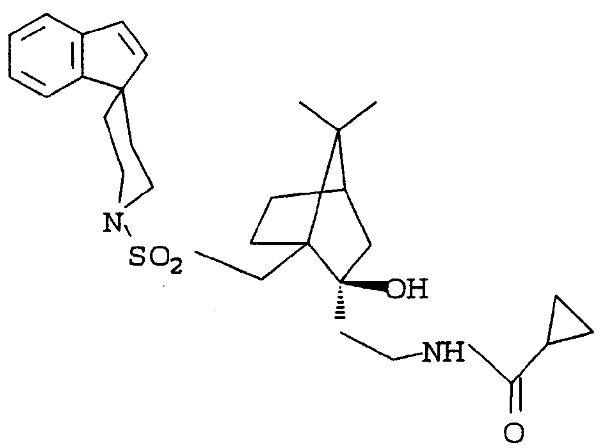

(1S-exo-N-[2-[2-hydroxy-7,7-dimethyl-1-[(spiro[1H-indene-1,4′-piperidin]1′-ylsulfonyl)methyl]bicyclo [2.2.1]hept-2-yl]ethyl]-cyclopropanecarboxamide

The title compound was prepared according to the procedure of Example 47 except that cyclopropylcarbonyl chloride (12.4 ml, 0.135 mmol) was substituted for 4-iodobenzoyl chloride.

Flash chromatography of the reaction mixture on silica gel (15% $Et_2O$ in $CH_2Cl_2$) gave the title compound (36.6 mg, 53% yield) as a white foam from Ether (m.p. 93-105°C shrink + foam).

TLC: $R_f$= 0.22 Silica GF (15% $Et_2O$ in $CH_2Cl_2$)
PMR: Consistent with Structure
HPLC: 96.8%
M.S.: (FAB) M + H + Thioglycerol = 621

| | |
|---|---|
| Anal. Calc'd for | $C_{29}H_{40}N_2O_4S$â0.20 $C_7H_{10}O$ |
| Cal'd: | C, 67.84; H, 8.03; N, 5.31 |
| Found: | C, 67.69; H, 8.06; N, 5.06 |

EXAMPLE 49

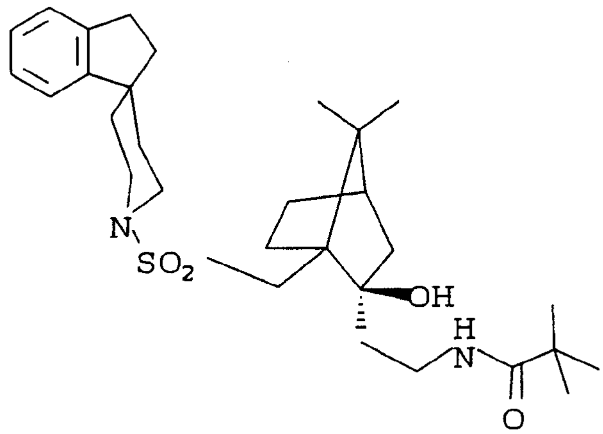

(1S-exo)-N-[2-[2-hydroxy-7,7-dimethyl-1-[(spiro[1H-indene-1,4′-piperidin[-1′-ylsulfonyl)Methyl]bicyclo[2.2.1]hept-2-yl]ethyl]-2,2-dimethyl-propanamide

The title compound was prepared according to the procedure of Example 47 except that trimethyl acetyl chloride (16.6 ml, 0.135 mmol) was substituted for 4-iodobenzoyl chloride.

Flash chromatography of the reaction on silica gel (13% $Et_2O$ in $CH_2Cl_2$) gave the title compound (32.8 mg, 46% yield) as a white foam from $Et_2O$ (m.p. 78-104°C, shrink + foam).

TLC: $R_f$ = 0.29 Silica GF (15% $Et_2O$ in $CH_2Cl_2$)
PMR: Consistent with structure
HPLC: 97.6%
M.S.: (FAB) M + H + Thioglycerol = 637
Anal. Calc'd for $C_{30}H_{44}N_2O_4S$â0.20 $C_4H_{10}O$â0.30 $H_2O$ =
Cal'd: C, 67.38; H, 8.56; N, 5.10
Found: C, 67.37; H, 8.52; N, 5.02

EXAMPLE 50

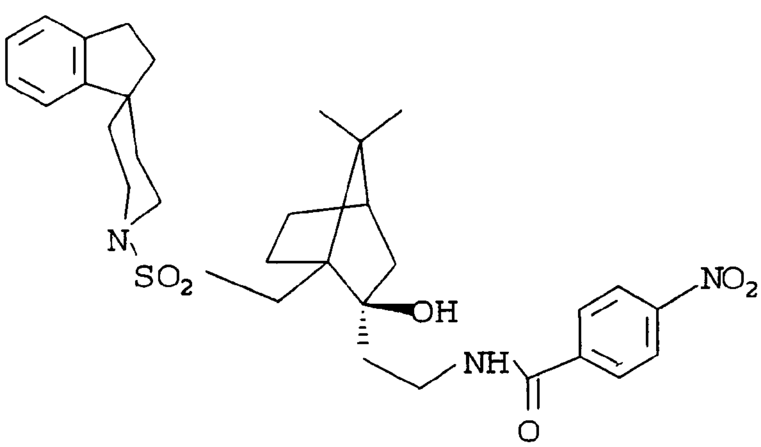

(1S-exo)-N-[2-[2-hydroxy-7,7-dimethyl-1-[(spiro[1H-indene-1,4′-piperidin]-1′-ylsulfonyl)methyl]bicyclo[2.2,1]hept-2-yl]ethyl]-4-methoxy-benzamide

The title compound was prepared according to the procedure of Example 47 except that 4-nitrobenzoyl chloride (25.1 mg, 0.135 mmol) was substituted for 4-iodobenzoyl chloride.

Flash chromatography of the reaction on silica gel (10% $Et_2O$ in $CH_2Cl_2$) gave the title compound (41.6 mg, 52% yield) as a white foam from Ether (m.p. 118-33°C, shrink + foam).

TLC: $R_f$= 0.26 Silica GF (10% $Et_2O$ in $CH_2Cl_2$).
PMR: Consistent with structure
HPLC: 97.1%
M.S.: (FAB) M + H + Thioglycerol = 702.
Anal. Calc'd for $C_{32}H_{39}N_3O_6S$â0.30 $C_4H_{10}O$â0.20 $H_2O$
Cal'd: C, 64.35; H, 6.90; N, 6.78
Found: C, 64.32; H, 6.98; N, 6.63

EXAMPLE 51

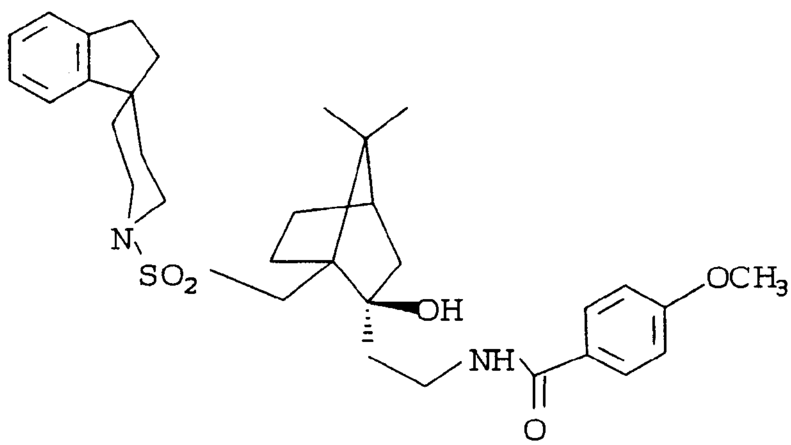

(1S-exo)-N-[2-[2-hydroxy-7,7-dimethyl-1-[(spiro[1H-indene-1,4′-piperidin]-1′-ylsulfonyl)methyl]ethyl]bicyclo[2.2.1]hept-2-yl]ethyl[-4-methoxy-benzamide

The title compound was prepared according to the procedure of Example 47 except that p-anisoyl chloride (18.3 ml, 0.135 mmol) was substituted for 4-iodobenzoyl chloride.

Flash chromatography of the reaction on silica gel (15 % $Et_2O$ in $CH_2Cl_2$) gave the title compound (36 mg, 46% yield) as a white solid from $Et_2O$ (m.p. 98-121°C, shrink).

TLC: $R_f$= 0.26 Silica GF (15% $Et_2O$in $CH_2Cl_2$).
PMR: Consistent with structure
HPLC: 95.5%
M.S.: M + H + Thioglycerol = 687
Anal. Calc'd for $C_{33}H_{42}N_2O_5S$â0.25 $C_4H_{10}O$
Cal'd: C, 68.36; H, 7.51; N, 4.69
Found: C, 68.14; H, 7.67; N, 4.55

EXAMPLE 52

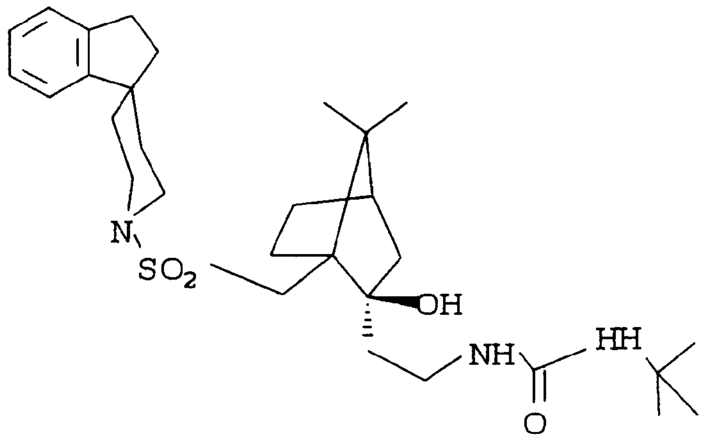

(1S-exo)-1′-[[[2-[2-[[[(1,1-dimethylethyl)amino]-carbonyl]amino]ethyl]-2-hydroxy-7,7-dimethylbicyclo-[2.2.1]hept-1-yl]methyl]sulfonyl]-spiro[1H-indene-1,4′-piperidine]

(1S)-1′-[[[endo-aminoethyl-7,7-dimethyl-exo-hydroxybicyclo(2.2.1)-hept-1-yl]methyl]sulfonyl] spiro(1H-indene-1,4′-piperidine) (60 mg, 0.135 mmol) was dissolved in THF (2 ml), treated with t-butyl isocyanate (15.4 ml, 0.135 mmol) and stirred 30 minutes at 25°C.

After removal of the solvent in vacuo, the residue was flash chromatographed on silica gel (25% $Et_2O$ in $CH_2Cl_2$ to give the title compound (27.9 mg, 38% yield) as a white solid from $Et_2O$ (m.p. 97-119°C, shrink and foam).

TLC: $R_f$= 0.21 Silica GF (20% $Et_2O$ in $CH_2Cl_2$)
PMR: Consistent with structure
HPLC: 95.1%
M.S.: (FAB) M + H + Thioglycerol = 652
Anal. Calc'd for $C_{30}H_{45}N_3O_4S$â0.25 $C_4H_{10}O$â0.30 $H_2O$
Cal'd: C, 65.58; H, 8.54; N, 7.40
Found: C, 65.70; H, 8.74; N, 7.05

EXAMPLE 53

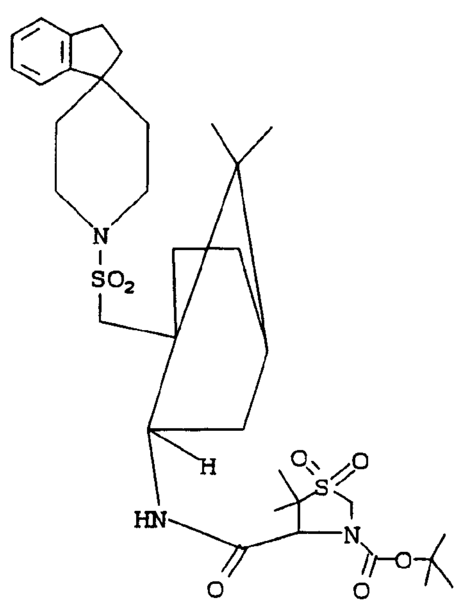

To a stirred solution of 10 ml of dry N,N-dimethylformamide containing endo(1S)-1′-(((2-amino-7,7-dimethylbicyclo(2.2.1) hept-1-yl)methyl)sulfonyl)-spiro(1H-indane-1,4′-piperidine) (670 mg, 1.66 mmole) and $N^a$-tert-butyloxycarbonyl-5,5-dimethyl-L-thiazolidine-4-carboxylic acid (500 mg, 1.91 mmole) was added 932 mg (2.11 mmole) of benzotriazol-1-yloxytris (dimethylamino)phosphonium hexafluorophosphate. The pH of the reaction mixture was adjusted to 8.5 with diisopropylethylamine and the reaction mixture was stirred at 23°C overnight. The reaction mixture was filtered and concentrated under reduced pressure. The residue was dissolved in ethyl acetate and this solution was washed in succession with saturated sodium bicarbonate solution (3 X 25 ml) and brine. The organic phase was dried (sodium sulfate) and concentrated to yield a brown solid which was purified by chromatography on silica gel (chloroform-methanol-concentrated ammonium hydroxide elution, 98:2:0.2 v/v) to yield a white solid. This material was dissolved in 10 ml of chloroform. The solution was cooled in an icebath and treated dropwise with a solution of 5 ml of chloroform containing 2.2 equivalents of m-chloroperoxy-benzoic acid. After addition was complete the ice bath was removed and stirring was continued for 24 hours. The reaction mixture was diluted to 100 ml with choroform and was washed with 1M sodium hydroxide solution (2 X 30 ml) and brine. The organic phase was dried (sodium sulfate) and concentrated under reduced pressure. The crude product was purified by chromatography on silica gel (chloroform-methanol-concentrated ammonium hydroxide elution, 98:2:0.2 v/v) to yield the title compound:

NMR: Consistent with structure and verifies presence of solvent;
HPLC: >94% pure at 214 nM;
FAB MS: 678 ($M^+$ + H);
Elem. Anal. calc'd for $C_{34}H_{51}N_3O_7S_2 \bullet 0.8CHCl_3$:
Calc'd: C, 54.04; H, 6.75; N, 5.43.
Found: C, 54.11; H, 6.64; N, 5.48.

EXAMPLE 54

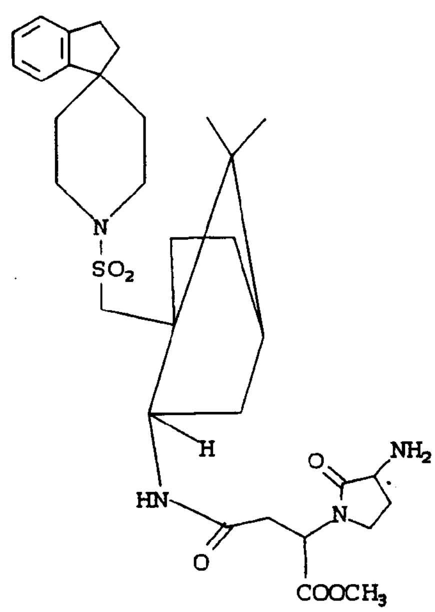

Endo(1S)-1′-(((2-amino-7,7-dimethylbicyclo-(2.2.1)hept-1-yl)methyl)sulfonyl)spiro(1H-indane-1,4′-piperi dine)(660 mg, 2.05 mmole) and (S)-3-[(tert-butyloxycarbonyl)amino]-2-oxo-1-pyrrolidine-(R)-2-methylcarboxysuccinic acid (826.5 mg, 2.05 mmole) were combined with 392 mg (2.05 mmole) of 1-ethyl-3-(3-dimethylaminopropyl) carbodiimide hydrochloride and 275 mg (2.05 mmole) of 1-hydroxybenzotriazole hydrate in 20 ml of dry methylene chloride at room temperature under nitrogen. The pH of the reaction mixture was adjusted to 8.5 with triethylamine and the resulting solution was stirred for 12 hours. The reaction mixture was diluted with methylene chloride (150 ml) and the resulting solution was washed with saturated sodium bicarbonate solution (2 X 40 ml), 10% citric acid solution (2 X 40 ml), and brine, then dried (magnesium sulfate) and concentrated to give the crude product. The analytically pure material was obtained via preparative HPLC chromatography employing a Vydac C-18 column (4.5 X 150 mm, water-acetonitrile-1% trifluoroacetic acid 45 minute gradient). The homogeneous fractions containing product were pooled and concentrated. The residue was dissolved in methylene chloride containing trifluoroacetic acid (50%)at room temperature. After 2 hours,

the volatiles were removed under reduced pressure to yield the title compound as a trifluoroacetate salt:
NMR: Consistent with structure and confirms presence of solvent;
HPLC: > 97% pure at 214 nm;
FAB MS: 615 ($M^+$ + H);
Elem. Anal. calc'd for $C_{34}H_{47}F_3N_4O_8S \bullet 0.3\ H_2O \bullet 0.8TFA$:
Calc'd: C, 51.59; H, 5.88; N, 6.74.
Found: C, 51.59; H, 5.89; N, 6.81.

EXAMPLE 55

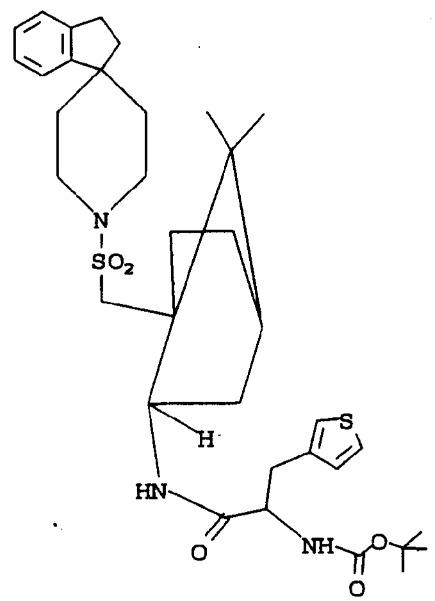

Endo-(1S)-1'-(((2-amino-7,7-dimethylbicyclo-(2.2.1) hept-1-yl)methyl)sulfonyl)spiro(1H-indane-1,4'-piperidine) (216 mg, 0.53 mmole) and $N^a$-tert-butyloxycarbonyl-D,L-(3-thienyl)alanine (160 mg, 0.59 mmole) were combined with 113 mg (0.59 mmole) of 1-ethyl-3-(3-dimethylaminopropyl) carbodiimide hydrochloride and 79 mg (0.59 mmole) of 1-hydroxybenzotriazole in 6 ml of dry methylene chloride at room temperature under nitrogen. The pH of the reaction mixture was adjusted to 9 with triethylamine and the resulting solution was stirred for 12 hours. An additional 20 mg of tert-butyloxycarbonyl-D,L-(3-thienyl)alanine, 11 mg of 1-ethyl-3-(3-dimethylaminopropyl)carbodiimide hydrochloride were added and stirring was continued for 6 hours more keeping the pH of the medium between 8 and 9. The reaction mixture was diluted with methylene chloride (150 ml) and the resulting solution was washed with saturated sodium bicarbonate solution (2 X 40 ml), 10% citric acid solution (2 X 40 ml), and brine, then dried (magnesium sulfate) and concentrated to give the crude product. The analytically pure material was obtained via flash column chromatography on silica gel (chloroform-methanol-concentrated ammonium hydroxide elution, 95:5:0.5 v/v) :
NMR: Consistent with structure and confirms presence of solvent;
HPLC: > 99% pure at 214 nm;
FAB MS: 656 ($M^+$ + H);
Elem. Anal. calc'd for $C_{35}H_{49}N_3O_5S_2 \bullet 0.5\ H_2O$:
Calc'd: C, 63.22; H, 7.58; N, 6.32.
Found: C, 63.26; H, 7.54; N, 6.10.

EXAMPLE 56

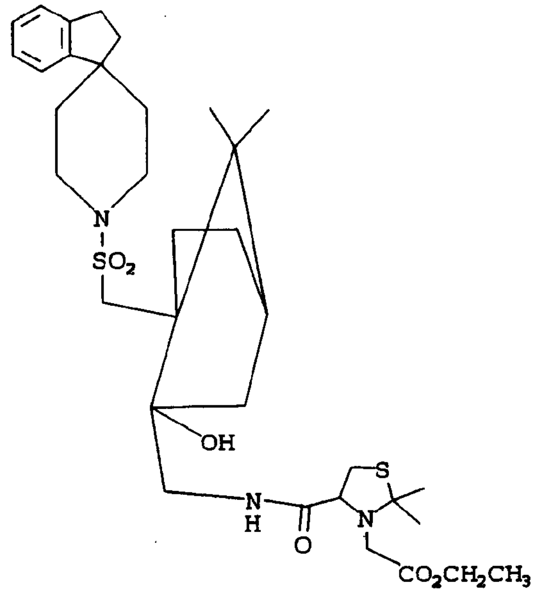

Endo(1S)-1'-(((2-amino-7,7-dimethylbicyclo-(2.2.1) hept-1-yl)methyl)sulfonyl)spiro(1H-indane-1,4'-piperidine)(67 mg, 0.156mmole) and N-ethyl-carboxymethyl-5,5-dimethyl-L-thiazolidine-4-carboxylic acid (57 mg, 0.23 mmole) were combined with 44 mg (0.23 mmole) of 1-ethyl-3-(3-dimethylaminopropyl) carbodiimide hydrochloride and 31 mg (0.23 mmole) of 1-hydroxybenzotriazole hydrate in 5 ml of dry methylene chloride at room temperature under nitrogen. The pH of the reaction mixture was adjusted to 8.5 with triethylamine and the resulting solution was stirred for 3 hours. The reaction mixture was diluted with methylene chloride (50 ml) and the resulting solution was washed with saturated sodium bicarbonate solution (2 X 40 ml), 10% citric acid solution (2 X 40 ml), and brine, then dried (magnesium sulfate) and concentrated to give the crude product. The analytically pure material was obtained via preparative thick layer chromatography (2 X 20 X 20 mm, pre-coated $SiO_2$ plates, hexane-ethyl acetate elution, 2:1 v/v). The analytical product was isolated in homogeneous form as a solid:

NMR: Consistent with structure and confirms presence of solvent;

FAB MS: 660 ($M^+$ + H);

Elem. Anal. calc'd for $C_{34}H_{47}N_3O_6S_2$•0.85 $H_2O$•0.1EtOAc:

Calc'd: C, 60.40; H, 7.59; N, 6.14.

Found: C, 60.37; H, 7.36; N, 6.13.

EXAMPLE 57

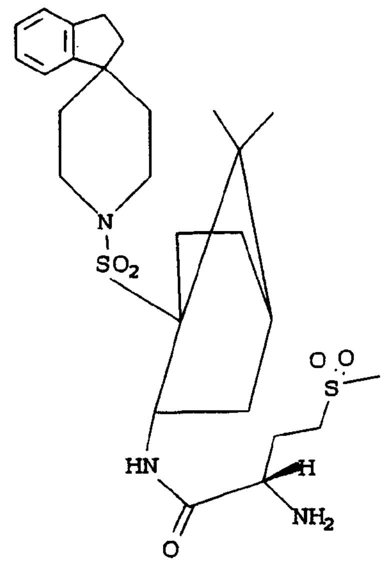

To a stirred solution of 50 ml of dry N,N-dimethylformamide containing endo(1S)-1'-(((2-amino-7,7-dimethylbicyclo(2.2.1) hept-1-yl)methyl) sulfonyl)spiro(1H-indane-1,4'-piperidine) (4.0g, 9.9 mmole) and Nα-tert-butyloxycarbonyl-L-methionine-sulfone (2.84 g, 11.9 mmole) was added 5.65 g (12.87 mmole) of benzotriazol-1-yloxytris(dimethylamino) phosphonium hexafluorophosphate. The pH of the reaction mixture was adjusted to 8.5 with diisopropylethylamine and the reaction mixture was stirred at 23°C for two hours. The reaction mixture was filtered and concentrated under reduced pressure to give a solid. This material was dissolved in ethyl acetate and this solution was washed in succession with saturated sodium bicarbonate solution (3 X 25 ml), 10% citric acid solution (3 X 25 ml) and brine. The organic phase was dried (sodium sulfate) and concentrated to yield 2-tert-butyloxycarbonyl-amino-N-[1-[[(2,3-dihydrospiro[1H-indene-1,4'-piperidin]-1'-yl)sulfonyl]methyl]-7,7-dimethylbicyclo-[2.2.1]hept-2-yl]-4-(methylsulfonyl)-butanamide which was purified by chromatography on silica gel (chloroform-methanol-concentrated ammonium hydroxide elution, 95:5:0.5 v/v) to yield a solid. This material was dissolved in 50 ml of ethyl acetate and the resulting solution was cooled to 0°C and treated with a continuous stream of hydrogen chloride gas for 15 minutes. The ice bath was removed, the reaction vessel was capped, and stirring was continued for 45 minutes more at room temperature. The solvent and excess hydrogen chloride were removed under reduced pressure to give 2-amino-N-[1-[[(2,3-dihydrospiro[1H-indene-1,4'-piperidin]-1'-yl]sulfonyl]methyl]-7,7-dimethyl-bicyclo[2.2.1]hept-2-yl]-4-(methylsulfony l)-butanamide hydrochloride as an off-white solid (6.5 g).

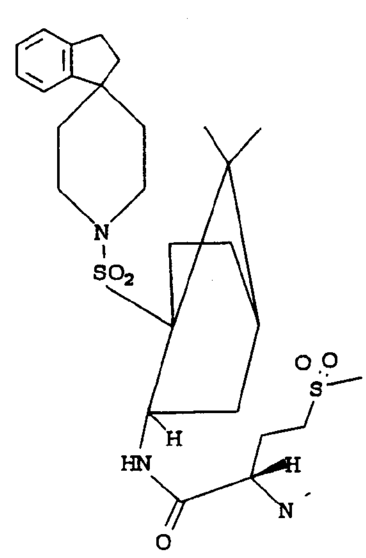

2-amino-N-[1-[[(2,3-dihydrospiro[1H-indene-1,4'-piperidin]-1'-yl)sulfonyl]methyl]-7,7-dimethyl-bicyclo[2.2.1]hept-2-yl]-4-(methylsulfonyl)-butanamide hydrochloride was chromatographed on silica gel (chloroform-methanol-concentrated ammonium hydroxide elution, 90:10:1 v/v) to afford a white solid which was dissolved in 15 ml of methanol containing 1% acetic acid. To this reaction mixture was added 3 ml of aqueous formaldehyde solution and 1.56 g (24.8 mmole) of sodium cyanoborohydride. The resulting reaction mixture was stirred at room temperature overnight. The reaction mixture was concentrated in vacuo and the residue was partitioned between ethyl acetate and saturated sodium bicarbonate solution. The phases were separated and the organic phase was washed with brine, dried (sodium sulfate), and concentrated under reduced pressure. The crude product was purified by chromatography on silica gel (chloroform-methanol-concentrated ammonium hydroxide elution, 95:5:0.5 v/v) to yield 2-dimethylamino-N-(1-[[(2,3-dihydrospiro[1H-indene-1,4'-piperidin]-1'-yl)-sulfonyl]methyl]-7,7-dimethylbicyclo[2.2.1]hept-2-yl]-4-(methylsulfonyl)-butanamide:

NMR: Consistent with structure and verifies presence of solvent;
HPLC: >98% pure at 214 nM;
FAB MS: 594($M^+$ + H);
Elem. Anal. calc'd for $C_{30}H_{47}N_3O_5S_2 \bullet 0.1CHCl_3$:
Calc'd: C, 59.67; H, 7.84; N, 6.94.
Found: C, 59.90; H, 7.24; N, 6.93.

EXAMPLE 58

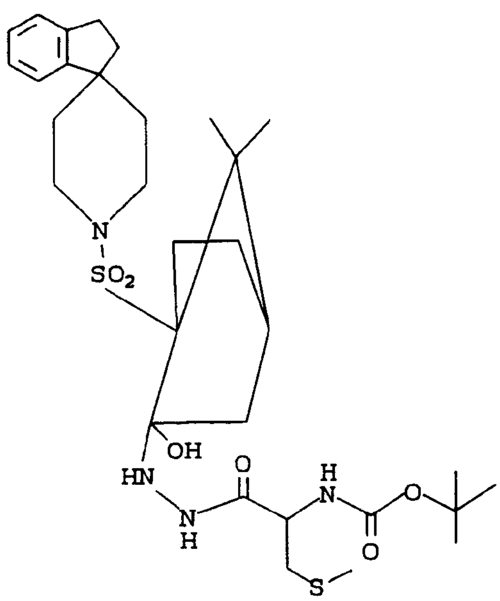

To a stirred solution of (1S)-1'-(((2-exo-hydroxy-2-endo-aminomethyl-7,7-dimethylbicyclo(2.2.1)hept-1-yl)methyl)sulfonyl)spiro(1H-indene-1,4'-piperidine) (300 mg, 0.694 mmole) in 10 ml of dry, degassed N,N-dimethylformamide was added N$^{a}$-tert-butyloxycarbonyl-S-methyl-L-cysteine (212 mg, 0.832 mmole) and 398 mg (0.902 mmole) of benzotriazol-1-yloxy tris(dimethylamino)phosphonium hexafluorophosphate (BOP) at room temperature. The resulting reaction mixture was protected from moisture and the pH was adjusted to 8-9 with diisopropylethylamine. After 24 hours all volatile components were removed under reduced pressure and the residue was partitioned between ethyl acetate and sodium bicarbonate solution. The phases were separated and the organic phase was washed with saturated sodium bicarbonate solution (3 X 40 ml) and brine, then dried (magnesium sulfate) and concentrated. The crude product was chromatographed on silica gel (hexane-ethyl acetate elution, 3:1 v/v) to give the title compound:

NMR: Consistent with structure and verifies presence of solvent;
HPLC: >98% pure at 214 nM;
FAB MS: 650 ($M^+$ + H);
Elem. Anal. calc'd for $C_{33}H_5N_3O_6S_2$:
Calc'd: C, 61.07; H, 7.71; N, 6.47.
Found: C, 61.24; H, 7.98; N, 6.13.

EXAMPLE 59

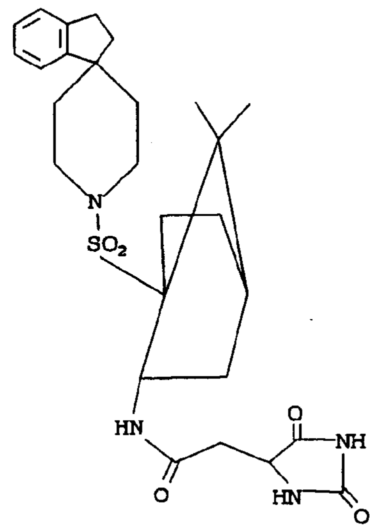

To a stirred solution of (1S)-1'-(((2-exohydroxy-2-endo-aminomethyl-7,7-dimethylbicyclo(2.2.1)hept-1-yl)methyl)sulfonyl)spiro(1H-indene-1,4'-piperidine) (125 mg, 0.311 mmole) in 7 ml of dry, degassed N,N-dimethylformamide was added S-hydantoinacetic acid (59 mg, 0.372 mmole) and 178 mg (0.404 mmole) of benzotriazol-1-yloxy tris(dimethylamino)phosphonium hexafluorophosphate (BOP) at room temperature. The resulting reaction mixture was protected from moisture and the pH was adjusted to 8-9 with diisopropylethylamine. After 24 hours all volatile components were removed under reduced pressure and the residue was partitioned between ethyl acetate (100 ml) and sodium bicarbonate solution. The phases were separated and the organic phase was washed with saturated sodium bicarbonate solution (3 X 40 ml) and brine, then dried (magnesium sulfate) and concentrated. The crude product was initially flash chromatographed on silica gel (chloroform-methanol elution, 96:4, v/v) to give semi-pure material from which the title compound was obtained as a white solid after preparative thick layer chromatography on silica gel (chloroform-methanol-concentrated ammonium hydroxide elution, 96:4:0.4, v/v) and trituration with ether-petroleum ether:

NMR: Consistent with structure and verifies presence of solvent;
FAB MS: 543 ($M^+$ + H);
Elem. Anal. calc'd for C28H48N405S:
Calc'd: C, 61.96; H, 7.00; N, 10.32.
Found: C, 61.70; H, 7.36; N, 10.09.

EXAMPLE 60

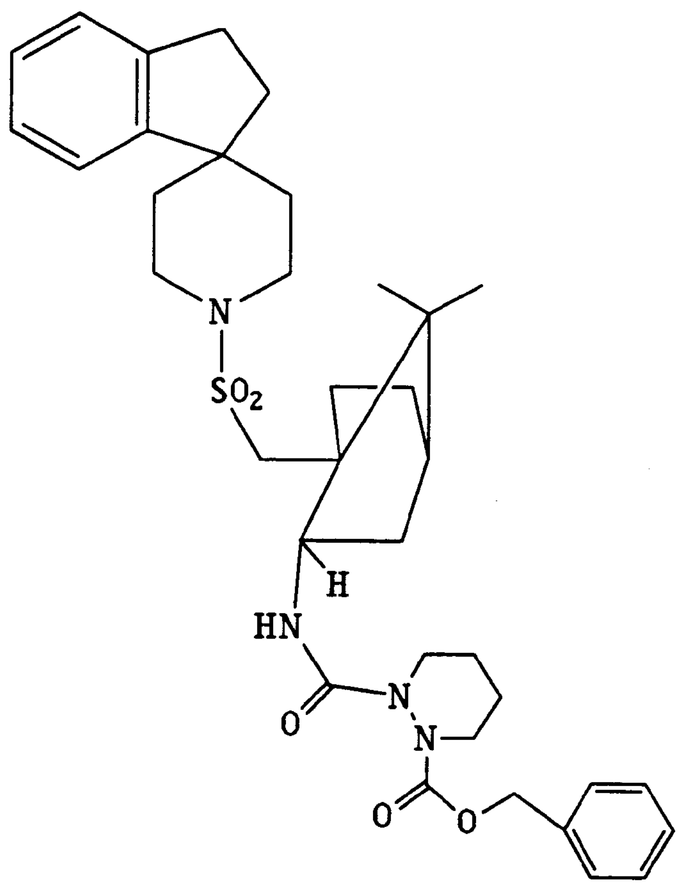

(1S)-1'-(((7,7-dimethyl-2-endo-(4-nitrophenyloxycarbbonylamino)-bicyclo-(2.2.1)-hept-1-yl)-methyl)-sulfonyl)spiro(1H-indan-1,4'-piperidine

(1S)-1'-(((7,7-dimethyl-(2-endoamino)-bicyclo(2.2.1)-hept-1-yl)-methyl)sulfonyl)spiro(1H-indene-1,4-pipe rdine)[3.47mmol] and 4-Nitrophenyl chloroformate [3.64mmol] were combined in THF. The reaction mixture was treated with triethylamine [4.54 mmol] and allowed to stir for 3 hours. The reaction mixture was concentrated to dryness and the resulting residue was purified by a silica gel column, while eluting with 1% ethylacetate in methylene chloride. The product fractions were combined and concentrated to dryness in vacuo. (1S)-1'-(((7,7-dimethyl-(4-nitrophenyloxycarbonyl-2-endoamino)-bicyclo-(2.21)-hept-1-yl)-methyl)-sulfonyl)spiro(1H-indene -1,4'-piperidine was obtained as a white solid from ether.

(1S)-1'-(((7,7-dimethyl-2-endo-(4-nitrophenyloxycarbonylamino)-bicicylo-(2.2.1)-hept-1-yl)-methyl)sulfo nyl)spiro(1H-indene-1,4'-piperdine)-[0.278mmol] and (benzyloxycarbonyl) piperazic acid [0.334mmol] were combined in DMF. The reaction mixture was treated with triethylamine [0.401mmol] and allowed to stir for 2 hours. The reaction mixture was concentrated to dryness and the resulting residue was dissolved in $CH_2Cl_2$. This solution was placed on a silica gel column and eluted with 5% methanol in $CH_2Cl_2$ and then with 96/4/0.4 of $CH_2Cl_2$/methanol/acetic acid. The product fractions were combined and evaporated to dryness. The title compound was obtained as a white solid from ether and was dried in vacuo overnight.

| | |
|---|---|
| m.p.: | 90-120°C |
| NMR: | Consistent with structure |
| HPLC: | >98% Pure |
| MS: | M-->$H^+$=693.6 |
| CHN: | CALC'D FOR $C_{37}H_{48}N_4O_7S$·0.20mol $C_4H_{10}O$·0.25mol $H_2O$; C-63.74, H-7.15,N-7.87. Found C-63.78, H-7.08,N-7.81. |

EXAMPLE 61

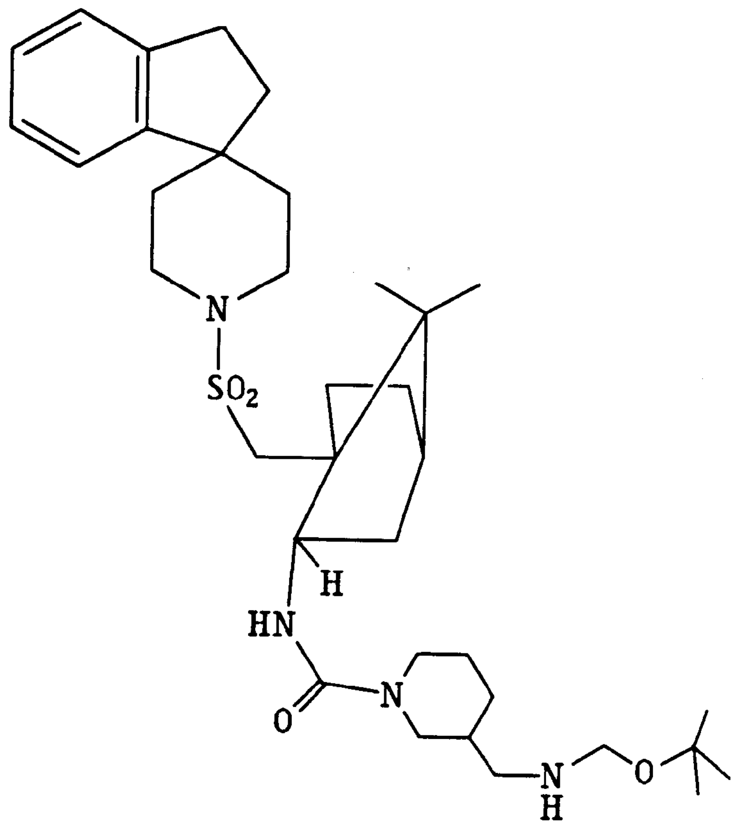

The procedure of Example 60, second paragraph was carried out using (1S)-1′-(((7,7-dimethyl-2-endo-(4-nitrophenyloxycarbonylamino)-bicyclo-(2.2.1)-hept-1-yl)-methyl)sulfonyl)spiro(1H-indan-1,4′-piperidine)[0.193mmol], triethylamine [0.29mmol], and substituting 3-(t-butoxycarbonylaminomethyl)piperidine [0.212mmol] for (benzyloxycarbonyl) piperazic acid. Chromatographic elution was with 5% ether in $CH_2Cl_2$, 10% ether in $CH_2Cl_2$, and then 1% methanol in $CH_2Cl_2$. The title compound was obtained from ether and dried in vacuo, overnight.

| | |
|---|---|
| m.p.: | 95-105°C |
| NMR: | Consistent with structure |
| HPLC: | >97% Pure |
| MS: | $M+H^+$=643.4 |
| CHN: | CALC'D FOR $C_{35}H_{54}N_4O_5S \cdot 0.20\ H_2O$; C-65.02,H-8.48,N-8.67. Found C-64.98,H-8.43,N-8.86. |

EXAMPLE 62

Endo-(1S)-1'-(((2-(L-4(tert,butoxycarbonylamino)glutaramyl)amino-7,7-dimethylbicyclo(2.2.1)hept-1-yl)-methyl)-sulfonyl)spiro-(1H)-indan-1,4',piperidine

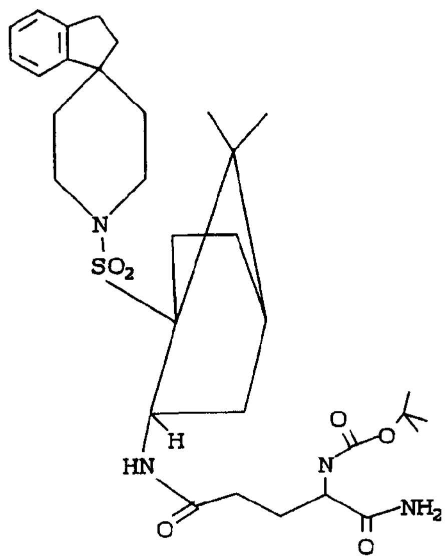

In an oven dried flask (50ml) under nitrogen was dissolved endo-(1S)-1'-(((2-amino-7,7-dimethyl-bicyclo(2.2.1)hept-1-yl)-methyl)-sulfonyl)spiro-(1H)-indan-1,4'-piperidine (50mg, 0.125mmol), L-4(tert-butoxycarbonyl)glutaramic acid (34mg, 0.14mmol), 1-ethyl-3-(3-dimethylaminopropyl)carbodiimide hydrochloride (30mg, 0.15mmol), and 1-hydroxybenztriazole hydrate (20mg 0.15mmol) in dimethyl formamide (1ml). Triethylamine (50μl) was added and a white solid separated. After stirring for 1 hour, the solvent was removed under reduced pressure. The residue was dissolved in ether:methylene chloride (3:1). The cloudy solution was washed with sodium bicarbonate (sat., aqueous), water, potassium hydrogen sulfate (10%, aqueous) and brine. After drying over sodium sulfate, the organic material was filtered and concentrated. Silica "flash" chromatography using methylene chloride methanol (9:1) gave the product which was isolated as an oil upon evaporation of the solvents. Hexane:ether addition and removal under reduced pressure produced the title compound as a white foam.

| | |
|---|---|
| HPLC: | >99% |
| MS: | $M+H^+$=631.3 |
| NMR | consistent with structure |
| Analysis | For $C_{33}H_{50}N_4O_6S$•0.5 DMF•0.5 $H_20$ |
| | Cal'd C-61.26,H-8.12,N-9.32. |
| | Found C-61.27,H-8.05,N-9.31. |

EXAMPLE 63

Endo-(1S)-1'-(((2-(4(imidazole-2-ethylacetyl)amino-7,7-dimethylbicyclo(2.2.1)hept-1-yl)-methyl)spiro-(1H)-indan-(1,4'),piperdine hydrochloride

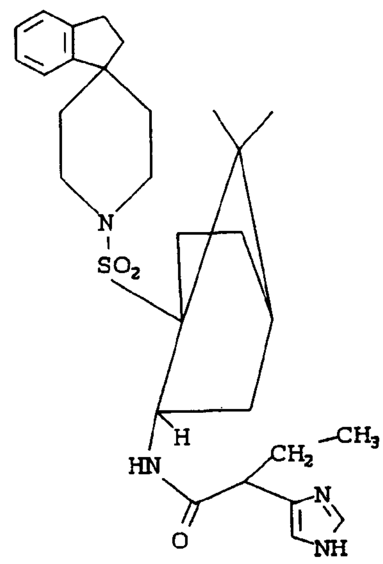

Step 1: Endo-(1S)-1'-(((2-4(3-methylbenzyloxy)imidazole)-2(ethyl acetyl)amino-7,7-dimethylbicyclo-(2.2.1)hept-1-yl)-methyl)-sulfonyl)spiro(1H)-indan-(1,4')-piperidine

Into an over dried flask (50ml) under nitrogen was placed dimethylformamide (4ml). Endo(1S)-1'-((2-amino-7,7-dimethylbicyclo(2.2.1)hept-1-yl)-methyl)sulfonyl)spiro(1H)-indan-(1,4')-piperidine (300mg,0.74mmol), 4(3-methylbenzyloxy)imidazole-2-ethylacetic acid (219mg, 0.8mmol), 1-ethyl-3-methyl-(3-dimethylaminopropyl)carbodiimidehydrochloride(153mg, 0.8mmol) and 1-hydroxybenztriazolehydrate(108mg, 0.83mmol) were added and the mixture was stirred until solution was achieved. Triethylamine (400μl) was added to make the solution basic (pH 9) and a solid separated. After stirring overnight room temperature, the solvent was removed under reduced pressure. The resulting gum was dissolved in methyenechloride:ether (1:3) and the cloudy solution was extracted with sodium bicarboante (sat., aqueous) and brine. After drying the organic layers over sodium sulfate, the solution was filtered and was concentrated. The product was purified by silica gel "flash" chromatography using methylene chloride, methanol, ammonium hydroxide (9:1:1) as solvents. The product fractions were concentrated to give the title compound as white foam.

| | |
|---|---|
| HPLC: | >95% (42% + 53%) |
| MS: | $M+H^+$=659.3 |
| NMR ($CDCl_3$) | consistent with structure |
| Analysis | For $C_{38}H_{50}N_4O_4S$•$0.65H_20$ |
| | Cal'd C-68.05,H-7.71,N-8.36. |
| | Found C-38.03,H-7.87,N-8.60 |

Nitrogen was bubbled into a solution of Endo(1S)-1'-(((2-4-(3-methylbenzyloxyimidazole)-2-ethylacetylamino-7,7-dimethylbicyclo(2.2.1)hept-1-yl)-methyl)-sulfonyl)spiro-(1H)-indan-(1m4')piperidine (50mg, 0.076mmol) in absolute ethanol (20ml)-acetic acid (5ml). After 5 minutes, 10%Pd/C(50mg) was added and the mixture was hydrogentated at 55psi overnight. The catalyst was removed by filtration and the solvents were concentrated to dryness under reduced pressure. The residue was purified by silica gel "flash" chromatography using methylenechloride, methanol,ammonium hydroxide (9:1:1) as solvent. The product fractions were collected and concentrated. The gum was redissolved in methylene chloride and was filtered through a sintered glass funnel. After the solution was concentrated, the residue was dissolved in ethyl acetate (5ml). The solution was cooled to 0° and it was saturated with hydrogen chloride gas (bubbling 5 minutes). The solvent was removed under reduced pressure and ether was added and was removed to give the title compound as a white solid.

| | |
|---|---|
| HPLC: | >90% (42% + 45%) |
| MS: | $M+H^+$=539.2 (freebase) |
| NMR ($CDCl_3$) | consistent with structure |

Analysis for $C_{30}H_{42}N_4O_3S$•HCl•0.25 ether•130$H_2O$
Cal'd C-60.33,H-7.86,N-9.08.
Found C-60.33,H-7.48,N-9.04.

EXAMPLE 64

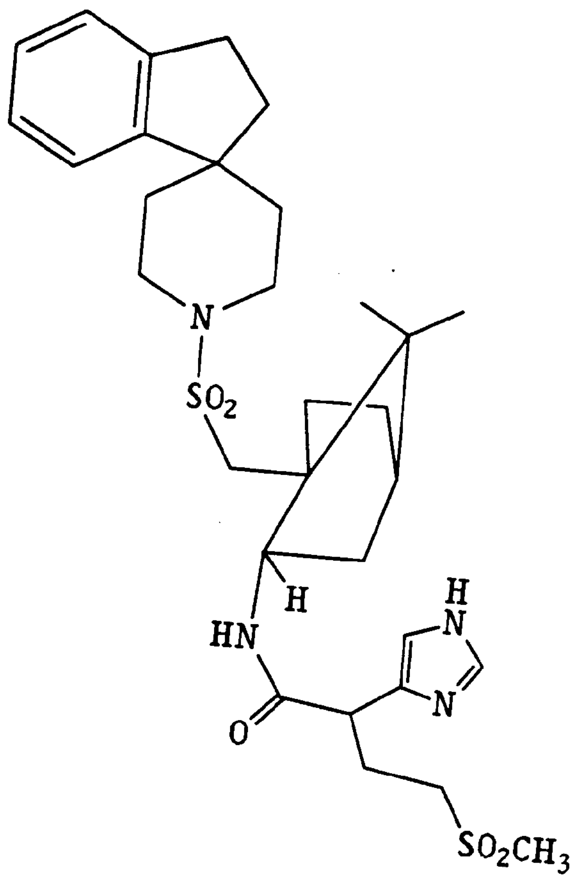

To a stirred solution of the product of Example A (500 mg, 1.24 mmol) in DMF (10 mL) was added the HCl salt of 2-(1-benzyloxymethylimidazoly-5-yl)-4-methyl-sulfonylbutanoic acid (506 mg; 1.30 mmol), DIEA (0.70 mL; 4.0 mmol), and BOP (600 mg; 1.35 mmol). After being stirred at ambient temperature for 14 h, the solvent was removed under reduced pressure. The residue was dissolved in EtOAc (75 mL) and washed with aqueous $NaHCO_3$ (3 x 50 mL). The organic phase was dried (MgSO4), filtered, and the solvent was removed under reduced pressure. The benzyloxymethyl protecting group was removed by dissolving the residue in a 4:1 solution of EtOH-HOAc (10 mL) and hydrogenating over palladium black (75 mg) under 1 atm of hydrogen for 24 h. The catalyst was removed by filtration through Celite and the filtrate solvents were removed under reduced pressure. The residue was purified by pressurized silica gel column chromatography using a 50:50:7 by volume mixture of $CHCl_3$, EtOAc, and 4% $NH_4OH$-MeOH as eluant. Two diastereomers were obtained and each was lyophilized from water containing 1% TFA. The title compound is the lower Rf isomer.

Higher Rf Isomer:
Anal: ($C_{31}H_{44}N_4O_5S_2$)·1.0 TFA·1.1 $H_2O$
calc. C 52.45 H 6.24 N 7.37
found C 52.69 H 6.12 N 7.32
TLC: $R_f$ 0.33 (50:50:7 $CHCl_3$:EtOAc:4%$NH_4OH$-MeOH)
HPLC (method A): retention time 8.96 min
FAB MS: m/z 617 ($M^+$ + H)
$^1H$ NMR (300 MHz, $CDCl_3$): free base: d 7.70 (s, 1H), 7.55 (br d, 1H), 7.15-7.25 (m, 4H), 7.00 (s, 1H), 4.35 (m, 1H), 2.90 (s, 3H), 1.00 (s, 3H), 0.98 (s, 3H)
Lower Rf Isomer:
anal: ($C_{31}H_{44}N_4O_5S_2$)·1.0 TFA·1.3 $H_2O$

calc. C 52.55 H 6.36 N 7.43
found C 52.89 H 6.13 N 7.43
TLC: $R_f$ 0.28 (50:50:7 $CHCl_3$:EtOAc:4%$NH_4OH$-MeOH)
HPLC (method A): retention time 9.15 min
FAB MS: m/z 617 ($M^+$ + H)
$^1H$ NMR (300 MHz, $CDCl_3$): free base: d 7.72 (s, 1H), 7.34 (br d, 1H), 7.15-7.25 (m, 4H), 6.98 (s, 1H), 4.35 (m, 1H), 2.90 (s, 3H), 1.00 (s, 3H), 0.96 (s, 3H)

EXAMPLE 65

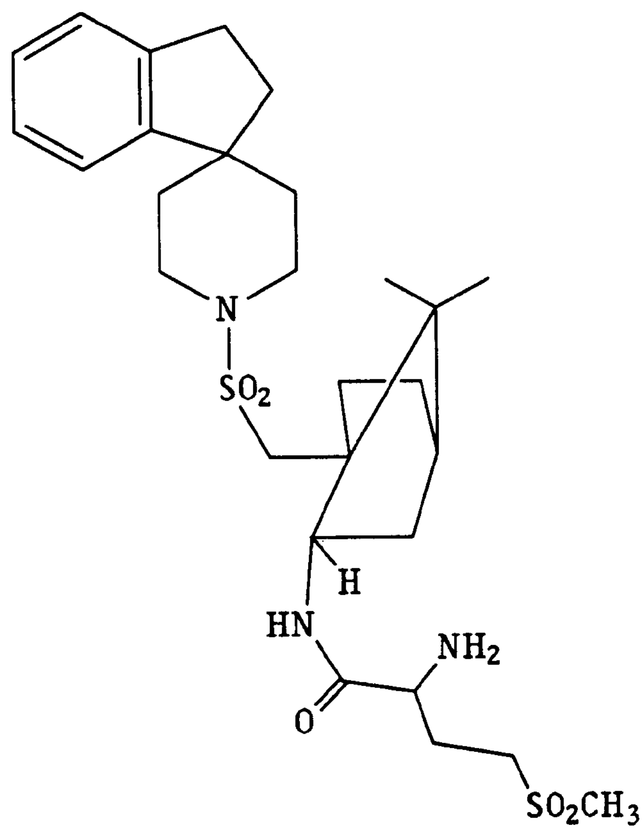

To a 0°C stirred solution of the product of Example A (1.10 g; 2.74 mmol) in $CHCl_3$ (100 mL) was added DIEA (0.75 mL; 4.3 mmol) and benzyl chloroformate (0.51 g; 3.0 mmol). The solution was stirred at 0°C for 1 h and then at ambient temperature for 14 h. The reaction mixture was concentrated under reduced pressure and the residue was dissolved in 25 mL of a solution of 10:1 MeOH-NH4OH. The mixture was concentrated under reduced pressure, the residue was dissolved in $CHCl_3$ (100 mL) and washed with 5% aqueous HCl (2 x 50 mL) and aqueous $NaHCO_3$ (100 mL). The organic phase was dried ($MgSO_4$), filtered, and the solvent was removed under reduced pressure. The residue was purified by pressurized silica gel column 5 chromatography using 1:4 EtOAc-hexanes as eluant. The benzyl urethane was obtained as a white foam.
TLC: $R_f$ 0.40 (1:3 EtOAc:hexanes)
HPLC (method A): retention time 12.25 min
FAB MS: m/z 537 ($M^+$ + H)
To a 0°C stirred solution of the benzyl urethane (1.25 g; 2.33 mmol) in DMF (20 mL) was added iodomethane (0.435 mL; 7.00 mmol) and sodium hydride (0.140 mg of a 60% dispersion in mineral oil; 3.50 mmol). The solution was stirrred at 0°C for 1 h and then at ambient temperature for 18 h. The reaction mixture was treated with HOAc (1 mL) and the solvents were removed under reduced pressure. The residue was dissolved in EtOAc (100 mL) and washed with aqueous $NaHCO_3$ (2 x 50 mL). The organic phase was dried ($MgSO_4$), filtered, and the solvent was removed under reduced pressure. The residue was purified by pressurized silica gel column chromatography using 1:5 EtOAc-hexanes as eluant. The N-methyl benzyl urethane was obtained as a white foam.
TLC: $R_f$ 0.34 (1:4 EtOAc:hexanes)

HPLC (method A): retention time 12.71 min
FAB MS: m/z 551 ($M^+$ + H)
To a stirred, argon purged solution of the N-methyl benzyl urethane (1.00 g; 1.82 mmol) in 96:4 MeOH-$HCO_2H$ (25 mL) was added palladium black (0.37 g). The reaction mixture was stirrred for 16 h at ambient temperature. The catalyst was removed by filtration through Celite, and the filtrate solvents were removed under reduced pressure. The residue was purified by pressurized silica gel column chromatography using 95:5:0.5 $CHCl_3$:MeOH:$NH_4OH$ as eluant. The N-methyl endo-amine product was obtained as a white foam.
TLC: $R_f$ 0.35 (92:8:0.8 $CHCl_3$:MeOH:$NH_4OH$)
HPLC (method A): retention time 7.88 min
FAB MS: m/z 417 ($M^+$ + H)
To a stirred solution of the N-methyl endo-amine (0.650 g; 1.56 mmol) in $CHCl_3$ (50 mL) was added the acid fluoride of Na-Boc-L-methionine sulfone (510 mg; 1.80 mmol) and DIEA (0.35 mL; 2.0 mmol). The mixture was stirred at ambient temperature for 48 h, and then extracted with 10% aqueous citric acid solution (2 x 30 mL), water (30 mL), and aqueous NaHCO3 (2 x 30 mL). The organic phase was dried ($MgSO_4$), filtered, and the solvent was removed under reduced pressure. The residue was dissolved in $CH_2Cl_2$ (10 mL), and to the solution was added TFA (6 mL). The mixture was stirred at ambient temperature for 1.5 h. The solvents were removed under reduced pressure and the residue was purified by preparative reverse phase HPLC using a water-acetonitrile gradient containing 0.1% TFA. The TFA salt of the title compound was obtained as a lyophilized powder.

| | |
|---|---|
| anal: | $(C_{29}H_{45}N_3O_5S_2)\cdot$0.4 TFA$\cdot$0.5 EtOAc |
| calc. | C 57.04 H 7.44 N 6.28 |
| found | C 57.04 H 7.55 N 6.28 |

TLC: $R_f$ 0.18 (95:5:0.5 CHCl3:MeOH:NH4OH)
HPLC (method A): retention time 9.80 min
FAB MS: m/z 580 ($M^+$ + H)
$^1$H NMR (300 MHz, $CDCl_3$): d 7.15-7.25 (m, 4H), 5.20 (m, 2H), 3.17 (s, 3H), 2.92 (s, 3H), 1.06 (s, 3H), 0.96 (s, 3H)

EXAMPLE 66

To a stirred solution of endo-(1S)-1′(((2-amino-7,7-dimethylbicyclo(2.2.1)-hept-1-yl)-methyl)-sulfonyl)spiro(1H-indan-1,4′-piperidine (94 mg; 0.17 mmol) in ethanol (20 mL) was added N-(3-bromopropyl)-phthalimide (69 mg; 0.255 mmol) followed by diisopropylethyl amine (0.044 mL; 0.255 mmol). The temperature was then increased to 50 °C. After approximately 18 hr the solution was cooled then concentrated under reduced pressure. The residue was dissolved in methylene chloride (150 mL), washed with 1M HCl (2 x 150 mL) then dried over sodium sulfate and concentrated. Purification by flash chromatography (5% methanol in methylene chloride) afforded the title compound (45 mg; 40%) as a white solid.

| | |
|---|---|
| Anal.: | $(C_{39}H_{50}N_4O_6S)\cdot$0.10 $H_2O\cdot$0.15 $CH_2Cl_2$ |
| Calc. | C, 65.54; H, 7.10; N, 7.81 |
| Found | C, 65.52; H, 7.09; N, 7.71 |

HPLC: (Vydac C18 Column; gradient from 95/5 to /100 H20/CH3CN with 0.1% TFA. 15 min. gradient, flow rate = 1.5 ml/min.)
$R_t$ = 13.81 min. Purity = 100%
$^1$HNMR: Consistent with structure
FABMS: m/z = 703 ($M^+$ + H)

In addition to those compounds specifically exemplified above, additional compounds of the present invention are set forth in tabular form below. These compounds are synthesized by use of the synthetic routes and methods described in the above Schemes and Examples and variations thereof well known to those of ordinary skill in the art, and not requiring undue experimentation. All variables listed in the Tables below are with reference to the following generic structure:

R =

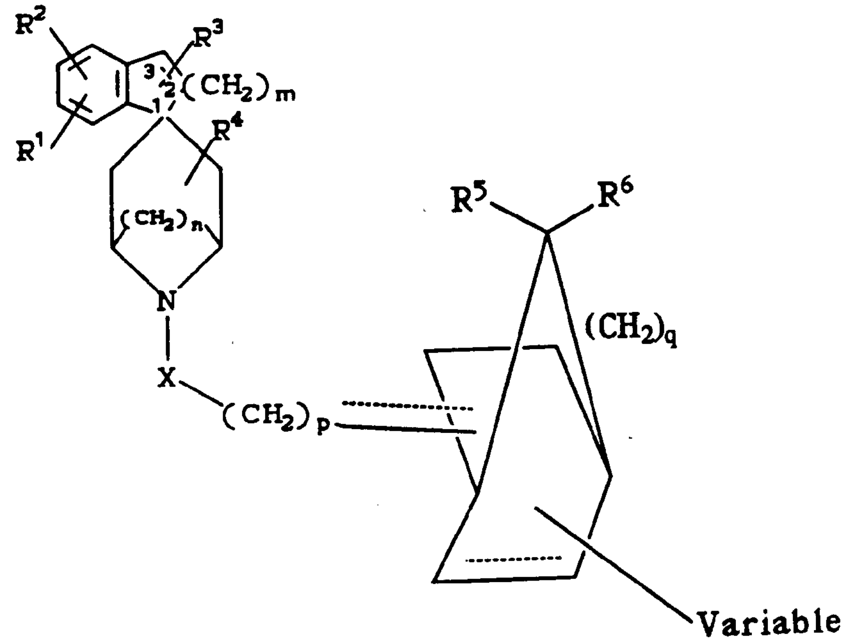

TABLE 1

Variable =

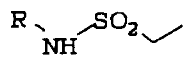

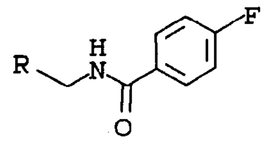

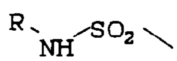

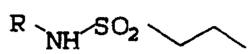

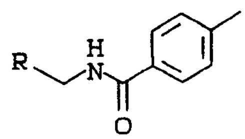

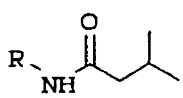

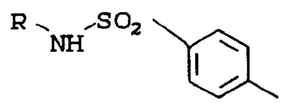

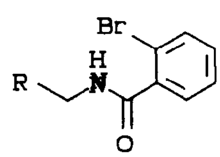

TABLE 1 cont'd

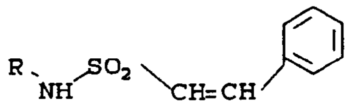

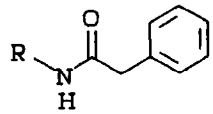

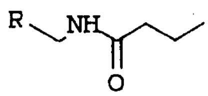

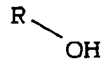

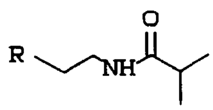

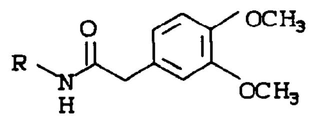

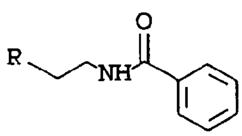

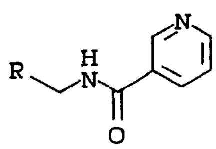

TABLE 1 cont'd

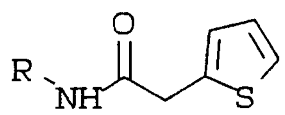

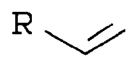

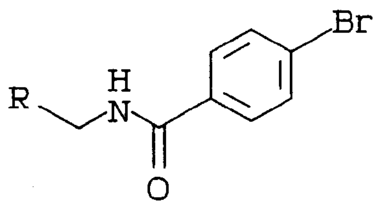

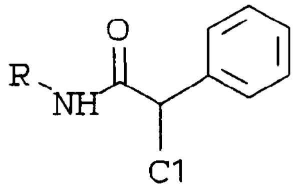

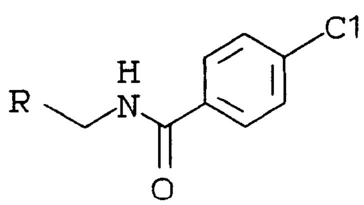

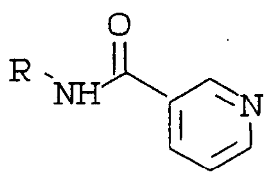

TABLE 2

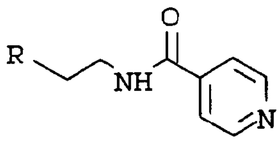

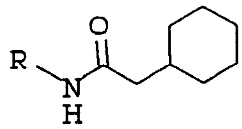

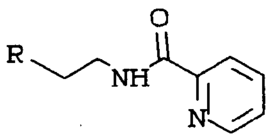

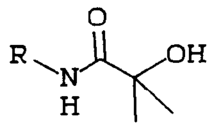

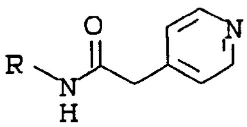

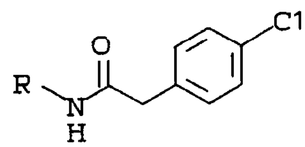

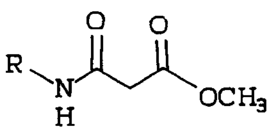

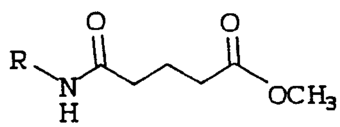

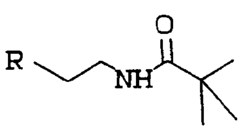

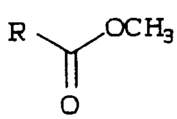

TABLE 2 cont'd

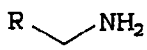

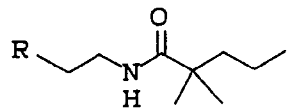

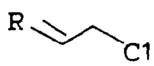

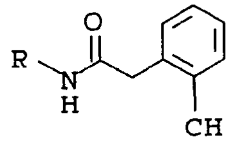

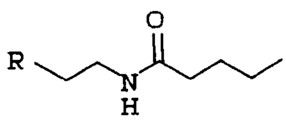

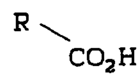

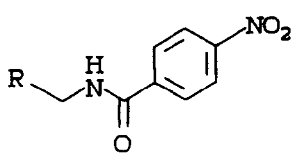

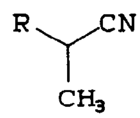

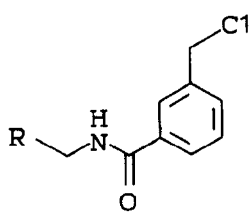

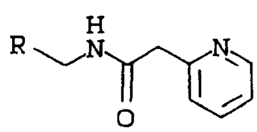

TABLE 3

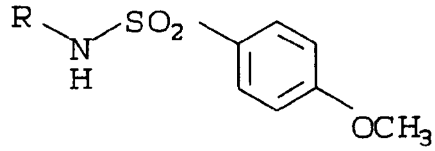

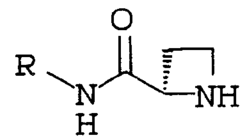

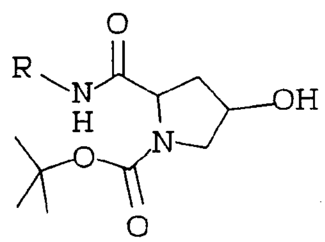

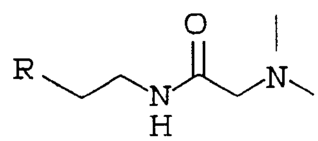

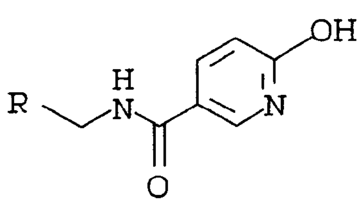

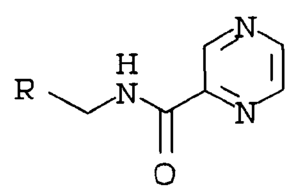

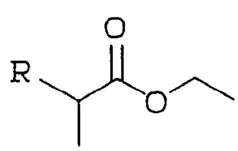

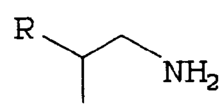

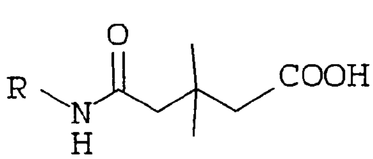

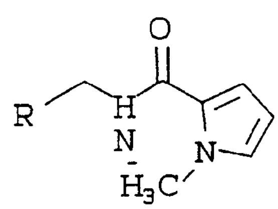

TABLE 3 cont'd

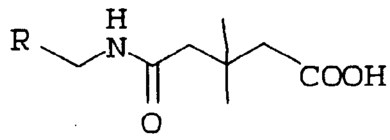

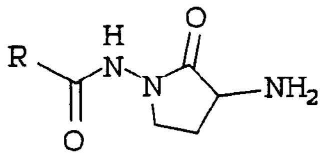

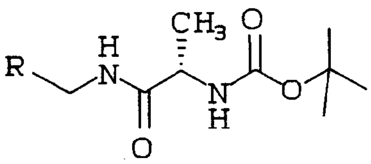

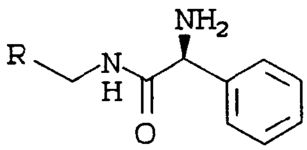

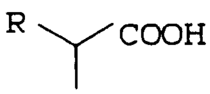

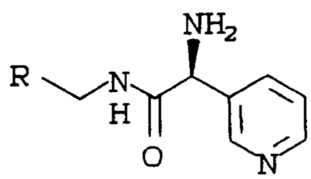

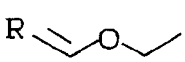

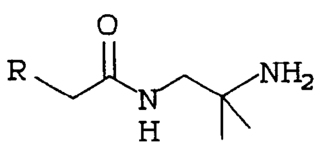

TABLE 4

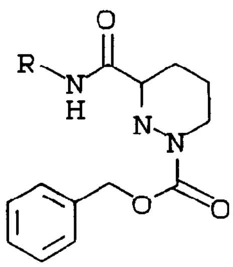

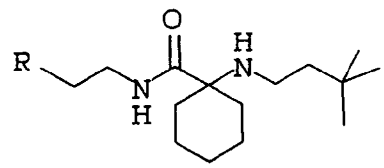

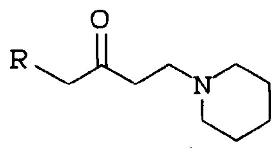

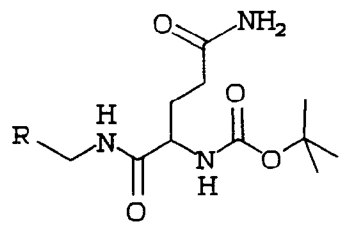

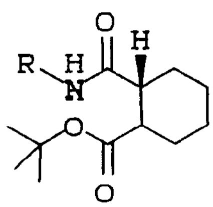

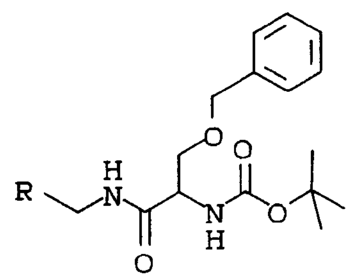

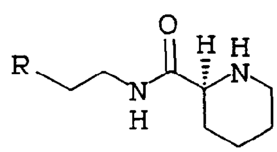

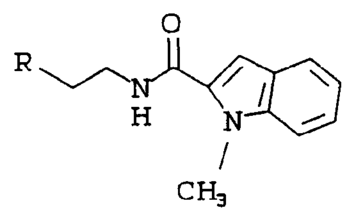

TABLE 4 cont'd

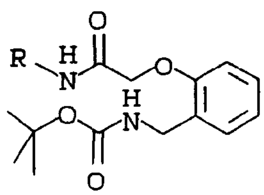

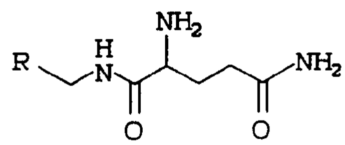

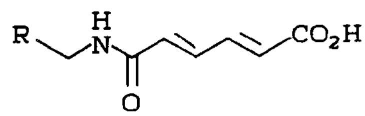

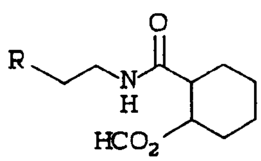

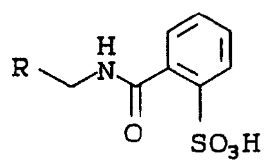

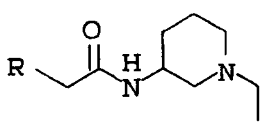

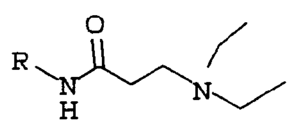

TABLE 5

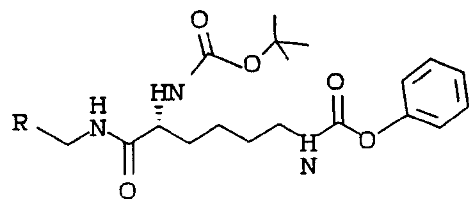

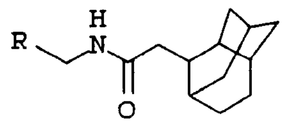

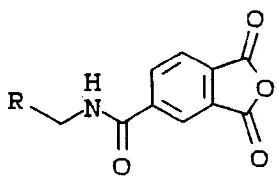

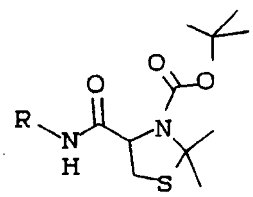

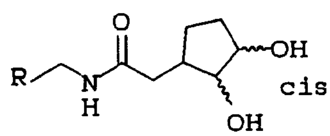

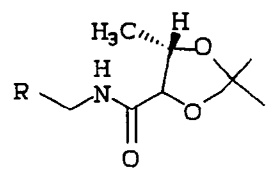

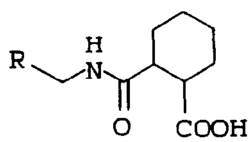

TABLE 5 cont'd

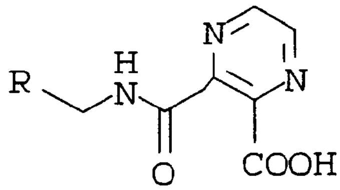

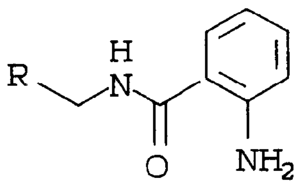

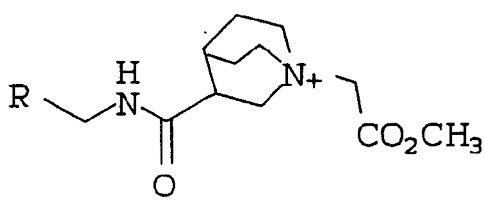

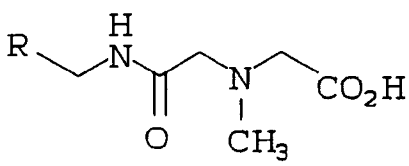

TABLE 6

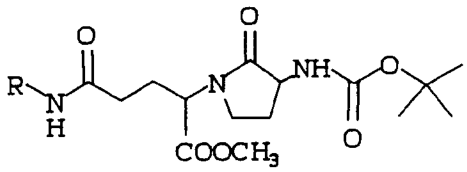

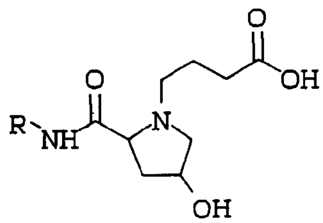

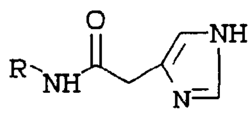

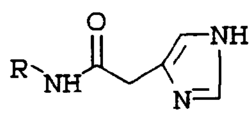

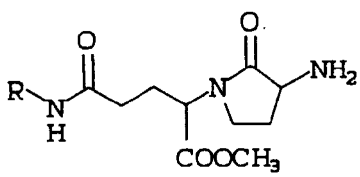

TABLE 6 CONT'D

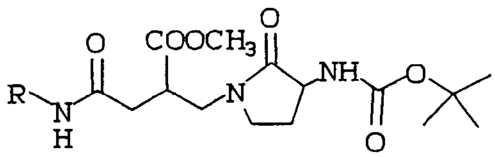

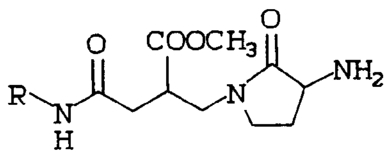

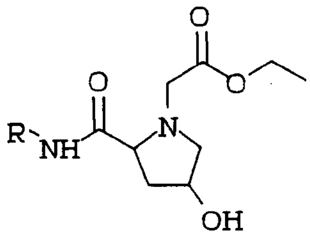

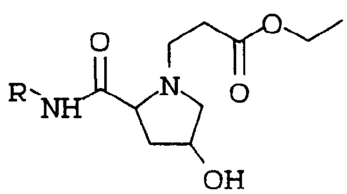

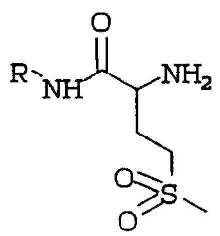

TABLE 7

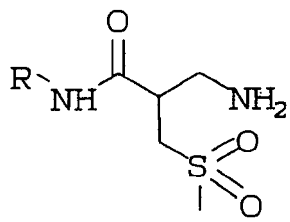

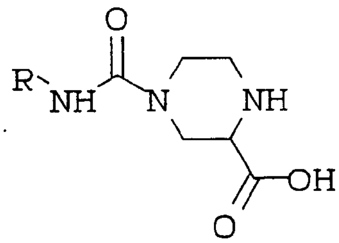

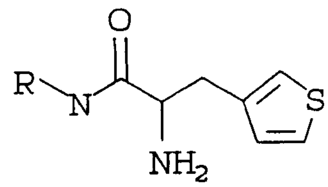

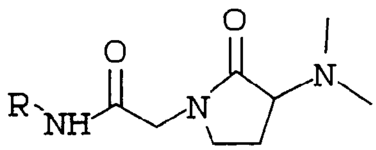

TABLE 7 cont'd

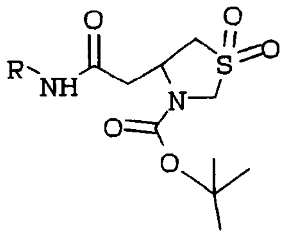 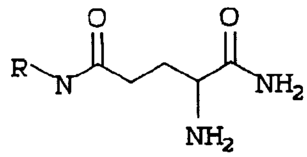

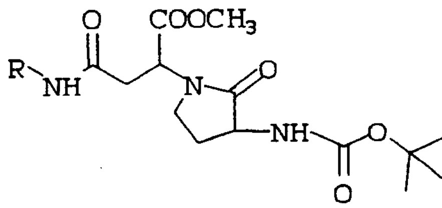 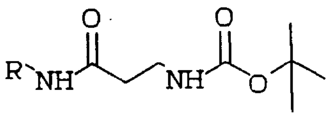

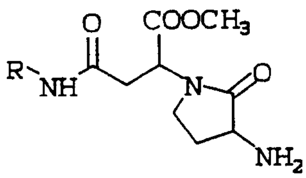 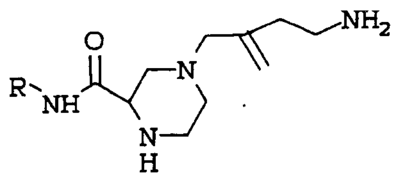

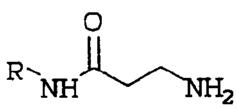

TABLE 8

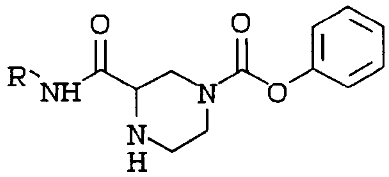

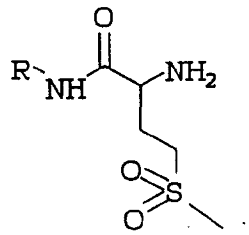

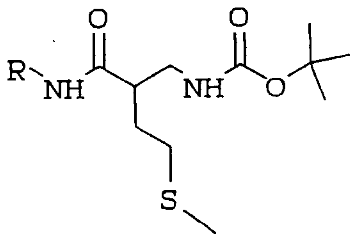

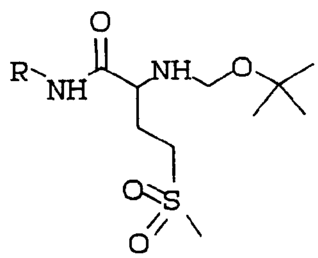

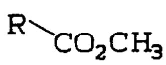

TABLE 8 cont'd

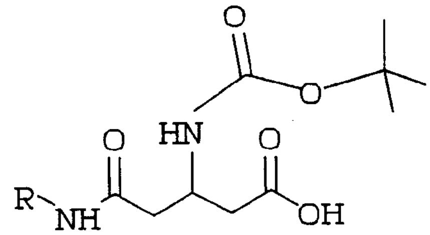

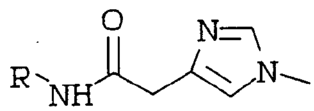

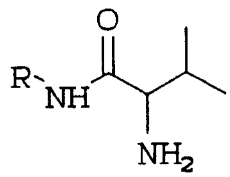

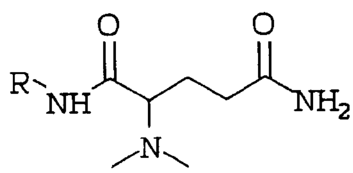

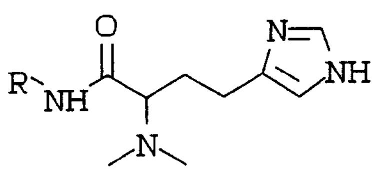

TABLE 9

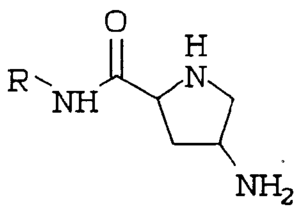

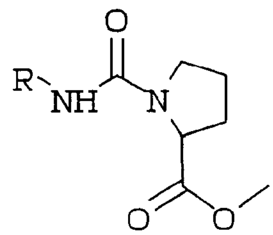

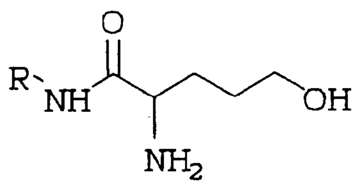

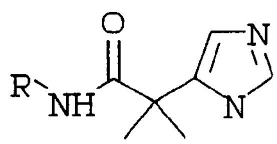

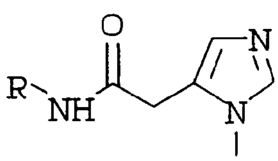

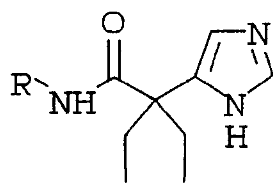

TABLE 9 cont'd

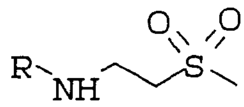

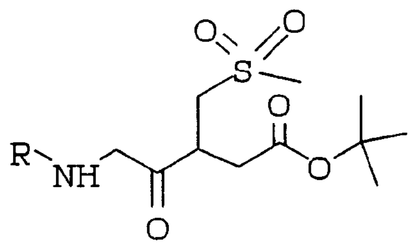

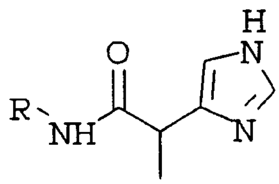

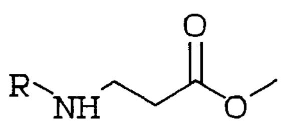

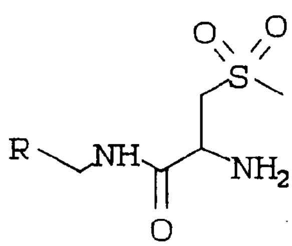

## TABLE 10

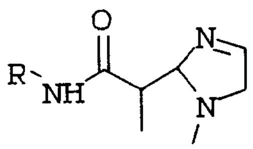

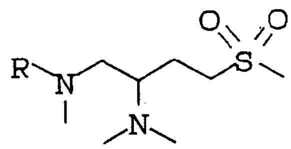

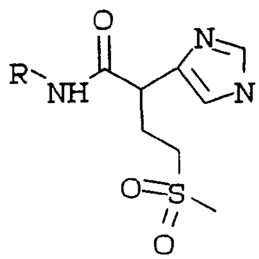

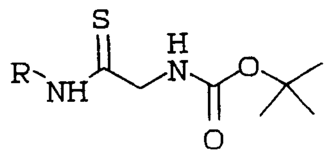

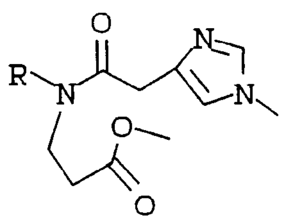

TABLE 10 cont'd

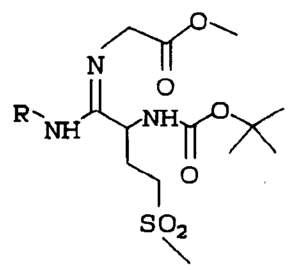

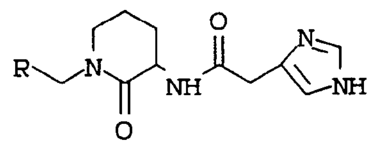

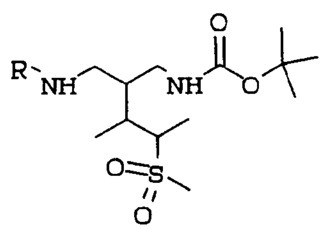

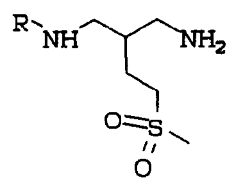

RADIOLIGAND BINDING ASSAYS

The high affinity binding of [$^3$H] Oxytocin(OT)([tyrosyl, 3,5-[$^3$H]OT; 30-60 Ci/mmol; New England Nuclear. Boston, MA) to uterine OT receptors was based on an assay* using a crude membrane preparation of uteri taken from diethylstilbestrol dipropionate (DES)-treated (0.3 mg/kg, ip; 18-24) rats. Competition studies were conducted at equilibrium (60 minutes; 22°C) using 1 nM[$^3$H]OT in the following assay buffer: 50 mM Tris-HCl, 5 mM $MgCl_2$, and 0.1% BSA, pH 7.4. Nonspecific binding (10% of the total binding) was determined using 1mM unlabeled OT and the binding reaction was terminated by filtration through glass fiber filters using a cell harvester (model 7019, Skatron, Inc., Sterling, VA).

The measurement of [$^3$H]Vasopressin (AVP) ([phenylalanyl-3,4,5-$^3$H]AVP; 80-90 Ci/mmol; New England Nuclear) binding to a crude membrane preparation of male rat liver (AVP-$V_1$ sites) or kidney medulla (AVP-$V_2$ sites) was determined according to the method of Butlen, et al.** Competition assays were conducted at equilibrium (30 minutes at 30°C) using 1 nM [$^3$H]AVP (liver) or 2 nM [$^3$H]AVP (kidney) in the following assay buffer: 100 mM Tris-HCl, 5 mM $MgCl_2$, 0.1% BSA, 50 mM phenylmethylsulfonylfluoride, and 50 mg/ml bactracin, pH 8.0. Nonspecific binding (5-10% of the total binding) was determined using 10 mM unlabeled AVP, and the binding reaction was terminated by filtration as described above for the [$^3$H]OT binding assay.

$K_i$; values were obtained for each compound from three to six separate determinations of the $IC_{50}$ values ($K_i = IC_{50}/l + c/K_d$)*** using $K_d$ values obtained from saturation binding assay: [$^3$H]OT (uterus), 0.7 nM; [$^3$H]AVP

* Fuchs, A-R; Fuchs, F; Soloff, MS. 1985 J. Clin. Endocrinol. Metab. 60:37.
** Butlen, D; Guillon; G; Rajerison, R.M.; Jard, S; Sawyer, W.H.; Manning, M. 1978 Mol Pharmacol 14:1006.
*** Cheng, Y-C; Prusoff, W.H.; 1973 Biochem Pharmacol 22:3099.

(liver), 0.4 nm; $[^{3}H]$ (kidney), 1.4 nM.

While the invention has been described and illustrated with reference to certain preferred embodiments thereof, those skilled in the art will appreciate that various changes, modifications and substitutions can be made therein without departing from the spirit and scope of the invention. For example, effective dosages other than the preferred dosages as set forth hereinabove may be applicable as a consequence of variations in the responsiveness of the mammal being treated for prevention of preterm labor, or for other indications for the compounds of the invention indicated above. Likewise, the specific pharmacological responses observed may vary according to and depending upon the particular active compound selected or whether there are present pharmaceutical carriers, as well as the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

1. A compound of the structural formula:

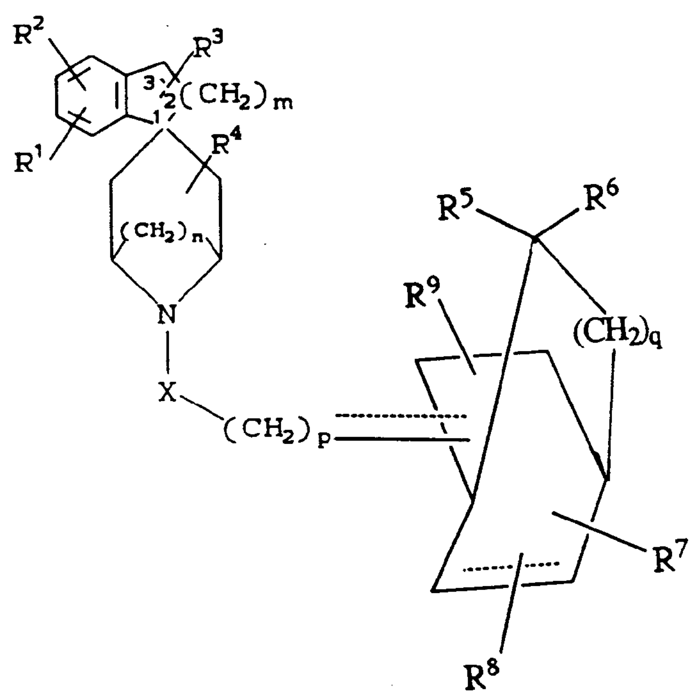

and the pharmaceutically acceptable salts thereof, wherein:

X is

C=O,
$CH_2$,
$PO_2H$,
O,
$PO_2H$-OY, where Y is $C_{1-10}$ alkyl,
S,
SO,
$SO_2$,
O
C-NH-, and
$SO_2NH$;

m is an integer of from one to three;
n is an integer of from zero to two;
p is an integer of from zero to two;

q is an integer of from zero to one;
r is an integer of from zero to five;
$R^1$ and $R^2$ are independently
hydrogen,
halogen,
hydroxy,
$C_{1-10}$ alkyl,
$C_{1-10}$ alkoxy and
trifluoromethyl;
$R^3$ is hydrogen,
halogen,
hydroxy, and
oxo
with the proviso that when $R^3$ is oxo, the 2,3 bond is saturated;
$R^4$ is hydrogen,
phenyl, and
$C_{1-15}$ alkyl;
$R^5$ and $R^6$ are independently
hydrogen,
$C_{1-10}$ alkyl, and
$C_{1-10}$ hydroxyalkyl;
further wherein $R^5$ and $R^6$ together form
oxo,
fused phenyl and
unsubstituted or substituted $C_{3-6}$cycloalkyl,
wherein said substituents are
hydroxy,
$C_{1-10}$ alkyl,
$C_{1-10}$ hydroxyalkyl
$C_{1-10}$ alkoxy
$C_{1-10}$ alkoxyalkoxyalkoxyalkyl;
$R^7$, $R^8$ and $R^9$ are independently
hydrogen,
unsubstituted or substituted $C_{1-10}$ alkyl wherein said substituents are
cyano when C is two to ten,
hydroxyl,
carboxyl,
$C_{1-10}$ alkoxy,
$C_{1-10}$ alkoxycarbonyl and
unsubstituted or substituted 5-membered heterocyclic rings containing 1 hetero atom, wherein said hetero atom is N and said substituents are
oxo,
amino,
$C_{1-10}$ alkylamino,
$C_{1-10}$ carboxyalkylcarbonylamino,
$C_{1-10}$ dicarboxyalkylamino and
$C_{1-10}$ dialkoxycarbonylalkylamino;
$C_{1-10}$ dialkyl,
$C_{7-10}$ alkoxy,
$C_{1-10}$ alkoxycarbonyl,
$C_{1-10}$ alkoxycarbonylalkylaminocarbonyl,
fused phenyl,
cyano,
$C_{2-10}$ cyanoalkyl,
phosphate,
$C_{1-10}$ alkyl substituted phosphonate,

sulfate,
$C_{1-10}$ alkyl substituted sulfonate,
halogen substituted $C_{1-10}$ alkyl substituted sulfonate,

$$-(CH_2)_r-\underset{R^{11}}{N}-R^{10},$$

wherein $R^{10}$ is
hydrogen,
$C_{1-10}$ alkylsulfonyl,
$C_{1-10}$ alkarylsulfonyl,
$C_{1-10}$ aralkylsulfonyl
$C_{1-10}$ alkoxyarylsulfonyl,
aminosulfonyl,
$C_{1-10}$ alkylaminosulfonyl
$C_{1-10}$ dialkylaminosulfonyl,
unsubstituted or substituted $C_{3-15}$ cycloalkylalkyl, bicycloalkyl or tricycloalkyl wherein said substituent is oxo and
sulfonyl substituted by unsubstituted or substituted $C_{3-15}$ cycloalkylalkyl, bicycloalkyl or tricycloalkyl wherein said substituent is
oxo,
oxime and
hydroxy;
and wherein $R^{11}$ is
hydrogen,
$C_{1-10}$ alkyl,
$C_{1-10}$ carboxyalkyl, and
$C_{1-10}$ alkoxycarbonylalkyl;

$$-(CH_2)_r-\underset{R^{11}}{N}-CO-R^{12},$$

wherein $R^{11}$ is as defined above and $R^{12}$ is
hydrogen,
amino,
$C_{1-10}$ alkylamino,
unsubstituted or substituted $C_{1-10}$ alkyl
wherein said substituents are
unsubstituted or substituted $C_{4-8}$ cycloalkyl wherein said substituent is
hydroxy and
carboxy;
$C_{10-15}$ bi and tricycloalkyl,
halogen,
hydroxy,
carboxy,
oxo,
oxime,
$C_{1-10}$ alkylthio,
$C_{1-10}$ alkylsulfinyl,
$C_{1-10}$ alkylsulfonyl,
$C_{1-10}$ alkoxycarbonyl;

unsubstituted and substituted 4, 5 and 7 membered heterocyclic rings having 1, 2, 3 or 4 hetero atoms or moieties wherein said hetero atoms or moieties are N, S, SO, $SO_2$ and O, wherein said substituents are

$C_{1-10}$ alkyl,
$C_{1-10}$ aralkyl,
$C_{1-10}$ aralkoxy,
$C_{1-10}$ alkaryl,
amino,
$C_{1-10}$ alkylamino,
$C_{1-10}$ dialkylamino,
oxo,
oxime,
fused phenyl,
$C_{1-10}$ alkoxycarbonyl,
$C_{1-10}$ alkyl carbonate,
$C_{1-10}$ alkyl ureide,
$C_{1-10}$ alkyl carbamate and
unsubstituted or substituted 5, 6 and 7 membered heterocyclic rings having 1, 2, 3 and 4 hetero atoms, wherein said hetero atoms are N, O and S and wherein said
substituents are
oxo and
fused phenyl;

unsubstituted or substituted phenyl, wherein said substituents are

halogen,
hydroxy,
carboxy,
$C_{1-10}$ alkyl,
$C_{1-10}$ carboxyalkyl,
$C_{1-10}$ alkoxy,
sulfate and
5, 6 and 7 membered heterocyclic rings having 1, 2, 3 or 4 hetero atoms, wherein said hetero atoms are N, O and S;

amino,
aminocarbonyl,
$C_{1-10}$ dialkylaminocarbonyl,
$C_{1-10}$ alkylamino,
$C_{1-10}$ dialkylamino,
$C_{1-10}$ alkylcarbamate,
$C_{1-10}$ alkylcarbonate,
$C_{1-10}$ alkylureide,
$C_{1-10}$ aralkylcarbamate,
unsubstituted or substituted aryloxy wherein said substituents are
amino,
$C_{1-10}$ alkyl and
$C_{1-10}$ aminoalkyl;
$C_{1-10}$ aralkyloxy,
unsubstituted or substituted $C_{1-10}$ alkaryloxy wherein said substituent is
$C_{1-10}$ alkyl carbamate,
N, N-$C_{1-10}$ alkyl-$C_{1-10}$ carboxyalkyl and
N, N, N-$C_{1-10}$ dialkyl-$C_{1-10}$ alkoxycarbonylalkyl;

unsubstituted or substituted $C_{3-8}$ cycloalkyl wherein said substituents are
$C_{1-10}$ alkyl and
carboxy;
N, N-$C_{1-10}$ dialkyl,
unsubstituted or substituted $C_{7-15}$

bicycloalkyl and tricycloalkyl wherein said substituent is
carboxy;
$C_{1-10}$ alkoxy,
unsubstituted or substituted phenyl, wherein said substituents are
amino,
halogen,
$C_{1-10}$ alkyl,
$C_{1-10}$ alkoxy,
nitro,
phenyl carbonyl and
unsubstituted or substituted 5, 6 and 7 membered heterocyclic rings containing 1, 2, 3 or 4 heterocyclic atoms,
wherein said hetero atoms are O, N and S and wherein said substituent is
oxo;
unsubstituted or substituted 4, 5, 6, 7 or 8 membered mono and bicyclic heterocyclic rings containing 1, 2, 3 or 4 hetero atoms or moieties wherein said hetero atoms or moieties are N, O, S, SO and $SO_2$ and wherein said substituents are
oxo,
hydroxy,
carboxy,
amino,
$C_{1-10}$ carboxyalkyl,
$C_{1-10}$ alkyl,
$C_{1-10}$ alkoxy,
$C_{1-10}$ aralkoxy,
$C_{1-10}$ alkaryloxy,
$C_{1-10}$ alkoxycarbonyl,
$C_{1-10}$ alkoxycarbonylamino,
$C_{1-10}$ alkoxycarbonylalkyl,
$C_{1-10}$ aralkoxycarbonyl and
substituted or unsubstituted aryl wherein said substituent is
$C_{1-5}$ alkyl,
carboxy and
halogen;
further wherein $R^7$ and $R^8$ are joined to form 5, 6 and 7 membered heterocyclic rings containing 2, 3 or 4 hetero atoms wherein said hetero atoms are N, O and S, and
$-(CH_2)_r$-CO-NH-$R^{13}$, wherein $R^{13}$ is
$C_{1-10}$ alkyl,
$C_{1-10}$ aminoalkyl,
$C_{1-10}$ alkoxycarbonylalkyl,
$C_{1-10}$ carboxyalkyl,
8 membered bicyclic heterocyclic rings containing 1 or 2 hetero atoms wherein said hetero atoms are N,
$-(CH_2)_r$NH-CO-NH-$R^{14}$, where $R^{14}$ is
hydrogen,
substituted or unsubstituted $C_{1-10}$ alkyl and $C_{7-15}$ bi- and tricycloalkyl
wherein said substituents are
carboxy,
$C_{1-10}$ alkoxycarbonyl,
aminocarbonyl and
$C_{1-10}$ mono and dialkylaminocarbonyl;
substituted and unsubstituted phenyl wherein said substituents are
hydroxy,
carboxy,
halogen,
$C_{1-10}$ alkyl and

$C_{1-10}$ carboxyalkyl; and

substituted and unsubstituted 5, 6 and 7 membered heterocyclic rings containing 1 or 2 hetero atoms or moieties wherein said hetero atoms or moieties are N, O, S, SO and $SO_2$ and wherein said substituents are

oxo,
carboxy,
amino,
$C_{1-10}$ carboxyalkyl,
$C_{1-10}$ alkyl and
$C_{1-10}$ alkoxycarbonylamino.

**2.** A compound of the structural formula:

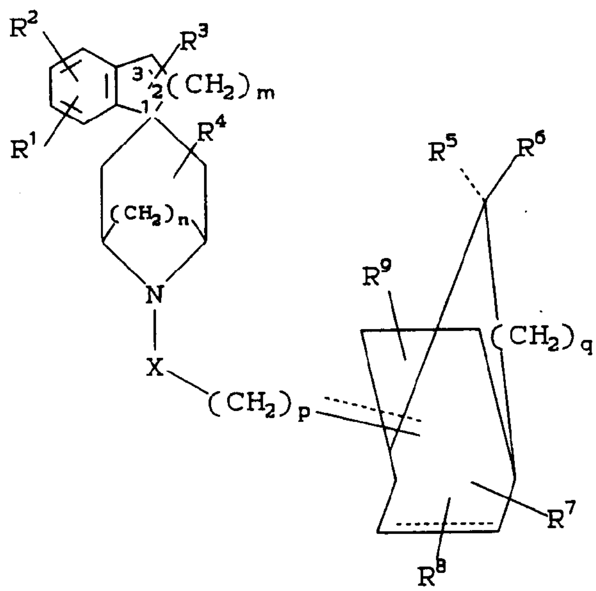

and the pharmaceutically acceptable salts thereof, wherein
X is
C=O,
$CH_2$,
$PO_2$,
O
P-OY, where Y is $C_{1-10}$ alkyl,
S,
SO and
$SO_2$;
m is an integer of from one to three;
n is an integer of from zero to two;
p is an integer of from zero to one;
q is an integer of from zero to one;
r is an integer of from zero to five;
$R^1$ and $R^2$ are independently
hydrogen,
halogen and
$C_{1-10}$ alkyl;
$R^3$ is hydrogen,
halogen,
hydroxy, and
oxo
with the proviso that, when $R^3$ is oxo, the 2,3 bond is saturated;

$R^4$ is hydrogen, or
phenyl;
$R^5$ and $R^6$ are independently
hydrogen and
$C_{1-10}$ alkyl;
further wherein $R^5$ and $R^6$ together form
oxo,
fused phenyl and
unsubstituted or substituted $C_{1-10}$ cycloalkyl,
wherein said substituents are
hydroxy,
$C_{1-10}$ hydroxyalkyl,
$C_{1-10}$ alloxyalkoxyalkoxyalkyl;
$R^7$, $R^8$ and $R^9$ are independently
hydrogen,
unsubstituted or substituted $C_{1-10}$ alkyl wherein
said substituents are
cyano when C is two to ten,
hydroxy,
carboxyl,
$C_{1-10}$ alkoxy,
$C_{1-10}$ alkoxycarbonyl and
unsubstituted or substituted 5 membered heterocyclic rings containing 1 hetero atom, wherein said hetero atom is N and said substituents are
oxo,
amino,
$C_{1-10}$ alkylamino,
$C_{1-10}$ carboxyalkylcarbonylamino,
$C_{1-10}$ dicarboxyalkylamino and
$C_{1-10}$ dialkoxycarbonylalkylamino,
$C_{1-10}$ dialkyl,
$C_{7-10}$ alkoxy,
$C_{1-10}$ alkoxycarbonyl,
$C_{1-10}$ alkoxycarbonylalkylaminocarbonyl,
fused phenyl,
phosphate,
$C_{1-10}$ alkyl substituted phosphonate,
sulfate,
$C_{1-10}$ alkyl substituted sulfonate,
halogen substituted $C_{1-10}$ alkyl substituted sulfonate,

$$-(CH_2)_r-\underset{R^{11}}{N}-R^{10},$$

wherein $R^{10}$ is
hydrogen,
$C_{1-10}$ alkylcarbonyl,
halogen,
sulfonyl,
$C_{1-10}$ alkylsulfonyl,
$C_{1-10}$ alkarylsulfonyl,
$C_{1-10}$ alkoxyarylsulfonyl,
aminosulfonyl,
dialkylaminosulfonyl,

unsubstituted or substituted $C_{3-15}$ cycloalkylalkyl wherein said substituent is
oxo and
sulfonyl substituted by unsubstituted or substituted $C_{3-15}$ cycloalkylalkyl wherein said substituent is
oxo;
and wherein $R^{11}$ is
hydrogen,
$C_{1-10}$ alkyl,
$C_{1-10}$ carboxyalkyl, and
$C_{1-10}$ alkoxycarbonylalkyl;

$$-(CH_2)_r-\underset{R^{11}}{N}-CO-R^{12},$$

wherein $R^{11}$ is as defined above and $R^{12}$ is
hydrogen,
amino,
$C_{1-10}$ alkylamino,
unsubstituted or substituted $C_{1-10}$ alkyl wherein said substitutents are
unsubstituted or substituted $C_{5-6}$ cycloalkyl wherein said substituent is hydroxy,
$C_{10-15}$ tricycloalkyl,
halogen,
hydroxy,
carboxy,
oxo
$C_{1-10}$ alkylthio,
$C_{1-10}$ alkoxycarbonyl;
unsubstituted and substituted 5, 6 and 7 membered heterocyclic rings having 1, 2, 3 or 4 hetero atoms wherein said hetero atoms are N, S and O, wherein said substituents are
$C_{1-10}$ alkyl,
$C_{1-10}$ aralkyl,
$C_{1-10}$ aralkoxy,
amino,
oxo
fused phenyl,
$C_{1-10}$ alkoxycarbonyl,
$C_{1-10}$ alkyl carbamate and
unsubstituted or substituted 5 membered heterocyclic rings having 1 hetero atom, wherein said hetero atom is N and wherein said substituents are
oxo and
fused phenyl;
unsubstitued or substituted phenyl,
wherein said substituents are
hydroxy,
carboxy,
$C_{1-10}$ alkyl,
$C_{1-10}$ carboxyalkyl,
$C_{1-10}$ alkoxy,
sulfate and
5 and 6 membered heterocyclic rings having 1 or 2 hetero atoms wherein said hetero atoms are N and S;

amino,
alkylamino,
dialkylamino,
$C_{1-10}$ alkylcarbamate,
$C_{1-10}$ aralkylcarbamate,
unsubstituted or substituted aryloxy
wherein said substituents are
amino,
$C_{1-10}$ alkyl and
$C_{1-10}$ aminoalkyl;
$C_{1-10}$ aralkyloxy,
unsubstituted or substituted $C_{1-10}$
alkaryloxy wherein said substituent is
$C_{1-10}$ alkyl carbamate,
N, N-$C_{1-10}$ alkyl-$C_{1-10}$ carboxyalkyl
and
N, N, N-$C_{1-10}$-dialkyl-$C_{1-10}$
alkoxycarbonylalkyl;
unsubstituted and substituted $C_{3-6}$
cycloalkyl wherein said substituents are
$C_{1-10}$ alkyl and
carboxy;
N, N-$C_{1-10}$ dialkyl,
unsubstituted or substituted $C_7$ bicycloalkyl
wherein said substituent is
carboxy;
$C_{1-10}$ alkoxy,
unsubstituted or substituted phenyl, wherein said substituents are
amino,
halogen,
$C_{1-10}$ alkyl,
$C_{1-10}$ alkoxy,
nitro,
phenyl carbonyl and
unsubstituted or substituted 5 membered heterocyclic rings containing 1 heterocyclic atom, wherein said hetero atom is O and wherein said substituent is
oxo;
unsubstituted or substituted 4, 5, 6, 7 or 8 membered mono and bicyclic heterocyclic rings containing 1 or 2 hetero atoms wherein said hetero atoms are N, O and S and wherein said substituents are
oxo,
hydroxy,
carboxy,
$C_{1-10}$ carboxyalkyl,
$C_{1-10}$ alkyl,
$C_{1-10}$ alkoxy,
$C_{1-10}$ aralkoxy,
$C_{1-10}$ alkoxycarbonyl,
$C_{1-10}$ alkoxycarbonylalkyl and
$C_{1-10}$ aralkoxycarbonyl,

further wherein $R^7$ and $R^8$ are joined to form a 5 membered heterocyclic ring containing 2 hetero atoms wherein said hetero atoms are N and O and
-$(CH_2)_r$-CO-NH-$R^{13}$, wherein $R^{13}$ is
$C_{1-10}$ alkyl and
$C_{1-10}$ aminoalkyl, and
$C_{1-10}$ alkoxycarbonylalkyl.

3. A compound of the formula:

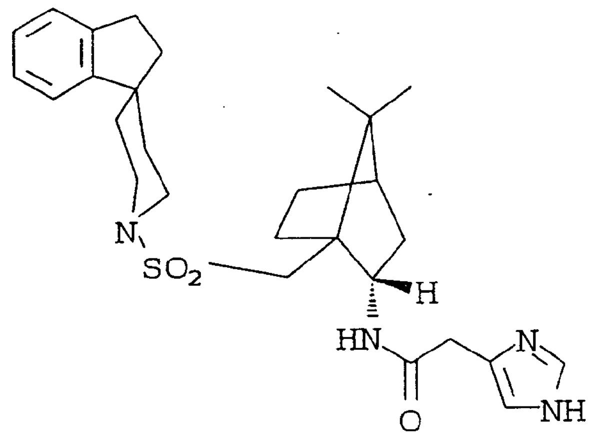

4. A compound of the formula

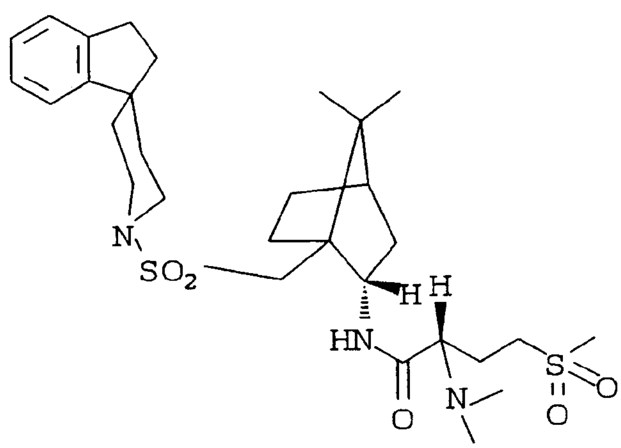

and the pharmaceutically acceptable salts thereof.

5. A compound of the formula

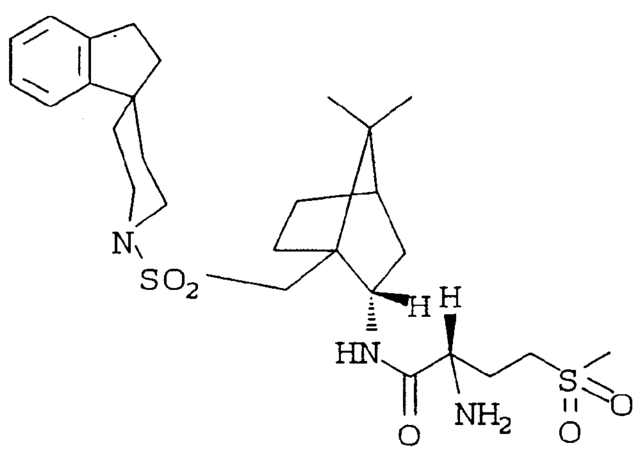

and the pharmaceutically acceptable salts thereof.

6. A pharmaceutical composition comprising a compound as claimed in claim 1 or claim 3 in a pharmacologically effective amount for antagonizing the binding of oxytocin to its receptor site, and a pharmaceutically acceptable carrier.

7. The use of a compound as claimed in claim 1 or claim 3 for the manufacture of a medicament for preventing pre-term labor.

8. The use of a compound as claimed in claim 1 or claim 3 for the manufacture of a medicament for treating dysmenorrhea.

9. The use of a compound as claimed in claim 1 or claim 3 for the manufacture of a medicament for stopping labor preparatory to cesarean delivery.

10. The use of a compound as claimed in claim 1 or claim 3 for the manufacture of a medicament for antagonizing the binding of oxytocin to its receptor site.

11. The use of a compound as claimed in claim 1 or claim 3 for the manufacture of a medicament for antagonizing the binding of vasopressin to its receptor site.